# EUROPEAN PATENT SPECIFICATION

(11) **EP 1 848 424 B1**
(45) Date of publication and mention of the grant of the patent: **05.04.2017**
(21) Application number: 06719896.0
(22) Date of filing: 30.01.2006
(51) Int. Cl.: A61K 31/353, C07D 311/58, A61P 9/00, A61P 9/08

(54) **PHARMACEUTICAL COMPOSITION COMPRISING HYDROXYLATED NEBIVOLOL**
PHARMAZEUTISCHE ZUSAMMENSETZUNG ENTHALTEND HYDROXYLIERTES NEBIVOLOL
PREPARATION PHARMACEUTIQUE COMPRENANT DU NEBIVOLOL HYDROXYLE

(30) Priority: 31.01.2005 US 648551 P; 03.01.2006 US 755856 P
(43) Date of publication of application: 31.10.2007
(73) Proprietor: MYLAN LABORATORIES, INC, Morgantown, WV 26504 (US)
(72) Inventor: O'DONNELL, John, P., Morgantown, WV 26505 (US); OWENS, Walter, Morgantown, WV 26508 (US); DUNCAN, Joseph, Morgantown, WV 26508 (US); SHAW, Andrew, Morgantown, WV 26508 (US); WU, Jinn, Princeton Junction, NJ 08550 (US)
(74) Representative: Samson & Partner Patentanwälte mbB
(86) International application number: PCT/US2006/003252
(87) International publication number: WO 2006/083779

(56) References cited:
- EP-A- 0 334 429
- COCKCROFT J: "Nebivolol: A review" EXPERT OPINION ON PHARMACOTHERAPY 2004 UNITED KINGDOM, vol. 5, no. 4, 2004, pages 893-899, XP009070520 ISSN: 1465-6566
- STOSCHITZKY K ET AL: "Different effects of exercise on plasma concentrations of nebivolol, bisoprolol and carvedilol" CARDIOVASCULAR DRUGS AND THERAPY 2004 UNITED STATES, vol. 18, no. 2, 2004, pages 135-138, XP002393073 ISSN: 0920-3206
- MCNEELY W ET AL: "NEBIVOLOL IN THE MANAGEMENT OF ESSENTIAL HYPERTENSION: A REVIEW" DRUGS, ADIS INTERNATIONAL LTD, AT, vol. 57, no. 4, April 1999 (1999-04), pages 633-651, XP009057403 ISSN: 0012-6667
- VAN PEER A ET AL: "Clinical pharmacokinetics of nebivolol. A review" DRUG INVESTIGATION 1991 NEW ZEALAND, vol. 3, no. SUPPL. 1, 1991, pages 25-30, XP009069916 ISSN: 0114-2402
- BROEDERS MARTIJN A W ET AL: "Nebivolol: A third-generation beta-blocker that augments vascular nitric oxide release: Endothelial beta2-adrenergic receptor-mediated nitric oxide production" CIRCULATION, vol. 102, no. 6, 8 August 2000 (2000-08-08), pages 677-684, XP002393075 ISSN: 0009-7322
- HENDRICKX J ET AL: "Location of the hydroxyl functions in hydroxylated metabolites of nebivolol in different animal species and human subjects as determined by on-line high-performance liquid chromatography-diode-array detection" JOURNAL OF CHROMATOGRAPHY A, ELSEVIER, AMSTERDAM, NL, vol. 729, no. 1, 5 April 1996 (1996-04-05), pages 341-354, XP004039196 ISSN: 0021-9673

## Description

This application claims priority under 35 USC §119 to U.S. Provisional Application No. 60/648,551, filed January 31,2005 and to U.S. Provisional Application No. 60/755,856, filed January 3,2006.

### FIELD OF THE INVENTION

Metabolites of nebivolol possess pharmacological properties that may be beneficial in treating a variety of diseases, such as cardiovascular diseases, which could include hypertension, atherosclerosis, and congestive heart failure. In particular, nebivolol and its key metabolites have effects on NO metabolism, independent of β1-selective interactions and the favorable effects of nebivolol metabolites on endothelial NO release has implications for its use in the treatment of cardiovascular disorders. This invention describes a 4-hydroxylated nebivolol metabolite, namely 4(S)-OH-(R,S,S,S)-nebivolol, compositions comprising the 4-hydroxyl nebivolol metabolite and methods of using such compositions to treat and/or prevent cardiovascular diseases or disorders.

### BACKGROUND OF THE INVENTION

The normal production of endothelial nitric oxide is important for maintenance of vascular function. Increasing endothelial NO release results in a reduction in smooth muscle cell contractility and decreased vascular resistance. Under conditions of hypertension and hyperlipidemia, however, endothelial dysfunction produces a marked decrease in NO release, resulting in: 1) increased vasoconstriction and vasospasm; 2) greater monocyte and LDL infiltration; 3) proliferation of vascular smooth cells; 4) increased oxidative stress; and 5) increased platelet aggregation. It has been suggested that substances that restore endothelial function and NO metabolism have benefit in the treatment of various cardiovascular disorders, including coronary artery disease and heart failure.

EP O 334 429 A1 discloses certain bis(benzopyran) derivates, among them nebivolol. Cockroft J., Expert Opin. Pharmacother. Vo). 5, No. 4 (2004) 893-899, McNeely W. et al. Drugs Vol. 57, No. 4, (1999), 633-651 and VanPeer a. et al. Drug Investigation, Vol. 3, (Suppl.1) (1991), 25-30, each disclose, among others, that in extensive metabolisers there is a substantial formation of the active hydroxy metabolites - without a specific metabolite being mentioned - which appears to compensate for the low concentration of unchanged drug in these individuals indicating that in poor metabolisers no dose adjustment is needed. Stoschitzky K. et al. Cardiovascular Drugs and Therapy Vol. 18, No. 2 (2004) 135-138, disclose that the 4-hydroxy nebivolol metabolite concentration is slightly elevated during exercise.

### SUMMARY OF THE INVENTION

This invention provides a 4-hydroxylated nebivolol metabolite, namely 4(S)-OH-(R,S,S,S)-nebivolol, and compositions comprising the 4-hydroxylated metabolite of nebivolol and optionally at least one compound used to treat cardiovascular diseases or a pharmaceutically acceptable salt there of. In addition, this invention provides pharmaceutical compositions for use in methods of treating and/or preventing cardiovascular diseases characterized by nitric oxide insufficiency, by administering the 4-hydroxylated metabolite of nebivolol that is capable of releasing a therapeutically effective amount of nitric oxide to a targeted site affected by the cardiovascular disease.

### BRIEF DESCRIPTION OF THE FIGURES

FIGURE 1: The effects of nebivolol and metabolites on peak NO release from human endothelial cells following acute treatment (50 µM treatment).
FIGURE 2: The effects of nebivolol and metabolites on peak NO release from human endothelial cells following acute treatment (50 µM treatment).
FIGURE 3: The effects of nebivolol and metabolites on peak NO release from human endothelial cells following acute treatment (10 µM treatment).
FIGURE 4: Shows peak NO release following treatment with acetylcholine, nebivolol and nebivolol metabolites (5µM Treatment)
FIGURE 5: Shows the concentration-response effects following acute treatment with nebivolol metabolites.
FIGURE 6: Shows the concentration-response following acute treatment with nebivolol, nebivolol enantiomers and 4-hydroxy nebivolol.
FIGURE 7: Shows the amount of NO released as measured following acute drug treatment (1.0 µM) of HUVECs isolated from White (open bars) and Black American (solid bars) donors using hydroxylated nebivolol. Values are mean ± S.D. (n=5).

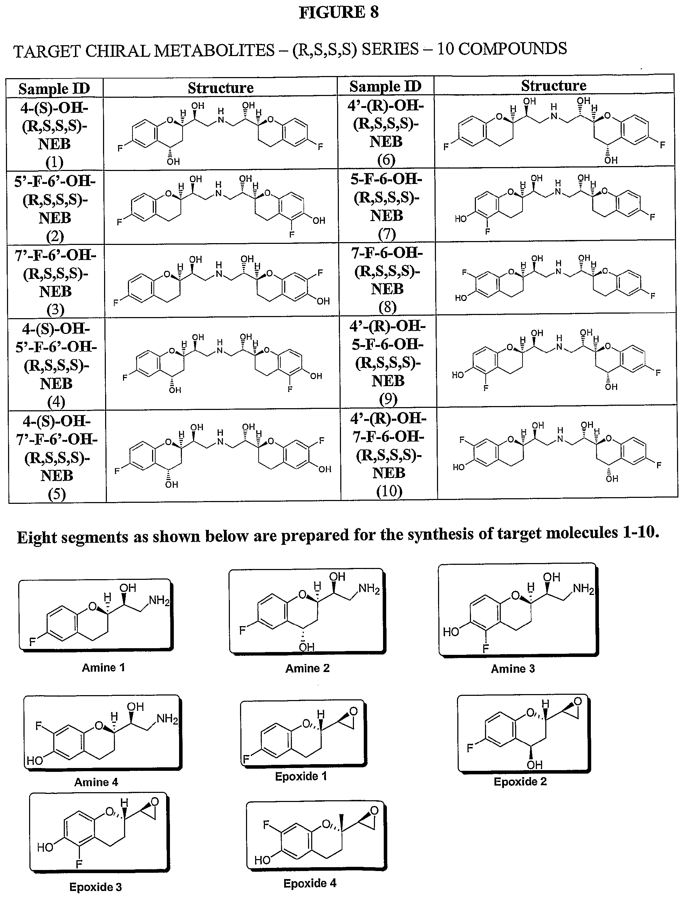

FIGURE 8: Target chiral metabolites to be synthesized - (R,S,S,S) Series - 10 Compounds.

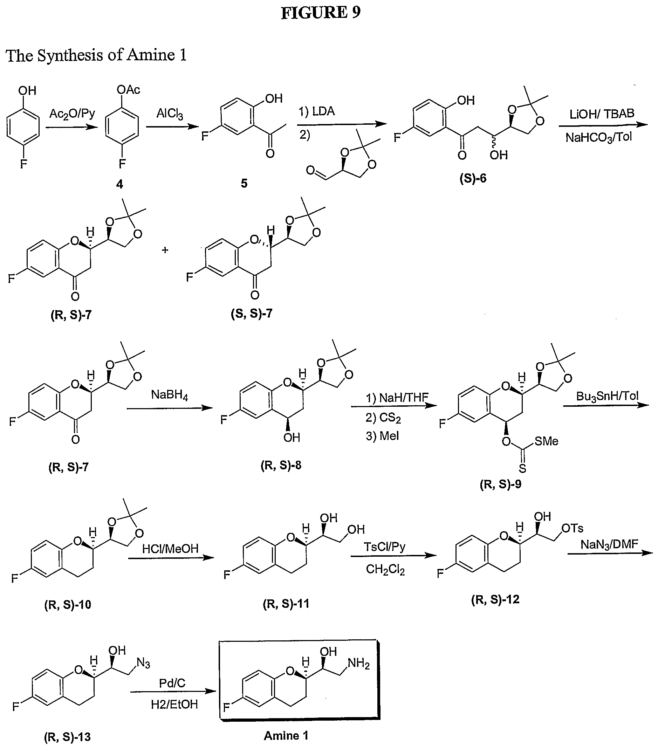

FIGURE 9: The Synthesis of Amine 1.

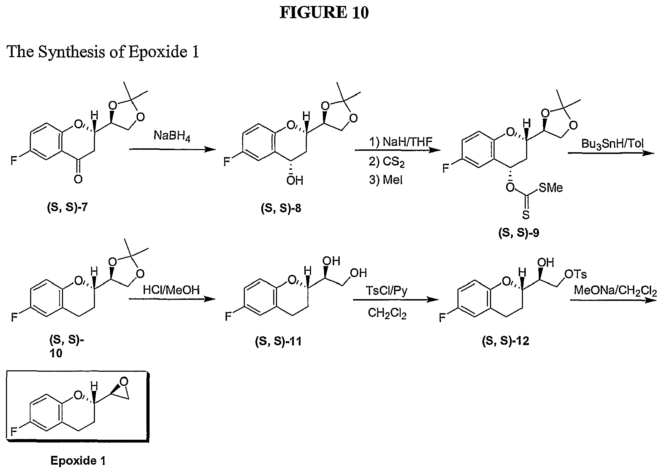

FIGURE 10: The synthesis of Epoxide 1.

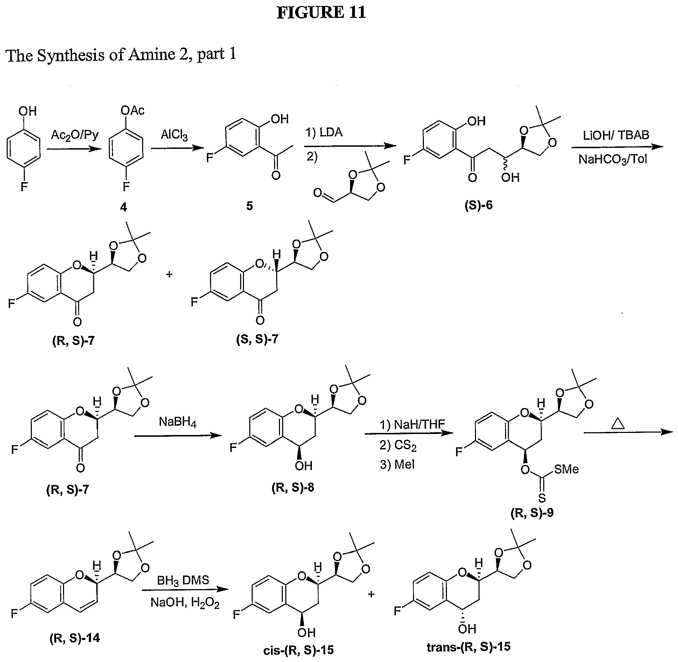

FIGURE 11: The synthesis of Amine 2, part 1

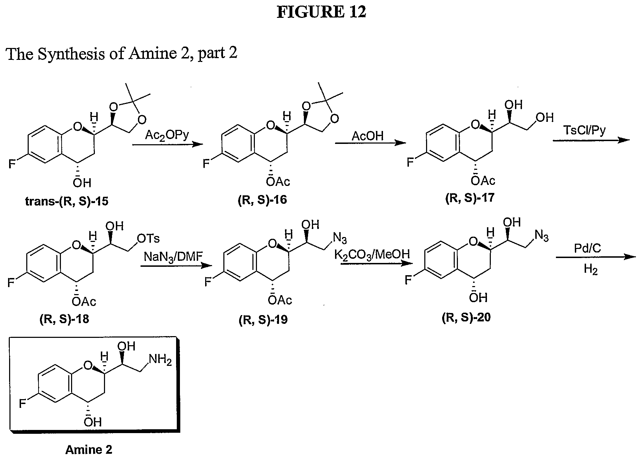

FIGURE 12: The synthesis Amine 2, part 2.

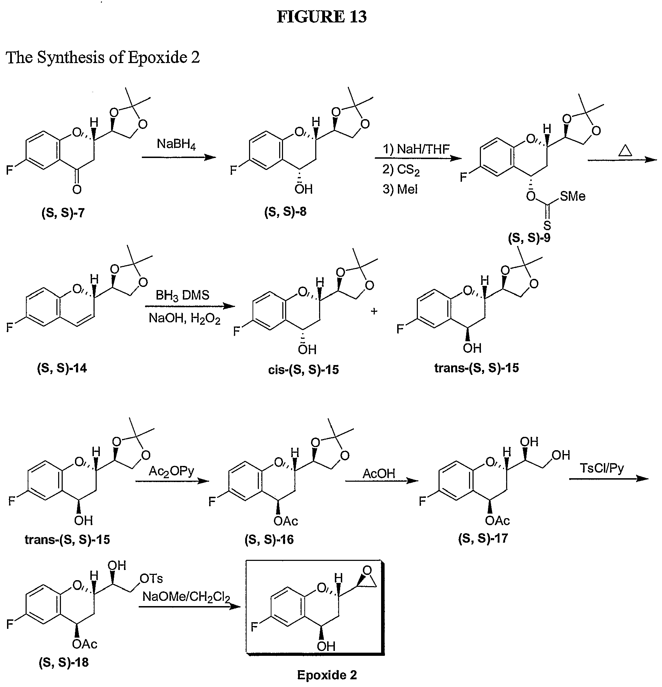

FIGURE 13: The syhnthesis of Epoxide 2

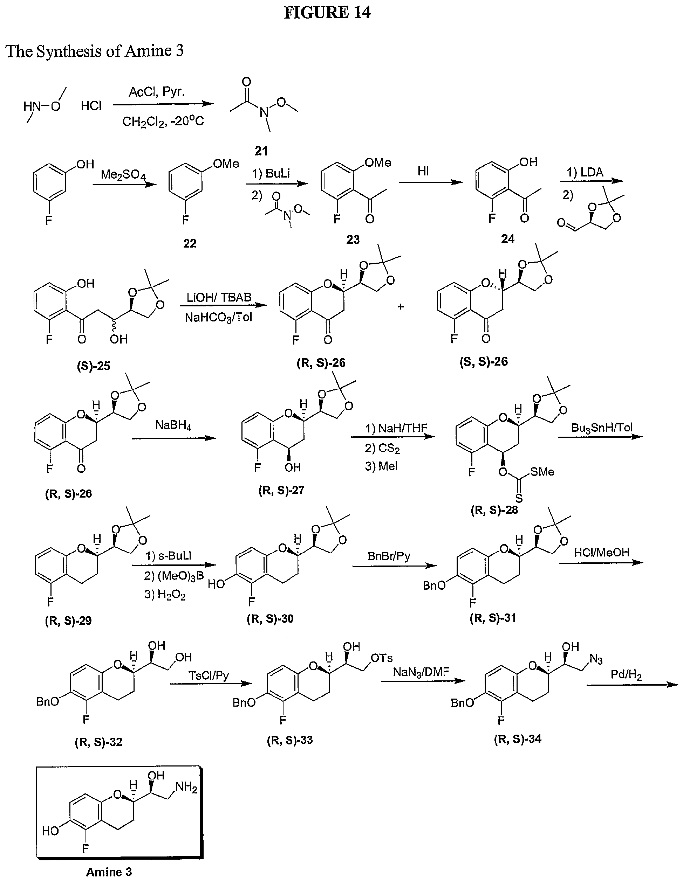

FIGURE 14: The synthesis of Amine 3.

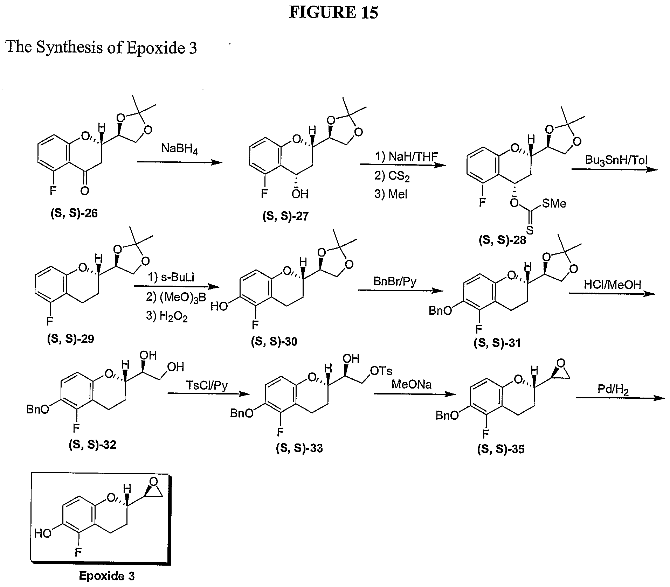

FIGURE 15: The synthesis of Epoxide 3.

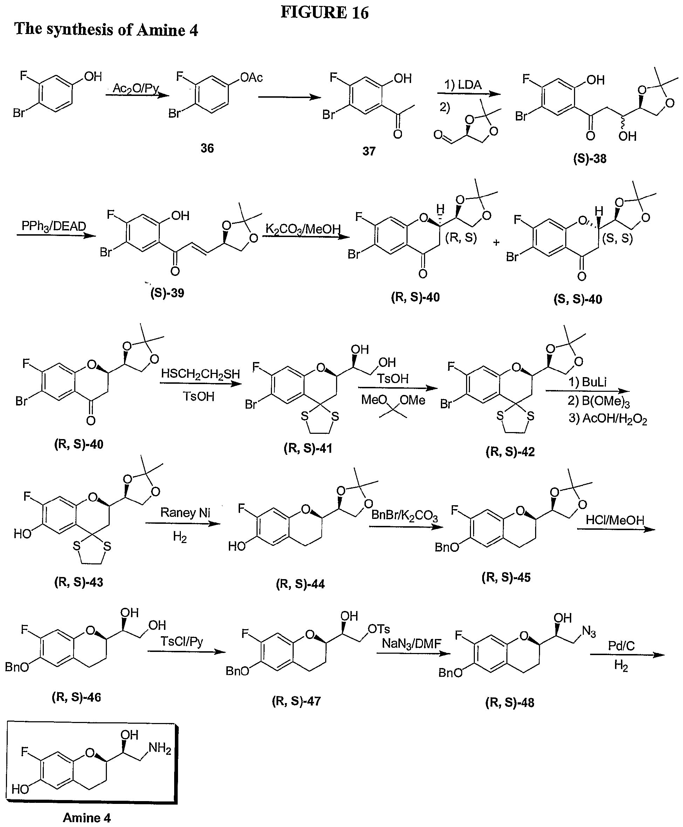

FIGURE 16: The synthesis of Amine 4.

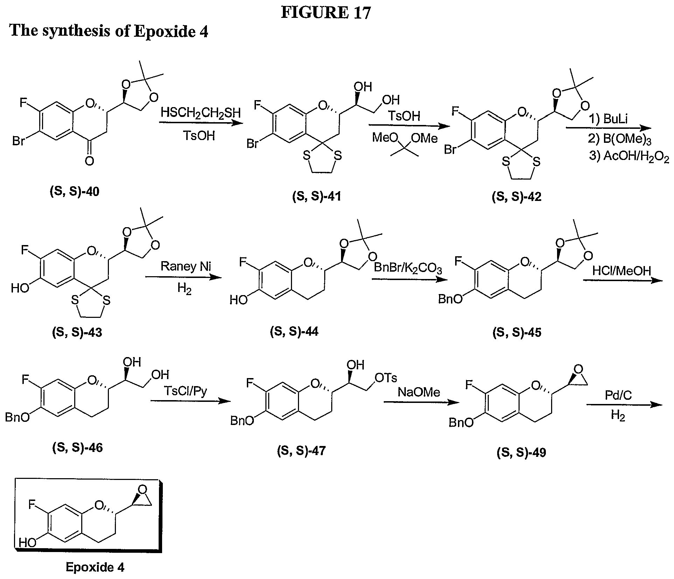

FIGURE 17: The synthesis of Epoxide 4.

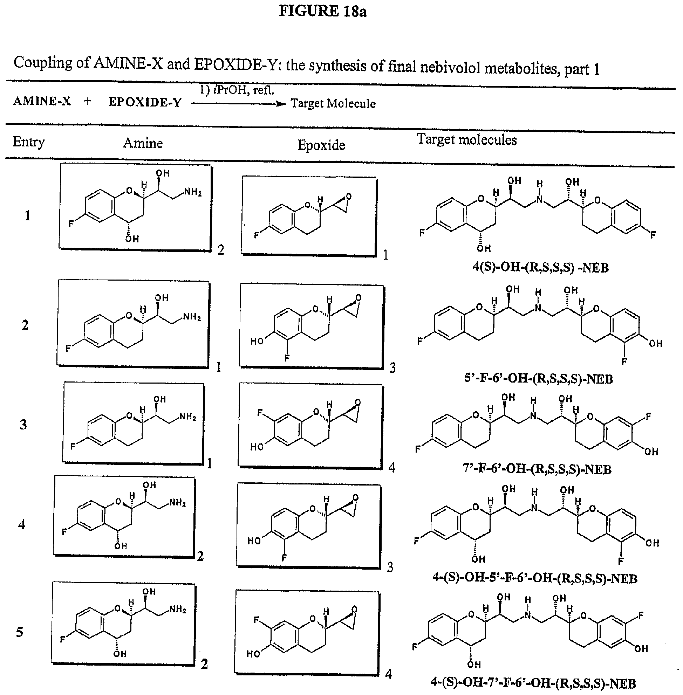

FIGURE 18a: Coupling of Amine-X and Epoxide-Y to produce targeted hydroxylated nebivolol metabolites, part 1.

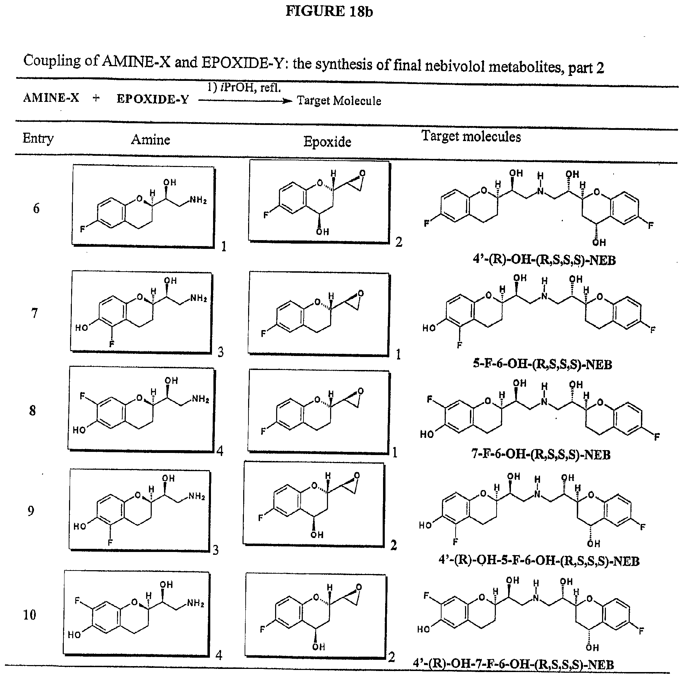

FIGURE 18b: Coupling of Amine-X and Epoxide-Y to produce targeted hydroxylated nebivolol metabolites, part 2.

### DETAILED DESCRIPTION OF THE INVENTION

### Definitions

For convenience, before further description of the present invention, certain terms employed in the specification, examples and appended claims are collected here. These definitions should be read in light of the remainder of the disclosure and understood as by a person of skill in the art. Unless defined otherwise, all technical and scientific terms used herein have the same meaning as commonly understood by a person of ordinary skill in the art.

The articles "a" and "an" are used herein to refer to one or to more than one (i.e., to at least one) of the grammatical object of the article. By way of example, "an element" means one element or more than one element.

"About the same time" means that within about thirty minutes of administering one compound (nebivolol) to the patient, the other active compound(s) is/are administered to the patient. "About the same time" also includes simultaneous administration of the compounds.

The phrase "angiotensin converting enzyme inhibitor" or "ACE inhibitor" as used herein refers to a compound that inhibits any enzyme from converting angiotensin to any other form. Angiotensin Converting Enzyme (ACE) Inhibitors

ACE inhibitors are effective antihypertensive agents that competitively inhibit angiotensin converting enzymes and prevent the conversion of angiotensin I to angiotensin II. Non-limiting examples of ACE inhibitors include alacepril, benazepril, captopril, ceronapril, cilazapril, delapril, enalapril, enalaprilat, fosinopril, imidapril, lisinopril, perindopril, quinapril, ramiprilat, ramipril, spirapril, temocapril, and trandolapril. Other ACE inhibitors may be identified using standard assaying techniques known to one of ordinary skill in the art.

The phrase "angiotensin II receptor antagonist" or "ARB" refers to a compound that binds to a receptor site on angiotensin II but does not cause any physiological changes unless another receptor ligand is present.

Angiotensin II Receptor Antagonist (ARB): ARBs are effective antihypertensive agents that have a very favorable side effect profile. Their mechanism of action is related to the selective blocking of the vasoconstrictor and aldosterone-secreting effects of angiotensin. Non-limiting examples of ARBs include candesartan, eprosartan, irbesartan, losartan, and valsartan. Other ARBs may be identified using standard assaying techniques known to one of ordinary skill in the art.

The term "antagonist" is art-recognized and refers to a compound that binds to a receptor site, but does not cause a physiological change unless another receptor ligand is present.

The term "bioavailable" is art-recognized and refers to a form of the subject invention that allows for it, or a portion of the amount administered, to be absorbed by, incorporated to, or otherwise physiologically available to a subject or patient to whom it is administered.

"Black" refers to a person of African descent or an African-American person but is not necessarily limited to those of African origin (e.g., Carribean).

"Therapeutically effective amount" refers to the amount of the compound and/or composition that is effective to achieve its intended purpose.

The phrase "cardiovascular agent" or "cardiovascular drug" refers to a therapeutic compound that is useful for treating or preventing a cardiovascular disease. Non-limiting examples of suitable compounds include (1) thiazide diuretics (including chlorothiazide (Diuril), chlorthalidone, hydrochlorothiazide, polythiazide (Renese), indapamide (Lozol) and metolazone (Mykrox, Zaroxolyn)); (2) loop diuretics (including bumetanide (Bumex) and furosemide (Lasix), torsemide (Demadex)); (3) potassium-sparing diuretics (including amiloride (Midamor) and triamterene (Dyrenium)); (4) aldosterone receptor blockers (including eplerenone (Inspra) and spironolactone (Aldactone)); (5) beta blockers (including atenolol (Tenormin), betaxolol (Kerlone), bisoprolol (Zebeta), metoprolol (Lopressor), metoprolol extended release (Toprol XL), nadolol (Corgard), propranolol (Inderal), propranolol long acting (Inderal LA) and timolol (Blocadren)); (6) beta blockers with intrinsic sympathomimetic activity (including acebutolol (Sectral), penbutolol (Levatol) and pindolol); (7) combined alpha and beta blockers (including carvedilol (Coreg) and labetalol (Normodyne, Trandate)); (8) ACE inhibitors (including benazepril (Lotensin), captopril (Capoten), enalapril (Vasotec), fosinopril (Monopril), lisinopril (Prinivil, Zestril), moexipril (Univasc), perindopril (Aceon), quinapril (Accupril), ramipril (Altace) and trandolapril (Mavik)); (9) angiotensin II antagonists (candesartan (Atacand), eprosartan (Teveten), irbesartan (Avapro), losartan (Cozaar), olmesartan (Benicar), telmisartan (Micardis) and valsartan (Diovan); (10) calcium channel blockers- non-dihydropyridines (including diltiazem extended release (Cardizem CD, Dilacor XR, Tiazac), diltiazem extended release (Cardizem LA), verapamil immediate release (Calan, Isoptin), verapamil long acting (Calan SR, Isoptin SR) and verapamil- Coer (Covera HS, Verelan PM)); (11) calcium channel blockers- dihydropyridines (including amlodipine (Norvasc), felodipine (Plendil), isradipine (DynaCirc CR), nicardipine sustained release (Cardene SR), nifedipine long-acting (Adalat CC, Procardia XL) and nisoldipine (Sular)); (12) alpha₁-blockers (including doxazosin (Cardura), prazosin (Minipress) and terazosin (Hytrin)); (13) central alpha₂-agonists and other centrally acting drugs (including clonidine (Catapres), clonidine patch (Catapres-TTS), methyldopa (Aldomet), reserpine, and guanfacine); (14) direct vasodilators (including hydralazine (Apresoline) and minoxidil (Loniten); (15) ACEIs and CCBs (including amlodipine/benazepril hydrochloride, enalapril maleate/felodipine and trandolapril/verapamil); (16) ACEIs and diuretics (including benazepril/hydrochlorothiazide, captopril/hydrochlorothiazide, enalapril maleate/hydrochlorothiazide, lisinopril/hydrochlorothiazide, moexipril HCl/hydrochlorothiazide and quinapril/hydrochlorothiazide; (17) ARBs and diuretics (including candesartan cilexetil/hydrochlorothiazide, eprosartan mesylate/ hydrochlorothiazide, irbesartan/ hydrochlorothiazide, losartan potassium/ hydrochlorothiazide, telmisartan/ hydrochlorothiazide, valsartan/ hydrochlorothiazide; (18) beta blockers and diuretics (including atenolol/chlorthalidone, bisoprolol fumarate/ hydrochlorothiazide, metoprolol tartrate/ hydrochlorothiazide, nadolol/bendrofluthiazide and timolol maleate/ hydrochlorothiazide; (19) centrally acting drug and diuretic (including methyldopa/ hydrochlorothiazide, reserpine/chlorothiazide and reserpine/ hydrochlorothiazide); (20) diuretic and diuretic (amiloride HCl/ hydrochlorothiazide, spironolactone/ hydrochlorothiazide and triamterene/ hydrochlorothiazide.

Cardiovascular disease or disorder refers to any cardiovascular disease or disorder known in the art, including, but not limited to, wherein the cardiovascular disease is selected from the group consisting of congestive heart failure, hypertension, pulmonary hypertension, myocardial and cerebral infarctions, atherosclerosis, atherogenesis, thrombosis, ischemic heart disease, post-angioplasty restenosis, coronary artery diseases, renal failure, stable, unstable and variant (Prinzmetal) angina, cardiac edema, renal insufficiency, nephrotic edema, hepatic edema, stroke, transient ischemic attacks, cerebrovascular accidents, restenosis, controlling blood pressure in hypertension, platelet adhesion, platelet aggregation, smooth muscle cell proliferation, pulmonary edema, and vascular complications associated with the use of medical devices.

The term "combination" refers to two or more different active agents which are administered at roughly about the same time (for example, where the active agents are in a single pharmaceutical preparation) or at different times (for example, one agent is administered to the subject before the other).

The terms "drug," "pharmaceutically active agent," "bioactive agent," "therapeutic agent," and "active agent" may be used interchangeably and refer to a substance, such as a chemical compound or complex, that has a measurable beneficial physiological effect on the body, such as a therapeutic effect in treatment of a disease or disorder, when administered in an effective amount. Further, when these terms are used, or when a particular active agent is specifically identified by name or category, it is understood that such recitation is intended to include the active agent per se, as well as pharmaceutically acceptable, pharmacologically active derivatives thereof, or compounds significantly related thereto, including without limitation, salts, pharmaceutically acceptable salts, N-oxides, prodrugs, active metabolites, isomers, fragments, analogs, solvates hydrates, radioisotopes, etc.

The phrase "effective amount" refers to that amount of a substance that produces some desired local or systemic effect at a reasonable benefit/risk ratio applicable to any treatment. The effective amount of such substance will vary depending upon the subject and disease condition being treated, the weight and age of the subject, the severity of the disease condition, the manner of administration and the like, which can readily be determined by one of ordinary skill in the art.

"Endothelial dysfunction" refers to the impaired ability of in any physiological processes carried out by the endothelium, in particular, production of nitric oxide regardless of cause. It may be evaluated by, such as, for example, invasive techniques, such as, for example, coronary artery reactivity to acetylcholine or methacholine, and the like, or by noninvasive techniques, such as, for example, blood flow measurements, brachial artery flow dilation using cuff occlusion of the arm above or below the elbow, brachial artery ultrasonography, imaging techniques, measurement of circulating biomarkers, such as, asymmetric dimethylarginine (ADMA), and the like. For the latter measurement the endothelial-dependent flow-mediated dilation will be lower in patients diagnosed with an endothelial dysfunction.

The phrase "endothelial nitric oxide synthase" or "eNOS" refers to enzymes that produce nitric oxide.

The term "pharmaceutically acceptable salts" is art-recognized and refers to the relatively non-toxic, inorganic and organic acid addition salts of compounds, including, for example, those contained in compositions of the present invention.

The term "pharmaceutically acceptable carrier" is art-recognized and refers to a pharmaceutically-acceptable material, composition or vehicle, such as a liquid or solid filler, diluent, excipient, solvent or encapsulating material, involved in carrying or transporting any subject composition or component thereof from one organ, or portion of the body, to another organ, or portion of the body. Each carrier must be acceptable in the sense of being compatible with the subject composition and its components and not injurious to the patient. Some examples of materials which may serve as pharmaceutically acceptable excipients include: (1) sugars, such as lactose, glucose and sucrose; (2) starches, such as corn starch and potato starch; (3) cellulose, and its derivatives, such as sodium carboxymethyl cellulose, ethyl cellulose and cellulose acetate; (4) powdered tragacanth; (5) malt; (6) gelatin; (7) talc; (8) excipients, such as cocoa butter and suppository waxes; (9) oils, such as peanut oil, cottonseed oil, safflower oil, sesame oil, olive oil, corn oil and soybean oil; (10) glycols, such as propylene glycol; (11) polyols, such as glycerin, sorbitol, mannitol and polyethylene glycol; (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) IV fluids, including but not limited to Ringer's solution, 5% dextrose in water, and half normal saline; (19) ethyl alcohol; (20) phosphate buffer solutions; and (21) other non-toxic compatible substances employed in pharmaceutical formulations.

"Patient" refers to animals, preferably mammals, most preferably humans, and includes males and females.

"Quality of life" refers to one or more of a person's ability to walk, climb stairs, do errands, work around the house, participate in recreational activities, and/or not requiring rest during the day, and/or the absence of sleeping problems or shortness of breath.

The term "prophylactic" or "therapeutic" treatment is art-recognized and refers to administration to the host of one or more of the subject compositions. If it is administered prior to clinical manifestation of the unwanted condition (e.g., disease or other unwanted state of the host animal) then the treatment is prophylactic, i.e., it protects the host against developing the unwanted condition, whereas if administered after manifestation of the unwanted condition, the treatment is therapeutic (i.e., it is intended to diminish, ameliorate or maintain the existing unwanted condition or side effects therefrom).

The term "structure-activity relationship" or "(SAR)" is art-recognized and refers to the way in which altering the molecular structure of a drug or other compound alters its interaction with a receptor, enzyme, nucleic acid or other target and the like.

It will be understood that "substitution" or "substituted with" includes the implicit proviso that such substitution is in accordance with permitted valence of the substituted atom and the substituent, and that the substitution results in a stable compound, e.g., which does not spontaneously undergo transformation such as by rearrangement, cyclization, elimination, or other reaction.

The term "substituted" is also contemplated to include all permissible substituents of organic compounds. In a broad aspect, the permissible substituents include acyclic and cyclic, branched and unbranched, carbocyclic and heterocyclic, aromatic and nonaromatic substituents of organic compounds. Illustrative substituents include, for example, those described herein above. The permissible substituents may be one or more and the same or different for appropriate organic compounds. For purposes of this disclosure, the heteroatoms such as nitrogen may have hydrogen substituents and/or any permissible substituents of organic compounds described herein which satisfy the valences of the heteroatoms.

The term "synthetic" is art-recognized and refers to production by in vitro chemical or enzymatic synthesis.

The phrase "therapeutic effect" is art-recognized and refers to a local or systemic effect in animals, particularly mammals, and more particularly humans caused by a pharmacologically active substance. The term thus means any substance intended for use in the diagnosis, cure, mitigation, treatment or prevention of disease or in the enhancement of desirable physical or mental development and/or conditions in an animal or human. The phrase "therapeutically-effective amount" means that amount of such a substance that produces some desired local or systemic effect at a reasonable benefit/risk ratio applicable to any treatment. The therapeutically effective amount of such substance will vary depending upon the subject and disease condition being treated, the weight and age of the subject, the severity of the disease condition, the manner of administration and the like, which can readily be determined by one of ordinary skill in the art.

The term "treating" is art-recognized and refers to curing as well as ameliorating at least one symptom of any condition or disease.

The term "metabolite" or "nebivolol metabolite" as used herein includes hydroxyl substitution at one or more of positions 3-8 and 3'-8', wherein positions 3-8 and 3'-8' shown on the parent nebivolol compound may each independently be hydroxyl groups, while positions 3-8 and 3'-8' may each independently be halogen atoms, such as fluorine, or each independently be hydrogen atoms. For example, the compound may be mono-hydroxylated at the 4, 4', 5, 5' 8, or 8' positions and di-fluorinated at the 6 and 6' positions. In another example, the compound could also be di-hydroxylated at the 4, 5'; 4', 5; 4,8'; 4',8, or 4',8' positions and be di-fluorinated at the 6 and 6' positions in all of these instances. This invention includes within its scope the stereoisomer 4(S)-OH-(R,S,S,S)-nebivolol. The terms "metabolite" and "nebivolol metabolite" includes all such structural isomers and stereoisomers and mixtures thereof, and those not explicitly described.

The phrase "therapeutic effect" is art-recognized and refers to a local or systemic effect in animals, particularly mammals, and more particularly humans caused by a pharmacologically active substance. The term thus means any substance intended for use in the diagnosis, cure, mitigation, treatment or prevention of disease or in the enhancement of desirable physical or mental development and/or conditions in an animal or human. The phrase "therapeutically-effective amount" means that amount of such a substance that produces some desired local or systemic effect at a reasonable benefit/risk ratio applicable to any treatment. The therapeutically effective amount of such substance will vary depending upon the subject and disease condition being treated, the weight and age of the subject, the severity of the disease condition, the manner of administration and the like, which can readily be determined by one of ordinary skill in the art.

The parent nebivolol compound and its hydroxylated metabolites can be synthesized by one skilled in the art following the methods described in, for example, U.S. Pat. Nos. 4,654,362, 5,759,580, 6,075,046, 6,545,040 and in EP 0 145 067, EP 0 334 429, and in WO 95/22325 and WO 96/19987; Van Lommen et al., J. Pharm. Belg., 45(6): 355-360 (1990); Chandrasekhar, S. et al., Tetrahedron, 56(34): 6339-6344 (2000); and Kendrick et al., J. Chromatogram. A., 729: 341-354 (1996).

The hydroxylated metabolites may also be prepared by the synthetic procedures found in Appendix 1. Reactions are performed in solvents appropriate to the reagents, and materials used are suitable for the transformations being effected. It is understood by one skilled in the art of organic synthesis that the functionality present in the molecule must be consistent with the chemical transformation proposed. This will, on occasion, necessitate judgment by the scientist as to the order of synthetic steps, protecting groups required, and deprotection conditions. Substituents on the starting materials may be incompatible with some of the reaction conditions required in some of the methods described, but alternative methods and substituents compatible with the reaction conditions will be readily apparent to one skilled in the art. The use of sulfur and oxygen protecting groups is known in the art for protecting thiol and alcohol groups against undesirable reactions during a synthetic procedure and many such protecting groups are known, e.g., T. H. Greene and P. G. M. Wuts, Protective Groups in Organic Synthesis, John Wiley & Sons, New York (1999).

The hydroxylated metabolites have basic properties and, consequently, they may be converted to their therapeutically active non-toxic acid addition salt forms by treatment with appropriate acids, such as, for example, inorganic acids, such as hydrohalic acid, e.g. hydrochloric, hydrobromic and the like, and sulfuric acid, nitric acid, phosphoric acid and the like; or organic acids, such as, for example, acetic, propanoic, hydroxyacetic, 2-hydroxypropanoic, 2-oxopropanoic, ethanedioic, propanedioic, butanedioic, (Z)-2-butenedioic, (E)-2-butenedioic, 2-hydroxybutanedioic, 2,3-dihydroxybutanedioic, 2-hydroxy-1,2,3-propane-tricarboxylic, methanesulfonic, ethanesulfonic, benzenesulfonic, 4-methylbenzenesulfonic, cyclohexanesulfamic, 2-hydroxybenzoic, 4-amino-2-hydroxybenzoic and the like acids. Conversely, the salt form can be converted by treatment with alkali into the free base form.

### HYDROXYLATED NEBIVOLOL METABOLITES

Hydoxylated metabolites of nebivolol have the ability to increase the capacity for NO release in human endothelial cells following (chronic) administration. While not being bound by theory, the hydroxylated metabolites of nebivolol are thought to function via activation of the L-arginine/nitric oxide pathway. They are endothelial-dependent vasodilatory agents with additional cardio-selective (i.e., beta-selective) blocking capacity, which have the potential to maintain endothelial function or improve endothelial dysfunction and be effective medications for the treatment of cardiovascular diseases, such as atherosclerosis, hypertension, coronary artery disease and congestive heart failure. Hydroxylated nebivolol metabolites could be used as a mono-therapy to treat or prevent the aforementioned disorders or in combination with other appropriate pharmaceutical agents either in the same dosage form/product or concomitantly administered as separate dosage forms/products to adequately treat/prevent the aforementioned disorders. Since these compounds have the capacity to increase endothelial nitric oxide concentrations, they may have added benefits in special populations such as diabetics, chronic smokers, Black-Americans, patients with acute coronary syndromes (ACS), coronary heart disease (CHD), metabolic syndrome and people suffering from obesity.

The direct endothelial benefit for the hydroxylated metabolites of nebivolol was observed at concentrations as low as 250 nM. By enhancing the ability of cells to release NO, these nebivolol metabolites may have strong vasodilatory effects and the ability to restore abnormalities in endothelial function associated with hypertension. Further, these metabolites have the potential for pronounced affects in special populations prone to cardiovascular disease, such as Black Americans, and these metabolites may have effects in HUVEC (Human Umbilical Vein Endothelial Cells) in concentrations as low as 250 nM.

Hydroxylated nebivolol metabolites are represented by the formula (I) 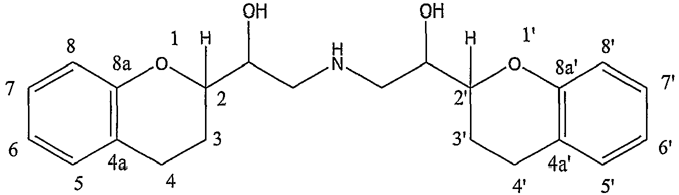 wherein positions 3-8 and 3'-8' shown on the parent nebivolol compound may each independently be hydroxyl groups (and at least one of them is an -OH), while positions 3-8 and 3'-8' may each independently be halogen atoms, such as fluorine, or each independently be hydrogen atoms. For example, the compound may be mono-hydroxylated at the 4, 4', 5, 5', 8, or 8' positions and di-fluorinated at the 6 and 6' positions. In another example, the compound could be di-hydroxylated at the 4, 5'; 4', 5; 4,8'; 4',8, or 4',8' positions and be di-fluorinated at the 6 and 6' positions in all of these instances. This invention includes within its scope the stereoisomer 4(S)-OH-(R,S,S,S)-nebivolol.

It is to be understood that the hydroxylated nebivolol metabolites can exist as the optically pure enantiomers, pure diastereomers, mixtures of enantiomers, mixtures of diastereomers, racemic mixtures of enantiomers, diastereomeric racemates or mixtures of diastereomeric racemates. It is to be understood that the compound has at a minimum 4 stereocenters, and if the pyran ring(s) is/are substituted with a hydroxyl functionality, the compound has minimum of 5 stereocenters.

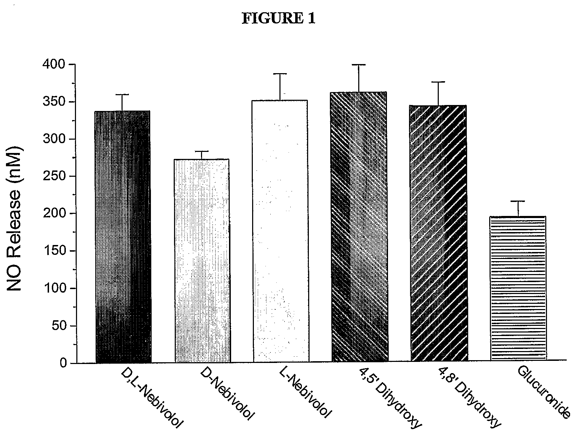

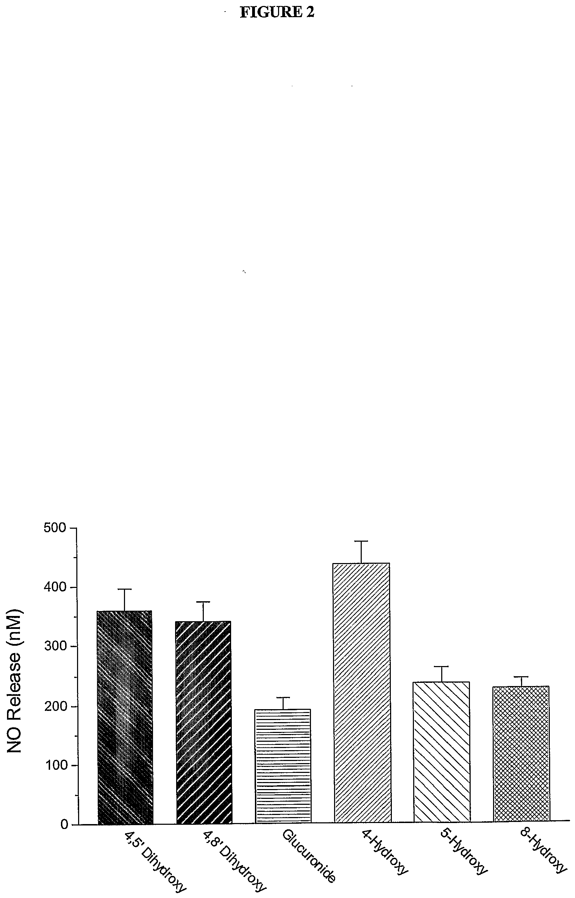

For instance, the hydroxy-substituted nebivolol metabolites 4,5'-dihydroxy-6,6'-difluoro, 4,8'-dihydroxy-6,6'-difluoro- and 4-hydroxy-6,6'-difluoro-increase NO release from human endothelial cell in concentration dependent fashion following acute administration. For example, the 4,5'-dihydroxy-6,6'-difluoro-, and 4,8'-dihydroxy-6,6'-difluoro- are agonists of NO release, with a maximum of about 330-350 nM NO at a 50µM drug concentration (see Figures 1 and 2). The hydroxylated metabolites of nebivolol are thought to function as anti-oxidants and prevent lipid peroxidation.

In part, the present invention features compositions comprising the 4(S)-OH-(R,S,S,S)-nebivolol metabolite and at least one other cardiovascular agent. In addition, the present invention features compositions comprising said nebivolol metabolite and at least one other active agent. The amount of each cardiovascular agent and/or other active agent present in the compositions may vary depending on a number of variables such as age, weight, gender, and health related issues. In general, the dosage of the cardiovascular agents will generally be in the range of about 0.01 ng to about 10 g per kg body weight, specifically in the range of about 1 ng to about 0.1 g per kg, and more specifically in the range of about 100 ng to about 10 mg per kg. When the other cardiovascular agent is an ACE inhibitor, the amount of the ACE inhibitor may be anywhere from 0.5 mg to about 80 mg. When the other cardiovascular agent is an ARB, the amount of ARB may be anywhere from about 1 mg to about 1200 mg. The amount of the other cardiovascular agent will depend in part on the particular cardiovascular agent used.

In addition to ACE inhibitors and ARBs, additional cardiovascular agents include, but are not limited to adrenergic blockers, adrenergic agonists, agents for pheochromocytoma, antianginal agents, antiarrhythmics, antiplatelet agents, anticoagulants, antihypertensives, antilipemic agents, antidiabetics, antiinflammatory agents, calcium channel blockers, CETP inhibitors, COX-2 inhibitors, direct thrombin inhibitors, diuretics, endothelin receptor antagonists, HMG Co-A reductase inhibitors, inotropic agents, rennin inhibitors, vasodilators, vasopressors, AGE crosslink breakers (advanced glycosylation end-product crosslink breakers, such as alagebrium, see USP 6,458,819), and AGE formation inhibitors (advanced glycosylation end-product formation inhibitors, such as pimagedine). Cardiovascular agents falling within these general categories are exemplified by the following:

### "Angiotensin I Converting Enzymes (ACE's) and Angiotensin II Receptor Antagonists (ARB's)"

"Angiotensin II receptor antagonists"(ARB's) are compounds which interfere with the activity of angiotensin II by binding to angiotensin II receptors and interfering with its activity. Angiotensin I and angiotensin II are synthesized by the enzymatic renin-angiotensin pathway. The synthetic process is initiated when the enzyme renin acts on angiotensinogen, a pseudoglobulin in blood plasma, to produce the decapeptide angiotensin I. Angiotensin I is converted by angiotensin converting enzyme (ACE) to angiotensin II (angiotensin-[1-8] octapeptide). The latter is an active pressor substance which has been implicated as a causative agent in several forms of hypertension in various mammalian species, e.g., humans.

Angiotensin II receptor antagonists (ARB's) are well known and include peptide compounds and non-peptide compounds. Most angiotensin II receptor antagonists are slightly modified congeners in which agonist activity is attenuated by replacement of phenylalanine in position 8 with some other amino acid; stability can be enhanced by other replacements that slow degeneration in vivo.

Examples of angiotensin II receptor antagonists include: telmisartan; olmesartan; peptidic compounds (e.g., saralasin and related analogs); N-substituted imidazole-2-one (U.S. Pat. No. 5,087,634); imidazole acetate derivatives including 2-N-butyl-4-chloro-1-(2-chlorobenzile) imidazole-5-acetic acid (see Long et al., J. Pharmacol. Exp. Ther. 247(1), 1-7 (1988)); 4,5,6,7-tetrahydro-1H-imidazo [4,5-c]pyridine-6-carboxylic acid and analog derivatives (U.S. Pat. No. 4,816,463); N2-tetrazole beta-glucuronide analogs (U.S. Pat. No. 5,085,992); substituted pyrroles, pyrazoles, and tryazoles (U.S. Pat. No. 5,081,127); phenol and heterocyclic derivatives such as 1,3-imidazoles (U.S. Pat. No. 5,073,566); imidazo-fused 7-member ring heterocycles (U.S. Pat. No. 5,064,825); peptides (e.g., U.S. Pat. No. 4,772,684); antibodies to angiotensin II (e.g., U.S. Pat. No. 4,302,386); and aralkyl imidazole compounds such as biphenyl-methyl substituted imidazoles (e.g., EP 253,310, Jan. 20,1988); ES8891 (N-morpholinoacetyl-(-1-naphthyl)-L-alanyl-(4, thiazolyl)-L-alanyl (35, 45)-4-amino-3-hydroxy-5-cyclo-hexapentanoyl-N-hexylamide, Sankyo Company, Ltd., Tokyo, Japan); SKF108566 (E-alpha-2-[2-butyl-1-(carboxy phenyl) methyl] 1H-imidazole-5-yl[methylane]-2-thiophenepropanoic acid, Smith Kline Beecham Pharmaceuticals, Pa.); Losartan (DUP753/MK954, DuPont Merck Pharmaceutical Company); Remikirin (RO42-5892, F. Hoffman LaRoche A G); A.sub.2 agonists (Marion Merrill Dow) and certain non-peptide heterocycles (G. D. Searle and Company). Other non-limiting examples of ARBs include candesartan, eprosartan, irbesartan, losartan, and valsartan. Other ARBs may be identified using standard assaying techniques known to one of ordinary skill in the art.

"Angiotensin converting enzyme" (ACE) is an enzyme which catalyzes the conversion of angiotensin I to angiotensin II. ACE inhibitors include amino acids and derivatives thereof, peptides, including di- and tri-peptides and antibodies to ACE which intervene in the renin-angiotensin system by inhibiting the activity of ACE thereby reducing or eliminating the formation of pressor substance angiotensin II. ACE inhibitors have been used medically to treat hypertension, congestive heart failure, myocardial infarction and renal disease. Classes of compounds known to be useful as ACE inhibitors include acylmercapto and mercaptoalkanoyl prolines such as captopril (U.S. Pat. No. 4,105,776) and zofenopril (U.S. Pat. No. 4,316,906), carboxyalkyl dipeptides such as enalapril (U.S. Pat. No. 4,374,829), lisinopril (U.S. Pat. No. 4,374,829), quinapril (U.S. Pat. No. 4,344,949), ramipril (U.S. Pat. No. 4,587,258), and perindopril (U.S. Pat. No. 4,508,729), carboxyalkyl dipeptide mimics such as cilazapril (U.S. Pat. No. 4,512,924) and benazepril (U.S. Pat. No. 4,410,520), phosphinylalkanoyl prolines such as fosinopril (U.S. Pat. No. 4,337,201) and trandolapril. Other non-limiting examples of ACE inhibitors include, but are not limited to, alacepril, benazepril, captopril, ceronapril, cilazapril, delapril, enalapril, enalaprilat, fosinopril, imidapril, lisinopril, perindopril, quinapril, ramipril, ramiprilat, spirapril, temocapril, trandolapril.

### OTHER ACTIVE AGENTS ALSO INCLUDE THE FOLLOWING:

### Adrenergic Blockers

Non-limiting examples of adrenergic blockers, both α- and β-adrenergic blockers, that may be used in the compositions of the present invention include Beta-adrenergic receptor blockers include, but are not limited to, atenolol, acebutolol, alprenolol, befunolol, betaxolol, bunitrolol, carteolol, celiprolol, hedroxalol, indenolol, labetalol, levobunolol, mepindolol, methypranol, metindol, metoprolol, metrizoranolol, oxprenolol, pindolol, propranolol, practolol, sotalolnadolol, tiprenolol, tolamolol, timolol, bupranolol, penbutolol, trimepranol, yohimbine, 2-(3-(1,1-dimethylethyl)-amino-2-hydroxypropoxy)-3-pyridenecarbonitrilHCl, 1-butylamino-3-(2,5-dichlorophenoxy)-2-propanol, 1-isopropylamino-3-(4-(2-cyclopropylmethoxyethyl)phenoxy)-2-propanol, 3-isopropylamino-1-(7-methylindan-4-yloxy)-2-butanol, 2-(3-t-butylamino-2-hydroxy-propylthio)-4-(5-carbamoyl-2-thienyl)thiazole, 7-(2-hydroxy-3-t-butylaminpropoxy)phthalide. The above-identified compounds can be used as isomeric mixtures, or in their respective levorotating or dextrorotating form.

### Adrenergic Agonists

Non-limiting examples of adrenergic agonists, both α- and β-adrenergic agonists, that may be used in the compositions of the present invention include adrafinil, adrenalone, albuterol, amidephrine, apraclonidine, bitolterol, budralazine, carbuterol, clenbuterol, clonidine, clorprenaline, clonidine, cyclopentamine, denopamine, detomidine, dimetofrine, dioxethedrine, dipivefrin, dopexamine, ephedrine, epinephrine, etafedrine, ethylnorepinephrine, fenoterol, fenoxazoline, formoterol, guanabenz, guanfacine, hexoprenaline, hydroxyamphetamine, ibopamine, indanazoline, isoetharine, isometheptene, isoproterenol, mabuterol, mephentermine, metaproterenol, metaraminol, metizoline, methoxamine, methylhexaneamine, methoxyphenamine, midodrine, modafinil, moxonidine, naphazoline, norepinephrine norfenefrine, octodrine, octopamine, oxyfedrine, oxymetazoline, phenylephrine hydrochloride, phenylpropanolamine hydrochloride, phenylpropylmethylamine, pholedrine, pirbuterol prenalterol, procaterol, propylhexedrine, protokylol, pseudoephedrine, reproterol, rilmenidine, rimiterol, ritodrine, salmeterol, solterenol, synephrine, talipexole, terbutaline, tetrahydrozoline, tiamenidine, tramazoline, tretoquinol, tuaminoheptane, tulobuterol, tymazoline, tyramine, xamoterol, xylometazoline, and mixtures thereof.

### Agents for Pheochromocytoma

Include but are not limited to chemotherapeutics.

### Antianginal agents

Include but are not limited to amlodipine besylate, amlodipine maleate, betaxolol hydrochloride, bevantolol hydrochloride, butoprozine hydrochloride, carvedilol, cinepazet maleate, metoprolol succinate, molsidomine, monatepil maleate, nitrates (including but not limited to glyceryl trinitrate (GTN, nitroglycerin, Nitro-Bid), isosorbide-5-mononitrate (5-ISMN, Ismo), amyl nitrate and nicorandil (Icorel)), primidolol, ranolazine hydrochloride, tosifen, verapamil hydrochloride).

### Antiarrhythmics

Non-limiting examples of antiarrhythmics that may be used in the compositions of the present invention include acebutolol, acecainide, adenosine, ajmaline, alprenolol, amiodarone, amoproxan, aprindine, aprotinolol, atenolol, azimilide, bevantolol, bidisomide, bretylium tosylate, bucumolol, butetolol, bunaftine, bunitrolol, bupranolol, butidrine hydrochloride, butobendine, capobenic acid, carazolol, carteolol, cifenline, cloranolol, disopyramide, dofetilide, encainide, esmolol, flecainide, hydroquinidine, ibutilide, indecainide, indenolol, ipratropium bromide, lidocaine, lorajmine, lorcainide, meobentine, mexiletine, moricizine, nadoxolol, nifenaolol, oxprenolol, penbutolol, pentisomide, pilsicainide, pindolol, pirmenol, practolol, prajmaline, procainamide hydrochloride, pronethalol, propafenone, propranolol, pyrinoline, quinidine, sematilide, sotalol, talinolol, tilisolol, timolol, tocainide, verapamil, viquidil, xibenolol, and mixtures thereof.

### Antiplatelet Agents

Non-limiting examples of antiplatelet agents that may be used in the compositions of the present invention include clopidogrel, dipyridamole, abciximab, and ticlopidine.

### Anticoagulants

Anti-coagulant agents are agents which inhibit the coagulation pathway by impacting negatively upon the production, deposition, cleavage and/or activation of factors essential in the formation of a blood clot. Non-limiting examples of anticoagulants (i.e. coagulation-related therapy) that may be used in the compositions of the present invention include Aggrenox, Agrylin, Amicar, Anturane, Arixtra, Coumadin, Fragmin, Heparin Sodium, Lovenox, Mephyton, Miradon, Persantine, Plavix, Pletal, Ticlid, Trental, Warfarin. Other "anti-coagulant" and/or "fibrinolytic" agents include Plasminogen (to plasmin via interactions of prekallikrein, kininogens, Factors XII, XIIIa, plasminogen proactivator, and tissue plasminogen activator[TPA]) Streptokinase; Urokinase: Anisoylated Plasminogen-Streptokinase Activator Complex; Pro-Urokinase; (Pro-UK); rTPA (alteplase or activase; r denotes recombinant); rPro-UK; Abbokinase; Eminase; Streptase Anagrelide Hydrochloride; Bivalirudin; Dalteparin Sodium; Danaparoid Sodium; Dazoxiben Hydrochloride; Efegatran Sulfate; Enoxaparin Sodium; Ifetroban; Ifetroban Sodium; Tinzaparin Sodium; reteplase; Trifenagrel; Warfarin; Dextrans.

Still other anti-coagulant agents include, but are not limited to, Ancrod; Anticoagulant Citrate Dextrose Solution; Anticoagulant Citrate Phosphate Dextrose Adenine Solution; Anticoagulant Citrate Phosphate Dextrose Solution; Anticoagulant Heparin Solution; Anticoagulant Sodium Citrate Solution; Ardeparin Sodium; Bivalirudin; Bromindione; Dalteparin Sodium; Desirudin; Dicumarol; Heparin Calcium; Heparin Sodium; Lyapolate Sodium; Nafamostat Mesylate; Phenprocoumon; Tinzaparin Sodium.

Inhibitors of platelet function are agents that impair the ability of mature platelets to perform their normal physiological roles (i.e., their normal function). Platelets are normally involved in a number of physiological processes such as adhesion, for example, to cellular and non-cellular entities, aggregation, for example, for the purpose of forming a blood clot, and release of factors such as growth factors (e.g., platelet-derived growth factor (PDGF)) and platelet granular components. One subcategory of platelet function inhibitors are inhibitors of platelet aggregation which are compounds which reduce or halt the ability of platelets to associate physically with themselves or with other cellular and non-cellular components, thereby precluding the ability of a platelet to form a thrombus.

Examples of useful inhibitors of platelet function include but are not limited to acadesine, anagrelide (if given at doses exceeding 10 mg/day), anipamil, argatroban, aspirin, clopidogrel, cyclooxygenase inhibitors such as nonsteroidal anti-inflammatory drugs and the synthetic compound FR-122047, danaparoid sodium, dazoxibenhydrochloride, diadenosine 5',5"'-P1,P4-tetraphosphate (Ap4A) analogs, difibrotide, dilazep dihydrochloride, 1,2- and 1,3-glyceryl dinitrate, dipyridamole, dopamine and 3-methoxytyramine, efegatran sulfate, enoxaparin sodium, glucagon, glycoprotein IIb/IIIa antagonists such as Ro-43-8857 and L-700,462, ifetroban, ifetroban sodium, iloprost, Integrilin (eptifibatide), isocarbacyclin methyl ester, isosorbide-5-mononitrate, itazigrel, ketanserin and BM-13.177, lamifiban, lifarizine, molsidomine, nifedipine, oxagrelate, PGE, platelet activating factor antagonists such as lexipafant, prostacyclin (PGI₂), pyrazines, pyridinol carbamate, ReoPro (i.e., abciximab), sulfinpyrazone, synthetic compounds BN-50727, BN-52021, CV-4151, E-5510, FK-409, GU-7, KB-2796, KBT-3022, KC-404, KF-4939, OP-41483, TRK-100, TA-3090, TFC-612 and ZK-36374, 2,4,5,7-tetrathiaoctane, 2,4,5,7-tetrathiaoctane 2,2-dioxide, 2,4,5-trithiahexane, theophylline, pentoxifylline, thromboxane and thromboxane synthetase inhibitors such as picotamide and sulotroban, ticlopidine, tirofiban, trapidil and ticlopidine, trifenagrel, trilinolein, 3-substituted 5,6-bis(4-methoxyphenyl)-1,2,4-triazines, and antibodies to glycoprotein IIb/IIIa as well as those disclosed in U.S. Pat. No. 5,440,020, and anti-serotonin drugs, Clopidogrel; Sulfinpyrazone; Aspirin; Dipyridamole; Clofibrate; Pyridinol Carbamate; PGE; Glucagon; Antiserotonin drugs; Caffeine; Theophylline Pentoxifylline; Ticlopidine.

### Antihypertensives

Non-limiting examples of antihypertensives that may be used in the compositions of the present invention include amlodipine, benidipine, benazepril, candesartan, captopril, darodipine, diltiazem HCl, diazoxide, doxazosin HCl, enalapril, eprosartan, losartan mesylate, felodipine, fenoldopam, fosinopril, guanabenz acetate, irbesartan, isradipine, lisinopril, mecamylamine, minoxidil, nicardipine HCl, nifedipine, nimodipine, nisoldipine, phenoxybenzamine HCl, prazosin HCl, quinapril, reserpine, terazosin HCl, telmisartan, and valsartan.

### Antilipemic Agents

Non-limiting examples of antilipemic agents that may be used in the compositions of the present invention include acipimox, aluminum nicotinate, atorvastatin, cholestyramine resin, colestipol, polidexide, beclobrate, fluvastatin, gemfibrozil, lovastatin, lysosomal acid lipase, icofibrate, niacin; PPARα agonist such as fibrates, which include, but are not limited to fenofibrate, clofibrate, pirifibrate, ciprofibrate, bezafibrate, clinofibrate, ronifibrate, theofibrate, clofibric acid, etofibrate,and gemfibrozil; pravastatin sodium, simfibrate, simvastatin, niceritrol, nicoclonate, nicomol. oxiniacic acid, etiroxate, thyropropic acid, thyroxine, acifran, azacosterol, benfluorex, beta-benzalbutyramide, carnitine, chondroitin sulfate clomestrone, detaxtran, dextran sulfate sodium, 5, 8, 11, 14, 17-eicosapentaenoic acid, eritadenine, furazabol, meglutol, melinamide, mytatrienediol, ornithine, gamma-oryzanol, pantethine, pentaerythritol tetraacetate, alpha-phenylbutyramide, pirozadil, probucol, beta-sitosterol, sultosilic acid (piperazine salt), tiadenol, triparanol, xenbucin, and mixtures thereof.

### Antidiabetics

Non-limiting examples of antidiabetics that may be used in the compositions of the present invention include biguanides such as buformin, metformin, and phenformin; hormones such as insulin; sulfonylurea derivatives such as acetohexamide, 1-butyl-3-metanilylurea, carbutamide, chlorpropamide, glibornuride, gliclazide, glimepiride, glipizide, gliquidone, glisoxepid, glyburide, glybuthiazole, glybuzole, glyhexamide, glymidine, glypinamide, phenbutamide, tolazamide, tolbutamide, tolcyclamide; HDL agonists; PPARγ agonists such as thiazolidinediones such as pioglitazone, rosiglitazone, and troglitazone; and others including acarbose, calcium mesoxalate, miglitol, and repaglinide.

### Antiinflammatory Agents

Non-limiting examples of antiinflammatory agents that may be used in the compositions of the present invention include Alclofenac; Alclometasone Dipropionate; Algestone Acetonide; Alpha Amylase; Amcinafal; Amcinafide; Amfenac Sodium; Amiprilose Hydrochloride; Anakinra; Anirolac; Anitrazafen; Apazone; Balsalazide Disodium; Bendazac; Benoxaprofen; Benzydamine Hydrochloride; Bromelains; Broperamole; Budesonide; Carprofen; Cicloprofen; Cintazone; Cliprofen; Clobetasol Propionate; Clobetasone Butyrate; Clopirac; Cloticasone Propionate; Cormethasone Acetate; Cortodoxone; Deflazacort; Desonide; Desoximetasone; Dexamethasone Dipropionate; Diclofenac Potassium; Diclofenac Sodium; Diflorasone Diacetate; Diflumidone Sodium; Diflunisal; Difluprednate; Diftalone; Dimethyl Sulfoxide; Drocinonide; Endrysone; Enlimomab; Enolicam Sodium; Epirizole; Etodolac; Etofenamate; Felbinac; Fenamole; Fenbufen; Fenclofenac; Fenclorac; Fendosal; Fenpipalone; Fentiazac; Flazalone; Fluazacort; Flufenamic Acid; Flumizole; Flunisolide Acetate; Flunixin; Flunixin Meglumine; Fluocortin Butyl; Fluorometholone Acetate; Fluquazone; Flurbiprofen; Fluretofen; Fluticasone Propionate; Furaprofen; Furobufen; Halcinonide; Halobetasol Propionate; Halopredone Acetate; Ibufenac; Ibuprofen; Ibuprofen Aluminum; Ibuprofen Piconol; llonidap; Indomethacin; Indomethacin Sodium; Indoprofen; Indoxole; Intrazole; Isoflupredone Acetate; Isoxepac; Isoxicam; Ketoprofen; Lofemizole Hydrochloride; Lornoxicam; Loteprednol Etabonate; Meclofenamate Sodium; Meclofenamic Acid; Meclorisone Dibutyrate; Mefenamic Acid; Mesalamine; Meseclazone; Methylprednisolone Suleptanate; Morniflumate; Nabumetone; Naproxen; Naproxen Sodium; Naproxol; Nimazone; Olsalazine Sodium; Orgotein; Orpanoxin; Oxaprozin; Oxyphenbutazone; Paranyline Hydrochloride; Pentosan Polysulfate Sodium; Phenbutazone Sodium Glycerate; Pirfenidone; Piroxicam; Piroxicam Cinnamate; Piroxicam Olamine; Pirprofen; Prednazate; Prifelone; Prodolic Acid; Proquazone; Proxazole; Proxazole Citrate; Rimexolone; Romazarit; Salcolex; Salnacedin; Salsalate; Salicylates; Sanguinarium Chloride; Seclazone; Sermetacin; Sudoxicam; Sulindac; Suprofen; Talmetacin; Talniflumate; Talosalate; Tebufelone; Tenidap; Tenidap Sodium; Tenoxicam; Tesicam; Tesimide; Tetrydamine; Tiopinac; Tixocortol Pivalate; Tolmetin; Tolmetin Sodium; Triclonide; Triflumidate; Zidometacin; Glucocorticoids; Zomepirac Sodium. One preferred antiinflammatory agent is aspirin.

### Calcium Channel Blockers

Calcium channel blockers are a chemically diverse class of compounds having important therapeutic value in the control of a variety of diseases including several cardiovascular disorders, such as hypertension, angina, and cardiac arrhythmias (Fleckenstein, Cir. Res. v. 52, (suppl. 1), p. 13-16 (1983); Fleckenstein, Experimental Facts and Therapeutic Prospects, John Wiley, New York (1983); McCall, D., Curr Pract Cardiol, v. 10, p. 1-11 (1985)). Calcium channel blockers are a heterogeneous group of drugs that prevent or slow the entry of calcium into cells by regulating cellular calcium channels. (Remington, The Science and Practice of Pharmacy, Nineteenth Edition, Mack Publishing Company, Eaton, Pa., p.963 (1995)). Most of the currently available calcium channel blockers, and useful according to the present invention, belong to one of three major chemical groups of drugs, the dihydropyridines, such as nifedipine, the phenyl alkyl amines, such as verapamil, and the benzothiazepines, such as diltiazem. Non-limiting examples of calcium channel blockers that may be used in the compositions of the present invention include bepridil, clentiazem, diltiazem, fendiline, gallopamil, mibefradil, prenylamine, semotiadil, terodiline, verapamil, amlodipine, aranidipine, barnidipine, benidipine, cilnidipine, efonidipine, elgodipine, felodipine, isradipine, lacidipine, lercanidipine, manidipine, nicardipine, nifedipine, nilvadipine, nimodipine, nisoldipine, nitrendipine, cinnarizine, flunarizine, lidoflazine, lomerizine, bencyclane, etafenone, fantofarone, perhexiline, and mixtures thereof.

### CETP Inhibitors

A non-limiting example of a CETP inhibitor that may be used in the compositions of the present invention includes torcetrapib and JT7-705.

### COX-2 Inhibitors

Non-limiting examples of COX-2 inhibitors that may be used in the compositions of the present invention include compounds according to the following: all of the compounds and substances beginning on page 8 of Winokur WO99/20110 as members of three distinct structural classes of selective COX-2 inhibitor compounds, and the compounds and substances which are selective COX-2 inhibitors in Nichtberger, U.S. Pat. No. 6,136,804, Oct. 24, 2000, entitled "Combination therapy for treating, preventing, or reducing the risks associated with acute coronary ischemic syndrome and related conditions", and the compounds and substances which are selective COX-2 inhibitors in Isakson et al, PCT application WO/09641645 published Dec. 27, 1996, filed as PCT/US 9509905 on Jun. 12, 1995, entitled "Combination of a Cyclooxygenase-2 Inhibitor and a Leukotriene B4 Receptor Antagonist for the Treatment of Inflammations." The meaning of COX-2 inhibitor in this invention shall include the compounds and substances referenced and incorporated into Winokur WO99/20110 by reference to art therein, the compounds and substances referenced and incorporated into Nichtberger, U.S. Pat. No. 6,136,804, Oct. 24, 2000, by reference to art therein, and the compounds and substances which are COX-2 inhibitors referenced and incorporated into Isakson et al, PCT application WO/09641645 published Dec. 27, 1996, filed as PCT/US 9509905 on Jun. 12, 1995, entitled "Combination of a Cyclooxygenase-2 Inhibitor and a Leukotriene B4 Receptor Antagonist for the Treatment of Inflammations." The meaning of COX-2 inhibitor in this invention also includes rofecoxib, and celecoxib, marketed as VIOXX and CELEBREX by Merck and Searle/Pfizer respectively. Rofecoxib is discussed in Winokur, WO99/20110 as compound 3, on p.9. Celecoxib is discussed as SC-58635 in the same reference, and in T. Penning, Synthesis and biological evaluation of the 1,5-diarylpyrazole class of cyclooxygenase-2 inhibitors: identification of 4-[5-(4-methylphenyl)-3-(trifluoromethyl)-1H-pyrozol-1-yl]benzenesulfonami de (SC-58635, celecoxib)", J. Med. Chem. Apr. 25, 1997: 40(9): 1347-56. The meaning of COX-2 inhibitor in this invention also includes SC299 referred to as a fluorescent diaryloxazole. C. Lanzo et al, "Fluorescence quenching analysis of the association and dissociation of a diarylheterocycle to cyclooxygenasel-1 and cyclooxygenase-2: dynamic basis of cycloxygenase-2 selectivity", Biochemistry May 23, 2000, vol. 39(20):6228-34, and in J. Talley et al, "4,5-Diaryloxazole inhibitors of cyclooxygenase-2 (COX-2)", Med. Res. Rev. May 1999; 19(3): 199-208. The meaning of COX-2 inhibitor in this invention also includes valdecoxib, See, "4-[5-Methyl-3-phenylisoxazol-1-yl]benzenesulfonamide, Valdecoxib: A Potent and Selective Inhibitor of COX-2", J. Med. Chem. 2000, Vol. 43: 775-777, and parecoxib, sodium salt or parecoxib sodium, See, N-[[(5-methyl-3-phenylixoxazol-4yl)-phenyl]sulfonyl]propanimide, Sodium Salt, Parecoxib Sodium: A Potent and Selective Inhibitor of COX-2 for Parenteral Administration", J. Med. Chem. 2000, Vol. 43: 1661-1663. The meaning of COX-2 inhibitor in this invention also includes the substitution of the sulfonamide moiety as a suitable replacement for the methylsulfonyl moiety. See, J. Carter et al, Synthesis and activity of sulfonamide-substituted 4,5-diaryl thiazoles as selective cyclooxygenase-2 inhibitors." Bioorg. Med. Chem. Lett Apr. 19, 1999: Vol. 9(8): 1171-74, and compounds referenced in the article "Design and synthesis of sulfonyl-substituted 4,5-diarylthiazoles as selective cyclooxygenase-2 inhibitors", Bioorg. Med. Chem. Lett Apr. 19, 1999: Vol. 9(8): 1167-70. The meaning of this invention includes a COX-2 inhibitor, NS398 referenced in two articles: Attiga et al, "Inhibitors of Prostaglandin Synthesis Inhibit Human Prostate Tumor Cell Invasiveness and Reduce the Release of Matrix Metalloproteinases", 60 Cancer Research 4629-4637, Aug. 15, 2000, and in "The cyclooxygenase-2 inhibitor celecoxib induces apoptosis by blocking Akt activation in human prostate cancer cells independently of Bcl-2," Hsu et al, 275(15) J. Biol. Chem. 11397-11403 (2000). The meaning of COX-2 inhibitor in this invention includes the cyclo-oxygenase-2 selective compounds referenced in Mitchell et al, "Cyclo-oxygenase-2: pharmacology, physiology, biochemistry and relevance to NSAID therapy", Brit. J. of Pharmacology (1999) vol.128: 1121-1132, see especially p. 1126. The meaning of COX-2 inhibitor in this invention includes so-called NO-NSAIDs or nitric oxide-releasing-NSAIDs referred to in L. Jackson et al, "COX-2 Selective Nonsteroidal Anti-Inflammatory Drugs: Do They Really Offer Any Advantages?", Drugs, June, 2000 vol. 59(6): 1207-1216 and the articles at footnotes 27, and 28. Also included in the meaning of COX-2 inhibitor in this invention includes any substance that selectively inhibits the COX-2 isoenzyme over the COX-1 isoenzyme in a ratio of greater than 10 to 1 and preferably in ratio of at least 40 to 1 as referenced in Winokur WO 99/20110, and has one substituent having both atoms with free electrons under traditional valence-shell-electron-pair-repulsion theory located on a cyclic ring (as in the sulfylamine portion of celecoxib), and a second substituent located on a different ring sufficiently far from said first substituent to have no significant electron interaction with the first substituent. The second substituent should have an electronegativity within such substituent greater than 0.5, or the second substituent should be an atom located on the periphery of the compound selected from the group of a halogen F, Cl, Br or I, or a group VI element, S or O. Thus for purposes of this last included meaning of a COX-2 inhibitor, one portion of the COX-2 inhibitor should be hydrophilic and the other portion lipophilic. Also included as a COX-2 inhibitor are compounds listed at page 553 in Pharmacotherapy: A Pathophysiologic Approach, Depiro et al (McGraw Hill 1999) including nabumetone and etodolac. Recognizing that there is overlap among the selective COX-2 inhibitors set out in this paragraph, the intent of the term COX-2 inhibitor is to comprehensively include all selective COX-2 inhibitors, selective in the sense of inhibiting COX-2 over COX-1. The inventors add to the class of COX-2 inhibitors useful in the invention the drug bearing the name etoricoxib referenced in the Wall Street Journal, Dec. 13,2000, manufactured by Merck. See, also, Chauret et al., "In vitro metabolism considerations, including activity testing of metabolites, in the discovery and selection of the COX-2 inhibitor etoricoxib (MK-0663)," Bioorg. Med. Chem. Lett. 11(8): 1059-62 (Apr. 23, 2001). Another selective COX-2 inhibitor is DFU [5,5-dimethyl-3-(3-fluorophenyl)-4-(4-methylsulphonyl) phenyl-2(5H)-furanone] referenced in Yergey et al, Drug Metab. Dispos. 29(5):638-44 (May 2001). The inventors also include as a selective COX-2 inhibitor the flavonoid antioxidant silymarin, and an active ingredient in silymarin, silybinin, which demonstrated significant COX-2 inhibition relative to COX-1 inhibition. The silymarin also showed protection against depletion of glutathione peroxidase. Zhao et al, "Significant Inhibition by the Flavonoid Antioxidant Silymarin against 12-O-tetracecanoylphorbol 13-acetate-caused modulation of antioxidant and inflammatory enzymes, and cyclooxygenase 2 and interleukin-1 alpha expression in SENCAR mouse epidermis: implications in the prevention of stage I tumor promotion," Mol. Carcinog. December 1999, Vol 26(4):321-33 PMID 10569809. Silymarin has been used to treat liver diseases in Europe.

A number of the above-identified COX-2 inhibitors are prodrugs of selective COX-2 inhibitors, and exert their action by conversion in vivo to the active and selective COX-2 inhibitors. The active and selective COX-2 inhibitors formed from the above-identified COX-2 inhibitor prodrugs are described in detail in WO 95/00501, published Jan. 5, 1995, WO 95/18799, published Jul. 13, 1995 and U.S. Pat. No. 5,474,995, issued Dec. 12, 1995. Given the teachings of U.S. Pat. No. 5,543,297, entitled: "Human cyclooxygenase-2 cDNA and assays for evaluating cyclooxygenase-2 activity," a person of ordinary skill in the art would be able to determine whether an agent is a selective COX-2 inhibitor or a precursor of a COX-2 inhibitor, and therefore part of the present invention.

### "Direct thrombin inhibitors"

Non limiting examples of direct thrombin inhibitors include hirudin, hirugen, Hirulog, argatroban, PPACK, and thrombin aptamers.

### Diuretics

Non-limiting examples of diuretics that may be used in the compositions of the present invention include althiazide, bendroflumethiazide, benzthiazide, buthiazide, chlorthalidone, cyclopenthiazide, cyclothiazide, epithiazide, ethiazide, fenquizone, indapamide, hydroflumethiazide, methyclothiazide, meticrane, metolazone, paraflutizide, polythiazide, quinethazone, teclothiazide, trichloromethiazide, chlormerodrin, meralluride, mercamphamide, mercaptomerin sodium, mercumatilin sodium, mercurous chloride, mersalyl, acefylline, 7-morpholinomethyl-theophylline, pamabrom, protheobromine, theobromine, canrenone, oleandrin, spironolactone, acetazolamide, ambuside, azosemide, bumetanide, butazolamide, clopamide, clorexolone, disulfamide, ethoxzolamide, furosemide, mefruside, methazolamide, piretanide, torsemide, tripamide, xipamide, aminometradine, amisometradine, amanozine, amiloride, arbutin, chlorazanil, ethacrynic acid, etozolin, hydracarbazine, isosorbide, mannitol, metochalcone, muzolimine, perhexiline, ticrynafen, triamterene, urea, and mixtures thereof. Depending on the diuretic employed, potassium may also be administered to the patient in order to optimize the fluid balance while avoiding hypokalemic alkalosis. The administration of potassium can be in the form of potassium chloride or by the daily ingestion of foods with high potassium content such as, for example, bananas or orange juice.

### Endothelin Receptor Antagonists

Non-limiting examples of an endothelin receptor antagonist that may be used in the compositions of the present invention include bosentan, sulfonamide endothelin antagonists, BQ-123, SQ 28608, and the like); and mixtures thereof.

### HMG-CoA Reductase Inhibitor (Statins)

HMG-CoA (3-hydroxy-3-methylglutaryl-coenzyme A) reductase is the microsomal enzyme that catalyzes the rate limiting reaction in cholesterol biosynthesis (HMG-CoA6Mevalonate). An HMG-CoA reductase inhibitor inhibits HMG-CoA reductase, and as a result inhibits the synthesis of cholesterol. A number of HMG-CoA reductase inhibitors have been used to treat individuals with hypercholesterolemia. More recently, HMG-CoA reductase inhibitors have been shown to be beneficial in the treatment of stroke (Endres M, et al., Proc Natl Acad Sci USA, 1998, 95:8880-5).

HMG-CoA reductase inhibitors useful for co-administration with the agents of the invention include, but are not limited to, simvastatin (U.S. Pat. No. 4,444,784), lovastatin (U.S. Pat. No. 4,231,938), pravastatin sodium (U.S. Pat. No. 4,346,227), fluvastatin (U.S. Pat. No. 4,739,073), atorvastatin (U.S. Pat. No. 5,273,995), cerivastatin, and numerous others described in U.S. Pat. Nos. 5,622,985; 5,135,935; 5,356,896; 4,920,109; 5,286,895; 5,262,435; 5,260,332; 5,317,031; 5,283,256; 5,256,689; 5,182,298; 5,369,125; 5,302,604; 5,166,171; 5,202,327; 5,276,021; 5,196,440; 5,091,386; 5,091,378; 4,904,646; 5,385,932; 5,250,435; 5,132,312; 5,130,306; 5,116,870; 5,112,857; 5,102,911; 5,098,931; 5,081,136; 5,025,000; 5,021,453; 5,017,716; 5,001,144; 5,001,128; 4,997,837; 4,996,234; 4,994,494; 4,992,429; 4,970,231; 4,968,693; 4,963,538; 4,957,940; 4,950,675; 4,946,864; 4,946,860; 4,940,800; 4,940,727; 4,939,143; 4,929,620; 4,923,861; 4,906,657; 4,906,624 and 4,897,402.

Other non-limiting examples of HMG-CoA reductase inhibitors that may be used in the compositions of the present invention include mevastatin, pitavastatin, rosuvastatin, gemcabene, and probucol.

### Inotropic Agents

Non-limiting examples of inotropic agents that may be used in the compositions of the present invention include acefylline, acetyldigitoxins, 2-amino-4-picoline, amrinone, benfurodil hemisuccinate, bucladesine, camphotamide, convallatoxin, cymarin, denopamine, deslanoside, digitalin, digitalis, digitoxin, digoxin, dobutamine, docarpamine, dopamine, dopexamine, enoximone, erythrophleine, fenalsomine, gitalin, gitoxin, glycocyamine, heptaminol, hydrastinine, ibopamine, lanatosides, loprinine, milrinone, nerifolin, oleandrin, ouabain, oxyfedrine, pimobendan, prenalterol, proscillaridin, resibufogenin, scillaren, scillarenin, strophanthin, sulmazole, theobromine, vesnarinone, xamoterol, and mixtures thereof.

### "Renin inhibitors"

Renin inhibitors are compounds which interfere with the activity of renin. Renin inhibitors include amino acids and derivatives thereof, peptides and derivatives thereof, and antibodies to renin. Examples of renin inhibitors that are the subject of United States patents are as follows: urea derivatives of peptides (U.S. Pat. No. 5,116,835); amino acids connected by nonpeptide bonds (U.S. Pat. No. 5,114,937); di- and tri-peptide derivatives (U.S. Pat. No. 5,106,835); amino acids and derivatives thereof (U.S. Pat. Nos. 5,104,869 and 5,095,119); diol sulfonamides and sulfinyls (U.S. Pat. No. 5,098,924); modified peptides (U.S. Pat. No. 5,095,006); peptidyl beta-aminoacyl aminodiol carbamates (U.S. Pat. No. 5,089,471); pyrolimidazolones (U.S. Pat. No. 5,075,451); fluorine and chlorine statine or statone containing peptides (U.S. Pat. No. 5,066,643); peptidyl amino diols (U.S. Pat. Nos. 5,063,208 and 4,845,079); N-morpholino derivatives (U.S. Pat. No. 5,055,466); pepstatin derivatives (U.S. Pat. No. 4,980,283); N-heterocyclic alcohols (U.S. Pat. No. 4,885,292); monoclonal antibodies to renin (U.S. Pat. No. 4,780,401); and a variety of other peptides and analogs thereof (U.S. Pat. Nos. 5,071,837, 5,064,965, 5,063,207, 5,036,054, 5,036,053, 5,034,512, and 4,894,437).

### Vasodilators

Non-limiting examples of vasodilators that may be used in the compositions of the present invention include bencyclane, cinnarizine, citicoline, cyclandelate, ciclonicate, diisopropylamine dichloroacetate, eburnamonine, fasudil, fenoxedil, flunarizine, ibudilast, ifenprodil, isosorbide dinitrate, isosorbide mononitrate, lomerixine, nafronyl, nicametate, nicergoline, nimodipine, papaverine, pentifylline, tinofedrine, vancamine, vinpocetine, viquidil, amotriphene, bendazol, benfurodil hemisuccinate, benziodarone, chloracizine, chromonar, clobenfurol, clonitrate, cloricromen, dilazep, dipyridamole, droprenilamine, efloxate, erythrityl tetranitrate, etafenone, fendiline, floredil, ganglefence, heart muscle extract, hexestrol bis(alpha-diethylaminoethyl ether), hexobendine, hydralazine compound, itramin tosylate khellin, lidoflazine, mannitol hexanitrate, medibazine, nitroglycerin, isosorbide mononitrate, isosorbide dinitrate, and other nitrates, pentaerythritol tetranitrate, pentrinitrol, perhexiline, pimefylline, prenylamine, propatyl nitrate, pyridofylline, trapidil, tricromyl, trimetazidine, trolnitrate phosphate, visnadine, aluminum nicotinate, bamethan, bencyclane, betahistine, bradykinin, brovincamine, bufeniode, buflomedil, butalamine, cetiedil, ciclonicate, cinepazide, cinnarizine, cyclandelate, diisopropylamine dichloroacetate, eledoisin, fenoxedil, flunazine, hepronicate, ifenprodil, iloprost, inositol niacinate, isoxsuprine, kallidin, kallikrein, moxisylyte, nafronyl, nicametate nicergoline, nicofuranose, nicotinyl alcohol, nylidrin, pentifylline, pentoxifylline, piribedil, prostaglandin E1, suloctidil, tolazoline, xanthinol niacinate, and mixtures thereof.

Note that "hydralazine compound" refers to a compound having the formula: 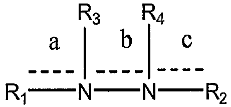 wherein a, b and c are each independently a single or a double bond; R₁ and R₂ are each independently a hydrogen, an alkyl, an ester or a heterocyclic ring; R₃ and R₄ are each independently a lone pair of electrons or a hydrogen, with the proviso that at least one of R₁, R₂, R₃ and R₄ is not a hydrogen. Examples of hydralazine compounds include, but are not limited to budralazine, cadralazine, dihydralazine, endralazine, hydralazine, pildralazine, todralazine and the like.

### Vasopressors

Non-limiting examples of vasopressors that may be used in the compositions of the present invention include amezinium methyl sulfate, angiotensin amide, dimetofrine, dopamine, etifelmin, etilefrin, gepefrine, metaraminol, methoxamine, midodrine, norepinephrine, pholedrine, synephrine, and mixtures thereof.

### AGE Crosslink Breakers (advanced glycosylation end-product crosslink breakers)

Non-limiting examples of AGE crosslink breakers that may be used in the compositions of the present invention include Alagebrium.

### AGE Formation Inhibitors (advanced glycosylation end product formation inhibitors)

Non-limiting examples of AGE formation inhibitors that may be used in the compositions of the present invention include Pimagedine.

### Other actives:

Non-limiting examples of other active ingredients that may be used in the pharmaceutical compositions of the present invention include, but are not limited to, the following representative classes of compounds, as well as their pharmaceutically acceptable salts, isomers, esters, ethers and other derivatives:
*Analgesics and anti-inflammatory agents*, such as aloxiprin, auranofin, azapropazone, benorylate, capsaicin, celecoxib, diclofenac, diflunisal, etodolac, fenbufen, fenoprofen calcium, flurbiprofen, ibuprofen, indomethacin, ketoprofen, ketorolac, leflunomide, meclofenamic acid, mefenamic acid, nabumetone, naproxen, oxaprozin, oxyphenbutazone, phenylbutazone, piroxicam, rofecoxib, sulindac, tetrahydrocannabinol, tramadol and tromethamine;
*antihelminthics*, such as albendazole, bephenium hydroxynaphthoate, cambendazole, dichlorophen, ivermectin, mebendazole, oxamniquine, oxfendazole, oxantel embonate, praziquantel, pyrantel embonate and thiabendazole;
*anti-asthma agents*, such as zileuton, zafirlukast, terbutaline sulfate, montelukast, and albuterol;
*anti-bacterial agents*, such as alatrofloxacin, azithromycin, baclofen, benzathine penicillin, cinoxacin, ciprofloxacin HCl, clarithromycin, clofazimine, cloxacillin, demeclocycline, dirithromycin, doxycycline, erythromycin, ethionamide, furazolidone, grepafloxacin, imipenem, levofloxacin, lorefloxacin, moxifloxacin HCl, nalidixic acid, nitrofurantoin, norfloxacin, ofloxacin, rifampicin, rifabutin, rifapentine, sparfloxacin, spiramycin, sulphabenzamide, sulphadoxine, sulphamerazine, ulphacetamide, sulphadiazine, sulphafurazole, sulphamethoxazole, sulphapyridine, tetracycline, trimethoprim, trovafloxacin, and vancomycin;
*anti-viral agents*, such as abacavir, amprenavir, delavirdine, efavirenz, indinavir, lamivudine, nelfinavir, nevirapine, ritonavir, saquinavir, and stavudine;
*anti-depressants*, such as amoxapine, bupropion, citalopram, clomipramine, fluoxetine HCl, maprotiline HCl, mianserin HCl, nortriptyline HCl, paroxetine HCl, sertraline HCl, trazodone HCl, trimipramine maleate, and venlafaxine HCl;
*anti-epileptics*, such as beclamide, carbamazepine, clonazepam, ethotoin, felbamate, fosphenytoin sodium, lamotrigine, methoin, methsuximide, methylphenobarbitone, oxcarbazepine, paramethadione, phenacemide, phenobarbitone, phenytoin, phensuximide, primidone, sulthiame, tiagabine HCl, topiramate, valproic acid, and vigabatrin;
*anti-fungal agents*, such as amphotericin, butenafine HCl, butoconazole nitrate, clotrimazole, econazole nitrate, fluconazole, flucytosine, griseofulvin, itraconazole, ketoconazole, miconazole, natamycin, nystatin, sulconazole nitrate, oxiconazole, erbinafine HCl, terconazole, tioconazole and undecenoic acid;
*anti-gout agents*, such as allopurinol, probenecid and sulphinpyrazone;
*anti-malarials*, such as amodiaquine, chloroquine, chlorproguanil HCl, halofantrine HCl, mefloquine HCl, proguanil HCl, pyrimethamine and quinine sulfate;
*anti-migraine agents*, such as dihydroergotamine mesylate, ergotamine tartrate, frovatriptan, methysergide maleate, naratriptan HCl, pizotifen maleate, rizatriptan benzoate, sumatriptan succinate, and zolmitriptan;
*anti*-*muscarinic agents*, such as atropine, benzhexol HCl, biperiden, ethopropazine HCl, hyoscyamine, mepenzolate bromide, oxyphencyclimine HCl and tropicamide;
*anti-neoplastic agents and immunosuppressants*, such as aminoglutethimide, amsacrine, azathioprine, bicalutamide, bisantrene, busulfan, camptothecin, capecitabine, chlorambucil, cyclosporin, dacarbazine, ellipticine, estramustine, etoposide, irinotecan, lomustine, melphalan, mercaptopurine, methotrexate, mitomycin, mitotane, mitoxantrone, mofetil mycophenolate, nilutamide, paclitaxel, procarbazine HCl, sirolimus, tacrolimus, tamoxifen citrate, teniposide, testolactone, topotecan HCl, and toremifene citrate;
*anti-protozoal agents*, such as atovaquone, benznidazole, clioquinol, decoquinate, diiodohydroxyquinoline, diloxanide furoate, dinitolmide, furazolidone, metronidazole, nimorazole, nitrofurazone, ornidazole and tinidazole;
*anti-psychotics*, such as aripiprazole, clozapine, ziprasidone, haloperidol, molindone, loxapine, thioridazine, molindone, thiothixene, pimozide, fluphenazine, risperidone mesoridazine, quetiapine, trifluoperazine, chlorprothixene, chlorpromazine, perphenazine, trifluopromazine, olanzapine;
*anti-thyroid agents*, such as carbimazole, paricalcitol, and propylthiouracil;
*anti-tussives*, such as benzonatate;
*anxiolytics, sedatives, hypnotics and neuroleptics*, such as alprazolam, amylobarbitone, barbitone, bentazepam, bromazepam, bromperidol, brotizolam, butobarbitone, carbromal, chlordiazepoxide, chlormethiazole, chlorpromazine, chlorprothixene, clonazepam, clobazam, clotiazepam, clozapine, diazepam, droperidol, ethinamate, fluanisone, flunitrazepam, triflupromazine, flupenthixol decanoate, fluphenthixol decanoate, flurazepam, gabapentin, haloperidol, lorazepam, lormetazepam, medazepam, meprobamate, mesoridazine, methaqualone, methylphenidate, midazolam, molindone, nitrazepam, olanzapine, oxazepam, pentobarbitone, perphenazine pimozide, prochlorperazine, pseudoephedrine, quetiapine, risperidone, sertindole, sulpiride, temazepam, thioridazine, triazolam, zolpidem, and zopiclone;
*corticosteroids,* such as beclomethasone, betamethasone, budesonide, cortisone acetate, desoxymethasone, dexamethasone, fludrocortisone acetate, flunisolide, fluocortolone, fluticasone propionate, hydrocortisone, methylprednisolone, prednisolone, prednisone and triamcinolone;
*anti-parkinsonian agents*, such as apomorphine, bromocriptine mesylate, lisuride maleate, pramipexole, ropinirole HCl, and tolcapone;
*gastrointestinal agents*, such as bisacodyl, cimetidine, cisapride, diphenoxylate HCl, domperidone, famotidine, lansoprazole, loperamide, mesalazine, nizatidine, omeprazole, ondansetron HCL, rabeprazole sodium, ranitidine HCl and sulphasalazine;
*keratolytics*, such as acitretin, calcipotriene, calcifediol, calcitriol, cholecalciferol, ergocalciferol, etretinate, retinoids, Targretin, and tazarotene;
*lipid regulating agents*, such as atorvastatin, bezafibrate, cerivastatin, ciprofibrate, clofibrate, fenofibrate, fluvastatin, gemfibrozil, pravastatin, probucol, and simvastatin;
*muscle relaxants*, such as dantrolene sodium and tizanidine HCl;
*nutritional agents*, such as calcitriol, carotenes, dihydrotachysterol, essential fatty acids, non-essential fatty acids, phytonadiol, vitamin A, vitamin B.sub.2, vitamin D, vitamin E and vitamin K;
*opioid analgesics*, such as codeine, dextropropoxyphene, diamorphine, dihydrocodeine, fentanyl, meptazinol, methadone, morphine, nalbuphine and pentazocine;
*sex hormones*, such as clomiphene citrate, cortisone acetate, danazol, dehydroepiandrosterone, ethynyl estradiol, finasteride, fludrocortisone, fluoxyrnesterone, medroxyprogesterone acetate, megestrol acetate, mestranol, methyltestosterone, norethisterone, norgestrel, oestradiol, conjugated estrogens, progesterone, rimexolone, stanozolol, stilbestrol, testosterone and tibolone;
*stimulants*, such as amphetamine, dexamphetamine, dexfenfluramine, fenfluramine and mazindol;
*drugs for rheumatoid arthritis* such as methotrexate, auranofin, aurothioglucose and gold sodium thiomalate;
*drugs for osteoporosis* such as alendronate and raloxifene;
*local anesthetics*;
*anti-herpes drugs* such as acyclovir, valacyclovir and famciclovir;
*anti-emetics* such as ondansetron and granisetron;
*Flavonoids and Isoflavonoids* which include the anthocyanidins and anthocyanins; proanthocyanidins; flavan-3-ols; flavonols; flavones; flavanones; isoflavanones; salts and esters thereof. This development is however, not limited to flavonoid compounds isolated from plant, part of plant or extracts of Astragalus Membranaceus, but encompasses any suitable flavonoid compound isolated from different sources or chemically synthesized. In addition, any suitable known or not yet discovered flavonoid compound, and isoflavonoid compound, is within the scope of the present technology. A number of flavonoids and isoflavonoids are described in USDA-Iowa State University Database on the Isoflavone Content of Foods, Release 1.3-2002, and in USDA Database for the Flavonoid Content of Selected Foods--2003 (http://www.nal.usda. gov/fnic/foodcomp/Data/isoflav/isoflav.html) and (http://www.nal.usda.gov/fnic/foodcomp/Data/Flav/flav.html) (both of them herewith incorporated by reference). It will be evident to any skilled person how to choose the suitable flavonoid and/or isoflavonoid compound for the purpose of the present development. For example, flavonoid compounds for the purpose of the present development may be, but are not limited to, (-)-epictechin, (+)-catechin, procyanidin B2, quercetin dehydrate, taxifolin, resveratrol, and the like.

*Carotenoids*, which are generally are tetraterpenes originating from the mevalonate and deoxyxylulose phosphate pathways (older sources sometimes refer to their source as the 'isoprenoid' pathway). Two molecules of the C₂₀ compound geranylgeranyldiphosphate (GGDP) condense to form the symmetrical carotenoid skeleton.

Carotenoids are divided into two subclasses, i.e., more polar compounds called xanthophylls, or oxycarotenoids, and the nonpolar hydrocarbon carotenes.

Terms such as carotenoid analog and carotenoid derivative may generally refer to in some embodiments chemical compounds or compositions derived from a naturally occurring carotenoid or simply to synthetic carotenoids. In some embodiments, terms such as carotenoid analog and carotenoid derivative may generally refer to chemical compounds or compositions which are synthetically derived from non-carotenoid based parent compounds; however, which ultimately substantially resemble a carotenoid derived analog. In certain embodiments, terms such as carotenoid analog and carotenoid derivative may generally refer to a synthetic derivative of a naturally occurring carotenoid. Examples of carotenoids are provided in the book "Carotenoids Handbook," edited by G. Britton et al, 2004, which is herein incorporated by reference.

It will be evident to any skilled person how to choose the suitable carotenoid compound for the purpose of the present development.

Examples of carotenoids include astaxanthin, zeaxanthin , lutein, lycopene, beta-carotene.

Other non-limiting examples of naturally occurring carotenoids include:
Aaptopurpurin; Actinioerythrin; Actinioerythrol; Adonirubin; Adonixanthin; A.g.470; A.g.471; Agelaxanthin C; Aleuriaxanthin; Alloxanthin; Amarouciaxanthin A; Amarouciaxanthin B; Anchovyxanthin; 3',4'-Anhydrodiatoxanthin; Anhydrodeoxyflexixanthin; Anhydroeschscholtzxanthin; Anhydrolutein; Anhydroperidinin; Anhydrorhodovibrin; Anhydrosaproxanthin; Anhydrowarmingol; Anhydrowarmingone; Antheraxanthin; Aphanicin; Aphanicol; Aphanin; Aphanol; Aphanizophyll; 8'-Apo-beta-caroten-8'-al; 10'-Apo-beta-caroten-10'-al; 12'-Apo-beta-caroten-12'al; 14'-Apo-beta-caroten-14'-al; 6'-Apo-psi-caroten-6'-al; 8'-Apo-psi-caroten-8'-al; beta-Apo-2-carotenal; beta-Apo-3-carotenal; beta-Apo-4-carotenal; beta-Apo-2'-carotenal; beta-Apo-8'-carotenal; beta-Apo-10'-carotenal; beta-Apo-12'-carotenal; beta-Apo-14'-carotenal; Apo-8,8'-carotenedial; 8'-Apo-beta-carotene-3,8'-diol; 4'-Apo-beta-caroten-4'-oic acid; 8'-Apo-beta-caroten-8'-oic acid; 10'-Apo-beta-caroten-10'-oic acid; 12'-Apo-beta-caroten-12'-oic acid; beta-Apo-2'-carotenoic acid; beta-Apo-2'-carotenoic acid methylester; beta-Apo-8'-carotenoic acid; beta-Apo-10'-carotenoic acid; beta-Apo-12'-carotenoic acid; 8'-Apo-beta- caroten-3-ol; beta-Apo-2'-carotenol; Apo-7-fucoxanthinol; Apo-2- lycopenal; Apo-3-lycopenal; Apo-6' lycopenal; Apo-8'-lycopenal; Apo-10'- violaxanthal; Apo-12'-violaxanthal; Apoviolaxanthinal; Apo-2-zeaxanthinal; Apo-3-zeaxanthinal; Apo-4-zeaxanthinal; Astacein; Astacene; Astacene dipalmitate; Astaxanthin; Asterinic acid; Asteroidenone; Asym. zeta- carotene; Aurochrome; Auroxanthin; Azafrin; Azafrinaldehyde;
Bacterial phytoene; Bacterioerythrin alpha; Bacterioerythrin beta; Bacteriopurpurin alpha; Bacterioruberin; alpha-Bacterioruberin; Bacterioruberin diglycoside; Bacterioruberin monoglycoside; alpha-Bacterioruberin monomethyl ether; Bisanhydrobacterioruberin; 3,4,3',4'-Bisdehydro-beta-carotene; Bisdehydrolycopene; 2,2'-Bis(4-hydroxy-3-methyl-2-butenyl)-beta,beta- carotene; 2,2'-Bis[3-(glucosyloxy)-3-methylbutyl]-3,4,3',4'-tetradehydro-1,2,1',2'-tetrahydro-psi,psi-carotene-1,1'-diol; 2,2'-Bis[4-(beta,D-glucopyranosyloxy)-3-methyl-2-butenyl]-gamma,gamma-carotene; 2,2'-Bis(4-hydroxy-3-methyl-2-butenyl)-gamma,gamma-carotene; 2,2'-Bis(4-hydroxy-3-methyl-2-butenyl)-[epsi],[epsi]-carotene; 2,2'-Bis(3-hydroxy-3-methylbutyl-3,4,3',4'-tetradehydro-1,2,1',2'tetrahydro-psi,psi-carotene-1,1'-diol; 2,2'-Bis(3-methyl-2-butenyl)-[epsi],[epsi]-carotene; 2,2'-Bis(3-methyl-2-butenyl-3,4,3',4'-tetradehydro-1,2-dihydro-psi,psi-caroten-1-ol; 2,2'-Bis(3-methyl-2-butenyl)-3,4,3',4'-tetradehydro-1,2,1',2'-tetrahydro-psi,psi-carotene-1,1'-diol; 2,2'-Bis(3-methyl-2-butenyl)-1,2,1',2'-tetrahydro-psi,psi-carotene-1,1'-diol; 2,2'-Bis(O-methyl-5-C-methylpentosyloxy)-3,4,3',4'-tetradehydro-1,2,1',2'-tetrahydropsi, psi-carotene-1,1'-diol; 3,3'-Bis(rhamnosyloxy)-beta,beta-carotene; 2,2'-Bis(rhamnosyloxy)-3,4,3',4'-tetradehydro-1,2,1',2'-tetrahydro-psi, psi-carotene-1,1'-diol; Bixin;
Caloxanthin; Calthaxanthin; Canthaxanthin; Capsanthin; Capsanthin epoxide; Capsanthinone; Capsanthone; Capsochrome; Capsorubin; Capsorubindione; Capsorubone; Carangoxanthin; 16'-Carboxyl-3',4'-dehydro-gamma-carotene; Carcinoxanthin; Caricaxanthin; beta-Carotenal; psi, psi-Caroten-20-al; Carotene; Carotene X; alpha-Carotene; beta-Carotene; beta,beta-Carotene; beta,gamma-Carotene; beta,[epsi]- Carotene; beta,[phi]-Carotene; beta,psi-Carotene; gamma-Carotene; gamma,gamma-Carotene; gamma,psi-Carotene; [delta]-Carotene; [epsi] -Carotene; [epsi] (1)-Carotene; [epsi],[epsi]-Carotene; [epsi],psi -Carotene; zeta-Carotene; zeta-Carotene, asym.; eta-Carotene; [theta]-Carotene; xi-Carotene; [phi]-Carotene; [phi],[phi]-Carotene; [phi],X-Carotene; [phi],psi-Carotene; X,X-Carotene; psi-Carotene; psi,alpha-Carotene; psi,psi-Carotene; [theta]-Carotene; beta-Carotene-5,6,5',6'-diepoxide; beta-Carotene 5,8,5',8'-diepoxide; beta, beta-Carotene-2,2'-diol; beta,beta-Carotene-2,3-diol; beta,beta- Carotene-3,4-diol; beta,beta-Carotene-3,3'-diol; beta,beta-Carotene-4,4'-diol; beta,[epsi]-Carotene-3,2'-diol; beta,[epsi]-Carotene-3,3'-diol; beta,psi-Carotene-2,3-diol; beta,psi-Carotene-3,3'-diol; [epsi],[epsi]-Carotene-3,3'-diol; [phi],[phi]-Carotene-3,3'-diol; psi,psi-Carotene-16,16'-diol; beta,beta-Carotene-3,3'-diol dipalmitate; beta,[epsi]-Carotene-3,3'-diol dipalmitate; beta,beta-Carotene-2,2'-dione; beta,beta-Carotene-3,4-dione; beta,beta-Carotene-4,4'dione; beta,psi-Carotene-3,4-dione; [epsi],[epsi]-Carotene-3,3'-dione; beta,chi-Carotene-3',6'-dione; beta,X-Carotene-3,4-dione; beta,psi-Carotene-4,4'-dione; beta,[phi]-Carotene-3,4- dione; psi,psi-Carotene-4,4'-dione; alpha-Carotene 5,6-epoxide; beta-Carotene 5,6-epoxide; zeta-Carotene epoxide; Carotene oxide; beta,beta-Carotene-3,4,3',4'-tetrol; beta,beta-Carotene-2,3,2',3'-tetrol; beta,beta-Carotene-3,4,3',4'-tetrone; chi,chi-Carotene-3,6,3',6'-tetrone; beta,beta-Carotene-2,3,2'-triol; beta,beta-Carotene-2,3,3'-triol; beta,beta-Carotene-3,4,3'-triol; beta,beta- Carotene-3,4,4'-triol; beta,[epsi]-Carotene-3,4,3'-triol; beta,[epsi]- Carotene-3,19,3'-triol; beta,[epsi]-Carotene-3,20,3'-triol; beta, beta-Carotene-3,4,4'-trione; beta,beta-Caroten-2-ol; beta,beta- Caroten-3-ol; beta,beta-Caroten-4-ol; beta,[epsi]-Caroten-2-ol; beta,[epsi]-Caroten-3-ol; beta,[epsi]-Caroten-3'-ol; beta,[epsi]-Caroten-4-ol; beta,[phi]-Caroten-3-ol; beta,X-Caroten-3-ol; beta,psi-Caroten-3-ol; beta,psi-Caroten-4'-ol; [epsi],psi-Caroten-3-ol; [phi],[phi]-Caroten-3-ol; [phi],[phi]-Caroten-16-ol; [phi],[phi]-Caroten- 20-ol; Carotenonaldehyd; beta-Carotenone; beta,beta-Caroten-2-one; beta,beta-Caroten-4-one; beta,[epsi]-Caroten-2-one; beta,[epsi]- Caroten-4-one; beta,psi-Caroten-4-one; gamma-Caroten-4-one; alpha-Carotone; Celaxanthin; Chiriquixanthin A; Chiriquixanthin B; Chlorellaxanthin; Chlorobactene; Chloroxanthin; Chrysanthemaxanthin; Citranaxanthin; alpha-Citraurin; beta-Citraurin; beta-Citraurinene; beta-Citraurinol; Citroxanthin; Compound X; C.p.: Corynebacterium poinsettiae; Corynexanthin; Corynexanthin glucoside; C.p.; C.p.; C.p.; Crocetin; gamma-Crocetin; Crocetindial(dehyde); Crocetin diglucosyl ester; Crocetin dimethyl ester; Crocetin gentiobiosyl glucosyl diester; Crocetin glucosyl methyl diester; Crocetin monogentiobiosyl ester; Crocetinsemialdehyde; Crocin; Crocoxanthin; Crustaxanthin; Cryptocapsin; Cryptocapsone; Cryptochrome; alpha-Cryptoeutreptiellanone; beta-Cryptoeutreptiellanone; Cryptoflavin; Cryptomonaxanthin; Cryptoxanthene; Cryptoxanthin; alpha-Cryptoxanthin; beta-Cryptoxanthin; Cryptoxanthin 5,6,5',6' diepoxide; Cryptoxanthin 5,6,5',8' diepoxide; Cryptoxanthin 5,8, 5',8' diepoxide; Cryptoxanthin 5,6-epoxide; Cryptoxanthin 5,8-epoxide; Cryptoxanthol; Cucurbitaxanthin; Cyclic zeta-carotene; Cynthiaxanthin; Decahydro-beta-carotene; 1,2,7,8,11,12,7',8',11',12'-Decahydro-psi,psi-carotene; 7,8,11,12,15,7',8',11',12',15' Decahydro- psi,psi-carotene; 1,2,7,8,11,12,7',8',11',12'-Decahydro-psi,psi-caroten-1-ol; Decahydrolycopene; Decaprenoxanthin; Decaprenoxanthin diglucoside; Decaprenoxanthin monoglucoside; Deepoxyneoxanthin; Dehydro- see also Bisdehydro-, Didehydro-, MonodehydroDehydroadonirubin; Dehydroadonixanthin; Dehydrocarotene II; Dehydrocarotene III; Dehydro-beta-carotene; 3,4-Dehydro-beta-carotene; 3',4'-Dehydro-gamma-carotene; 3',4'-Dehydrocryptoxanthin; Dehydrogenans-P; Dehydrogenans-P; Dehydrogenans-P; Dehydrogenans-P; Dehydrogenans-P 439 mono-OH; dehydrogenans-Phytoene; dehydrogenans-Phytofluene; Dehydrohydroxyechinenone; 3'-Dehydrolutein; 3,4-Dehydrolycopen-16-al; Dehydrolycopene; 3,4-Dehydrolycopene; 15,15'-Dehydrolycopersene; 7',8', 11',12'-Dehydrononapreno xanthin; 11',12'-Dehydrononaprenoxanthin; 3',4'-Dehydro-17'(or 18')-oxo-gamma-carotene; Dehydropapilioerythrin; 11,12-Dehydrophytoene; 11',12'-Dehydrophytoene; 2'-Dehydroplectaniaxanthin; Dehydroretrocarotene; 3,4-Dehydrorhodopin; Dehydrorhodovibrin; 3',4'-Dehydrorubixanthin; Dehydrosqualene; 7,8,7',8'-Dehydrozeaxanthin; 7,8-Dehydrozeinoxanthin; Demethyl(ated) spheroidene; Deoxyflexixanthin; Deoxylutein I; Deshydroxydecaprenoxanthin; Diadinochrome; Diadinoxanthin; Dianhydroeschscholtzxanthin; 4,4'-Diapo-zeta-carotene; 4,4'-Diapocaroten-4-al; 4,4'-Diapocarotene-4,4'-dial; 8,8'-Diapocarotene-8,8'- dial; 6,6'-Diapocarotene-6,6'-dioic acid; 8,8'-Diapocarotene-8,8'-dioic acid; 4,4'-Diapocaroten-4-oic acid; 4,4'-Diaponeurosporene; 4,4'-Diaponeurosporen-4-oic acid; 4,4'-Diapophytoene; 4,4'-Diapophytofluene; 4,4'-Diapo-7,8,11,12-tetrahydro lycopene; Diatoxanthin; Didehydro-, see also Dehydro-, Monodehydro 3',4'-Didehydro-2'-apo-beta-caroten-2'-al; 3',4'-Didehydro-2'-apo-beta-caroten-2'-ol; 7,8-Didehydroastaxanthin; 3',4'-Didehydro-beta,psi-caroten-16'-al; 3,4-Didehydro-psi,psi- caroten-16-al; 3,4-Didehydro-beta,beta-carotene; 4,4'-Didehydro-beta -carotene; 3,4-Didehydro-beta,[epsi]-carotene; 3,4-Didehydro-beta, [phi]-carotene; 3,4-Didehydro-beta,X-carotene; 3',4'-Didehydro-beta, psi-carotene; 3',4'-Didehydro-gamma,psi-carotene; 7,8-Didehydro- [phi],[phi]-carotene; 7,8-Didehydro-[phi],X-carotene; 3,4-Didehydro-psi, psi-carotene; 7,8-Didehydro-beta,beta-carotene-3,3'-diol; 7,8-Didehydro-beta,[epsi]-carotene-3,3'-diol; 3,4-Didehydro-beta,beta-carotene-2,2'-dione; 3',4'-Didehydro-beta,psi-caroten-16'-oic acid; 3',4'-Didehydro-beta,beta-caroten-3-ol; 3',4'-Didehydro-beta,beta- caroten-4-ol; 7,8-Didehydro-beta,[epsi]-caroten-3-ol; 7,8-Didehydro- beta,[phi]-caroten-3-ol; 7,8-Didehydro-beta,X-caroten-3-ol; 3',4'-Didehydro-beta,psi-caroten-3-ol; 3',4' Didehydro-beta,psi-caroten- 16'-ol; 3',4'-Didehydro-beta,psi-caroten-18'-ol; 3',4'-Didehydro- beta,beta-caroten-4-one; 3',4'-Didehydro-beta,psi-caroten-4-one; 7',8'-Didehydro-beta,beta-carotene 3,4,3'-triol; 3,4-Didehydro-1,2-dihydro-psi,psi-carotene; 3,4-Didehydro-1,2-dihydro-psi,psi- caroten-20-al; 6,7-Didehydro-5,6-dihydro-beta,beta-carotene-3,3'-diol; 3',4'-Didehydro-1',2'-dihydro-beta,psi-carotene-3,1'-diol; 3',4'-Didehydro-1',2'-dihydro-beta,psi-carotene-1',2'-diol; 3',4'-Didehydro-1',2'-dihydro-beta,psi-carotene-4,2'-dione; 3,4-Didehydro-1,2-dihydro-psi,psi-carotene-1,2-diol; 7',8'-Didehydro-5,6-dihydro-beta,beta-carotene-3,5,6,3'-tetrol; 6,7-Didehydro-5,6-dihydro-beta,beta-carotene-3,5,3'-triol; 7',8'-Didehydro-5,6-dihydro-beta,beta-carotene-3,5,3'-triol; 3',4'-Didehydro-1',2'-dihydro-beta,psi-carotene-2,1',2'-triol; 1',16'-Didehydro-1',2'-dihydro-beta,psi-caroten-2'-ol; 3',4'-Didehydro-1',2'-dihydro-beta,psi-caroten-1'-ol; 3',4'-Didehydro-1',2'-dihydro-beta,psi-caroten-2'-ol; 3,4-Didehydro-1,2-dihydro-psi,psi- caroten-1-ol; 3',4'-Didehydro-18'-hydroxy-gamma-carotene; 7,8-Didehydroisorenieratene; 3',4'-Didehydro-4-keto-gamma-carotene; 7,8-Didehydrorenieratene; 4',5'-Didehydro-4,5'-retro-beta,beta-carotene; 4',5'-Didehydro-4,5'-retro-beta,psi-carotene; Didehydroretro-gamma-carotene; 4',5'-Didehydro-4,5'-retro-beta,beta-carotene-3,3'-diol; 4',5'-Didehydro-4,5'-retro-beta,beta-carotene-3,3'-dione; 10',11'-Didehydro-5,8,11',12' tetrahydro-10'-apo-beta-carotene-3,5,8-triol; 6',7'-Didehydro-5,6,5',6' tetrahydro-beta,beta-carotene-3,5,6,3',5'-pentol; 6,7-Didehydro-5,6,5',6'-tetrahydro-beta,beta-carotene-3,5,3',5' tetrol; 3,4-Didehydro-1,2,7',8'-tetrahydro-psi,psi-caroten-1-ol; Didehydrotrikentriorhodin; 7,8-Didehydrozeaxanthin; Didemethylated spirilloxanthin; 1,2,1',2'-Diepoxy-2,2'-bis(3-hydroxy-3-methylbutyl)3,4-didehydro-1,2,1',2'-tetrahydro-psi,psi-carotene; Diepoxy-beta-carotene; 5,8,5',8'-Diepoxycryptoxanthin; 5,6,5',6'-Diepoxy-5,6,5',6'-tetrahydro-beta,beta-carotene; 5,6,5',8'-Diepoxy-5,6,5',8'tetrahydro- beta,beta-carotene; 5,8,5',8'-Diepoxy-5,8,5',8'tetrahydro-beta, beta-carotene; 5,6,5',6'-Diepoxy-5,6,5',6'-tetrahydro-beta,beta-carotene-3,3'-diol; 5,6,5',8'-Diepoxy-5,6,5',8'tetrahydro-beta,beta-carotene-3,3'-diol; 5,8,5',8'-Diepoxy-5,8,5',8'tetrahydro-beta,beta-carotene-3,3'-diol; 5,6,5',6'-Diepoxy-5,6,5',6'tetrahydro-beta,beta-caroten-3-ol; 5,6,5',8'-Diepoxy-5,6,5',8'tetrahydro-beta,beta-caroten- 3-ol; 5,8,5',8'-Diepoxy-5,8,5',8'tetrahydro-beta,beta-caroten-3-ol; 5, 6,5',8'-Diepoxyzeaxanthin; 5,8,5',8'-Diepoxyzeaxanthin; Digentiobiosyl 8, 8'-diapocarotene-8,8'-dioate; Di-(beta,D-glucopyranosyi)-4,4'- diapocarotene-4,4'-dioate; Diglucosyl 8,8'-diapocarotene-8,8'-dioate; Dihydroanhydrorhodovibrin; 9',10'-Dihydro-9'-apo-beta-carotene-3,9'-dione; 9',10'-Dihydro-9'-apo-[epsi]-carotene-3,9'-dione; 7',8'-Dihydro-7'-apo-beta-caroten-8'-one; 5',6'-Dihydro-5'-apo-18'-nor-beta-caroten-6'- one; 7,8-Dihydroastaxanthin; beta-Dihydrocarotene; 1,1'-Dihydro-beta- carotene; 3,4-Dihydro-beta-carotene; 7,7'-Dihydro-beta-carotene; 7',8'-Dihydro-beta,psi-carotene; 7',8'-Dihydro-gamma-carotene; 7',8'-Dihydro-gamma,psi-carotene; 7',8'-Dihydro-[delta]-carotene; 7',8'-Dihydro-[epsi],psi-carotene; 1,2-Dihydro-zeta-carotene; 1,2-Dihydro- psi,psi-carotene; 7,8-Dihydro-psi,psi-carotene; 7,8-Dihydro-beta,beta-carotene 3,3'-diol; 7',8'-Dihydro-beta,psi-carotene 3,17'-diol; 9',10'-Dihydro-beta,psi-carotene-3,17'-diol; 7',8'-Dihydro- [epsi],psi-carotene-3,17'-diol; 1,2-Dihydro-psi,psi-carotene-1,20-diol; 5,6-Dihydro-beta,beta-carotene 3,5,6,3'-tetrol; 5,6-Dihydro- beta,beta-carotene 3,5,3'-triol; 1',2'-Dihydro-beta,psi-caroten 1'-ol; 7',8'-Dihydro-beta,psi-caroten 3-ol; 1',2'-Dihydro-[phi],psi-caroten-1'-ol; 1,2-Dihydro-psi,psi-caroten-1-ol; 5,6-Dihydro-beta, beta-carotene-3,5,6,3'-tetrol; 5,6-Dihydro-beta,[epsi]-carotene-3,5,6, 3'-tetrol; 7,8 (or 7',8)-Dihydrodecaprenoxanthin monoglucoside; 1',2'-Dihydro-3',4'-dehydro-3,1'-dihydroxy-gamma-carotene; 1,2-Dihydro-3,4-dehydrolycopene; 1,2-Dihydro-3,4-dehydro-1-OH-lycopene; 7,8-Dihydro-4,4'-diapocarotene; 7',8'-Dihydro-4,4'-diapocaroten-4-al; 7',8'-Dihydro-4,4'-diapocaroten-4-oic acid; 1',2'-Dihydro-3',4'-didehydro-3,1'-dihydroxy- gamma-caroten-2'yl rhamnoside; 1',2'-Dihydro-1',2'-dihydroxy-4- ketotorulene; 1,2'-Dihydro-3,1'-dihydroxytorulene glucoside; 1',2'- Dihydro-3,1'-dihydroxytorulene rhamnoside; 1',2'-Dihydro-4,2'-diketotorulene; 3'-Dihydro-[boxH]-doradecin; 1',2'-Dihydro-1'-glucosyl-3,4-dehydrotorulene; 1',2'-Dihydro-1'-glucosyl-4-ketotorulene; 1',2'-Dihydro-1'-hydroxy-gamma-carotene; 1',2'-Dihydro-1'-hydroxychiorobactene; 1',2'-Dihydro-2'-hydroxy-3',4'-dehydro-4-keto-gamma-carotene; 1',2'-Dihydro-1'-hydroxy-3,4-dehydrotorulene glucoside; 1',2'-Dihydro-1'-hydroxy-4-keto-gamma-carotene; 1',2'-Dihydro-1'-hydroxy-4-ketotorulene; 1',2'-Dihydro-1'-hydroxy-4-ketotorulene glucoside; 1',2'-Dihydro-1'-hydroxysphe roideneone; 1',2'-Dihydro-1'-hydroxytorulene glucoside; 1',2'-Dihydro-1'-hydroxytorulene rhamnoside; 1, 2-Dihydrolycopene; 1',2'-Dihydrolycopene; 7,8-Dihydrolycopene; 1,2- Dihydro-1-methoxy-lycopen-20-al; Dihydromethoxylycopene; 5,6-Dihydro-4-methoxy-lycopen-6-one; 1,2-Dihydroneurosporene; 1',2'-Dihydroneurosporene; 1,2-Dihydro-1-OH-lycopene; 1',2'-Dihydro-1'-OH-torulene; 2'-Dihydrophillipsiaxanthin; Dihydrophytoene; 1,2-Dihydrophytoene; 1',2'-Dihydrophytoene; 1,2-Dihydrophytofluene; 1',2'-Dihydrophytofluene; 7,8-Dihydro-8,7'-retro-beta,beta-carotene; 7',8'-Dihydrorhodovibrin; 7,8 (or 7',8')-Dihydrosarcinaxanthin; 3,4-Dihydrospheroidene; 11',12'-Dihydrospheroidene; 3,4-Dihydrospirilloxanthin; 3,3'- Dihydroxycanthaxanthin; 3,3'-Dihydroxy-alpha-carotene; 3,4-Dihydroxy-beta-carotene; 2,3-Dihydroxy-beta,beta-carotene-4,4'-dione; 3,3'-Dihydroxy-[epsi]-carotene; 2,3'-Dihydroxy-beta,beta-carotene-4,4'- dione; 3,3'-Dihydroxy-beta,beta-carotene-4,4'-dione; 3,3'-Dihydroxy-beta,[epsi]-carotene-4,2'-dione; 3,3'-Dihydroxy-beta,chi-carotene-4,6'-dione; 3,3'-Dihydroxy-chi,chi-carotene-6,6'-dione; 2,3-Dihydroxy-beta,beta-caroten-4-one; 3,3'-Dihydroxy-beta,beta-caroten-4-one; 3,2'-Dihydroxy-beta,[epsi]-caroten-4-one; 3,3'-Dihydroxy-beta,[epsi]- caroten-4-one; 3,3'-Dihydroxy-beta,chi-caroten-6'-one; 3,8-Dihydroxy-chi,X-caroten-6-one; 3,3'-Dihydroxydehydro-beta-carotene; 3,3'-Dihydroxy-7,8-dehydro-beta-carotene; 3,3'-Dihydroxy-7,8,7',8'-dehydro-beta-carotene; 3,3'-Dihydroxy-7,8-dehydro-beta-carotene-5',6'-epoxide; 3,3'-Dihydroxy-2,3-didehydro-beta,beta-carotene-4,4'-dione; 3,3'- Dihydroxy-7,8-didehydro-beta,beta-carotene-4,4'-dione; 3',8'-Dihydroxy-7,8-didehydro-beta,chi-carotene-3',6'-dione; 3,3'-Dihydroxy-2,3-didehydro-beta,beta-caroten-4-one; 3,3'-Dihydroxy-7',8'-didehydro-beta,beta-caroten-4-one; 3,4'-Dihydroxy-2,3-didehydro-beta,beta- caroten-4-one; 3,3'-Dihydroxy-2,3-didehydro-beta,[epsi]-caroten-4-one; 3,8-Dihydroxy-7',8'-didehydro-chi,X-caroten-6-one; 3,6'-Dihydroxy-7,8-didehydro-6',7'dihydro-beta,[epsi]-carotene-3',8'-dione; 3,3'-Dihydroxy-7,8-didehydro-7',8'dihydro-beta,chi-carotene-6',8'-dione; 3,1'-Dihydroxy-3',4'-didehydro-1',2'-dihydro-beta,psi-caroten-4-one; 1',2'-Dihydroxy-3',4'-didehydro-1',2'-dihydro-beta,psi-caroten-4-one; 3,5-Dihydroxy-6,7-didehydro-5,6,7',8'-tetrahydro-7'-apo-beta-caroten-8'-one; 6,3'-Dihydroxy-7',8'-didehydro-5,6,7,8-tetrahydro-beta,beta-carotene-3,8-dione; 3,3'-Dihydroxy-5,8,5',8'-diepoxy-beta-carotene; 5,6-Dihydroxy-5,6-dihydro-10'-apo-beta-caroten-10'-al; 5,6-Dihydroxy-5,6-dihydro-10'-apo-beta-caroten-10'-oic acid; 5,6-Dihydroxy-5,6-dihydro 12'-apo-beta-caroten-12'-oic acid; 3,3'-Dihydroxy-7,8-dihydro-beta, beta-carotene-4,4'-dione; 3,1'-Dihydroxy-1',2'-dihydrotorulene; 1',2'-Dihydroxy-1',2'-dihydrotorulene; 3,3'-Dihydroxy-4,4'-diketo-beta- carotene; 3,3'-Dihydroxy-2,2'-dinor-beta,beta-carotene-4,4'-dione-3,3'-diacylate; 3,19-Dihydroxy-3',6'-dioxo-7,8-didehyro-beta,chi- caroten-17-al; 1,1'-Dihydroxy-2,2'-dirhamnosyl-1,2,1',2'-tetrahydro-3,4,3',4'-tetrahydrolycopene; 3,3'-Dihydroxyechinenone; 3,3'-Dihydroxy-5,6-epoxy-alphacarotene; 3,3'-Dihydroxy-5,8-epoxy-alpha-carotene; 3,3'- Dihydroxy-5,6-epoxy-beta-carotene; 3,3'-Dihydroxy-5,8-epoxy-beta-carotene; 2-(Dihydroxyisopentenyl)-2'-isopentenyl-beta-carotene; 3,3'-Dihydroxyisorenieratene; 3,3'-Dihydroxy-4-keto-gcarotene; 3,3'-Dihydroxyluteochrome; Dihydroxylycopene; 3,1'-Dihydroxy-2'-(5-C-methylpentosyloxy)-3',4'-didehydro-1',2'-dihydro-beta,psi-caroten-4- one; Dihydroxyneurosporene; 2',3'-Dihydroxy-2-nor-beta,beta-carotene-3,4-dione; 3,3'-Dihydroxy-2-nor-13-beta,beta-carotene-4,4'-dione-3-acylate; 3,3'-Dihydroxy-2-nor-13-beta,beta-carotene-4,4'-dione-3,3'- di-acylate; 1,2-Dihydroxyphytofluene; Dihydroxypirardixanthin; 3,3'- Dihydroxyretro-beta-carotene; 3,3'-Dihydroxy-2,3,2',3'-tetradehydro-beta,beta-carotene-4,4'-dione; 3,3'-Dihydroxy-7,8,7';8'-tetradehydro- beta,beta-carotene-4,4'-dione; 3,3'-Dihydroxy-2,3,2',3'-teradehydro-beta,beta-carotene-4,4'-dione dipalmitate; 3,3'-Dihydroxy-7,8,7',8'-tetradehydro-beta,beta-caroten-4-one; 1,1'-Dihydroxy-3,4,3',4'-tetradehydro-1,2,1',2'-tetrahydrod-psi,psi-carotene-2,2'-dione; 3,8'-Dihydroxy-5',6',7',8'-tetrahydro-5'-apo-18'-nor-beta-caroten-6'-one; 1,1'-Dihydroxy-1,2,1'2'-tetrahydro-zeta-carotene; 5,5'-Dihydroxy-5,6,5', 6'-tetrahydro-beta,beta-carotene-3,3'-dione; 3,3'-Dihydroxy-7,8,7';8'-tetrahydro-chi,chi-carotene-6,6'-dione; 9',10'-Dihydro-beta-zeacarotene 3,17'-iol; Diketo-, see also Dioxo- or -dione 2,2'- Diketobacterioruberin; 3,4-Diketo-beta-carotene; 4,4'-Diketo-beta-carotene; 4,4'-Diketo-gamma-carotene; 4,4'-Diketocynthiaxanthin; 3,3'- Diketodehydro-beta-carotene; 4,4'-Diketolycopene; Diketopirardixanthin; 3,3'-Diketoretro-beta-carotene; 3,3'-Diketoretrodehydro-beta-carotene; 2,2'-Diketospirilloxanthin; 4,4'-Diketo-7,8,7',8'-tetrade hydrozeaxanthin; 3,3'-Dimethoxy-beta,beta-carotene; 3,3'-Dimethoxy-beta,[epsi]-carotene; 3,3'-Dimethoxy-gamina-carotene; 3,3'-Dimethoxy-3',4'-dehydro-gamma-carotene; 1,1'-Dimethoxy-3,4-didehydro-1,2,1',2',7',8'-hexahydro-psi,psi-carotene; 1,1'-Dimethoxy-3,4-didehydro-1,2,1',2',7',8'-hexahydro-psi,psi-caroten-2-one; 1,1'-Dimethoxy-3,4-didehydro-1,2,1',2'-tetrahydro-psi,psi-carotene; 1,1'-Dimethoxy-3',4'-didehydro-1,2,1',2'-tetrahydro-psi,psi-caroten-4-one; 1,1'-Dimethoxy-1,2,7,8,1',2'-hexahydro-psi,psi-carotene; 1,1'-Dimethoxy-1,2,7,8,11,12,1',2'-octahydro-psi,psi-carotene; 1,1'-Dimethoxy-3,4,3',4'-tetradehydro-1,2,1',2'-tetrahydro-psi,psi-carotene; 1,1'-Dimethoxy-3,4,3',4'-tetradehydro-1,2,1',2'-tetrahydro-psi,psi-carotene-2,2'-dione; 1,1'-Dimethoxy-1,2,1',2'-tetrahydro-psi,psi-caroten-20-al; 1,1'-Dimethoxy-1,2,1',2'-tetrahydro-psi,psi-carotene; 1,1'-Dimethoxy-1,2,1',2'-tetrahydro-psi,psi-carotene-4,4'-dione; 1,1'-Dimethoxy-1,2,1',2'- tetrahydrolycopene; 1,1'-Dimethoxy-1,1',2,2'-tetrahydroneurosporene; Dimethylcrocetin; Dimethyl-6,6'-diapocarotene-6,6'-dioate; Dimethyl-8,8'-diapocarotene-8,8'-dioate; Dineapolitanosyl-8,8'-diapocarotene-8,8'-dioate; 2,2-Dinor-beta,beta-carotene-3,4,3',4'tetrone; Dinoxanthin; 3, 3'-Dioxi-4-oxo-beta-carotene; Dioxo-, see also Diketo- or-dione 5,6- Dioxo-10'-apo-5,6-seco-beta-caroten-10'-al; 5,6,5',6'-Diseco-beta, beta-carotene 5,6,5',6'-tetrone; 7,8,11,12,13,14,15,7',8',11',12',15'-Dodecahydro-13,15':14,15'biscyclo-15,15'-seco-psi,psi-caroten-15-ol; Dodecahydrolycopene; alpha-Doradecin; beta-Doradecin; alpha-Doradexanthin; beta-Doradexanthin;
Echinenone; Echininone; Eloxanthin; 6-Epikarpoxanthin; 3'- Epilutein; 5,6-Epoxy-alpha-carotene; 5,8-Epoxy-alpha-carotene; 5,8- Epoxy-beta-carotene; 1,2-Epoxy-1,2,7,8,11,12,7',8',11',12'-decahydro-psi,psi-carotene; 5,6-Epoxy-7',8'-didehydro-5,6-dihydro-beta,beta-carotene-3,3'-diol; 5,8-Epoxy-7',8'-didehydro-5,8-dihydro-beta,beta-carotene-3,3'-diol; 1',2'-Epoxy-3',4'-didehydro-1,2'-dihydro-beta,psi-caroten-2-ol; 5',6'-Epoxy-6,7-didehydro-5,6,5',6'-tetrahydro-beta,beta-carotene-3,5,19 (or 19'), 3'-tetrol; 5',6'-Epoxy-6,7-didehydro-5,6,5',6'-tetrahydro-beta,beta-carotene-3,5,3'-triol; 5',6'-Epoxy-6,7-didehydro-5,6,5,6'-tetrahydro-beta,beta-carotene-3,5,3'-triol 3-acetate; 5',8'-Epoxy-6,7-didehydro-5,6,5',8'-tetrahydro-beta,beta-carotene-3,5,3'-triol; 5,6-Epoxy-5,6-dihydro-12'-apo-beta-carotene-3,12'-diol; 5,8-Epoxy-5,8-dihydro-10'-apo-beta-carotene-3,10'-diol; 5,8-Epoxy-5,8-dihydro-12'-apo-beta-carotene-3,12'-diol; 5,6-Epoxy-5,6-dihydro-beta, beta-carotene; 5,8-Epoxy-5,8-dihydro-beta,beta-carotene; 5,6-Epoxy-5,6-dihydro-beta,[epsi]-Ecarotene; 5,8-Epoxy-5,8-dihydro-beta,[epsi]-carotene; 1',2'-Epoxy-1',2'-dihydro-beta,psi-carotene; 1',2'-Epoxy-1',2'-dihydro-[epsi],psi-carotene; 1,2-Epoxy-1,2-dihydro-psi,psi- carotene; 5,6-Epoxy-5,6-dihydro-psi,psi-carotene; 5,6-Epoxy-5,6-dihydro-beta,beta-carotene-3,3'-diol; 5,8-Epoxy-5,8-dihydro-beta, beta-carotene-3,3'-diol; 5,6-Epoxy-5,6-dihydro-beta,[epsi]-carotene-3,3'-diol; 5,6-Epoxy-5,6-dihydro-beta,[epsi]-carotene-3,3'-diol dipalmitate; 5,8-Epoxy-5,8-dihydro-beta,[epsi]-carotene-3,3'-diol; 5,6-Epoxy-5,6-dihydro-beta,[epsi]-carotene-3,3',6'-triol; 5,8-Epoxy-5,8-dihydro-beta,[epsi]-carotene-3,3',6'-triol; 5,6-Epoxy-5,6-dihydro-beta,beta-caroten-2-ol; 5,6-Epoxy-5,6-dihydro-beta,beta-caroten-3-ol; 5',8'-Epoxy-5',8'-dihydro-beta,beta-caroten-3-ol; 5,6-Epoxy-5,6-dihydro-beta,[epsi]-caroten-2-ol; 5,6-Epoxy-5,6-dihydro-beta,psi-caroten-3-ol; 5,8-Epoxy-5,8-dihydro-beta,psi-caroten-3-ol; 5,8-Epoxy-3,3'-dihydroxy-alpha-carotene; 5,6-Epoxy-3,3'-dihydroxy-7',8'didehydro-5,6,7,8-tetrahydrod-beta,beta-caroten-8-one; 5',6'-Epoxy-3,3'-dihydroxy-7,8-didehydro-5',6'-dihydro-10,11,20-trinor-beta,beta-caroten-19',11'-olide; 5',6'-Epoxy-3,3'-dihydroxy-4,7-didehydro-5',6'-dihydro-10,11,20-trinor-beta,beta-caroten-19',11'-olide 3-acetate; 5',6'-Epoxy-3,3'-dihydroxy-7,8-didehydro-5',6'-dihydro-10,11,20-trinor-beta,beta-caroten-19',11'-oxide 3-acetate; 5,6-Epoxy-3,3'-dihydroxy-5,6-dihydro-beta,chi-caroten-6'-one; 5,8-Epoxy-3,3'-dihydroxy-5,8-dihydro-beta,chi-caroten-6'-one; 5,6-Epoxy-3,3'-dihydroxy-5,6,7',8'-tetrahydro-beta,[epsi]-caroten-11',19'-olide; 1',2'-Epoxy-2'-(2,3-epoxy-3-methylbutyl)-2-(3-hydroxy-3-methylbutyl)-3',4'-didehydro-1,2,1',2'-tetrahydro-psi,psi-caroten-1-ol; 1,2-Epoxy-1,2,7,8,7',8'-hexahydro-psi,psi-carotene; 5,6-Epoxy-3-hydroxy-8'-apo-beta-caroten-8'-al; 5,6-Epoxy-5,6-dihydro-10'-apo-beta-carotene-3,10'-diol; 5,8-Epoxy-3-hydroxy-gamma-carotene; 5,8-Epoxy-3-hydroxy-5,8-dihydro-8'-apo-beta-caroten-8'-al; 5,6-Epoxy-3-hydroxy-5,6-dihydro-10'-apo-beta-caroten-10'-al 502; 5,6-Epoxy-3-hydroxy-5,6-dihydro-12'-apo-beta-caroten-12'-al; 5,6-Epoxy-3-hydroxy-5,6,7',8',-tetrahydro-7'-apo-beta-caroten-8'-one; 5,8-Epoxylutein; 1,2-Epoxy-1,2,7,8,11,12,7',8'octahydro-psi,psi-carotene; 1,2-Epoxy-1,2,7,8,7',8',11',12'octahydro-psi,psi-carotene; 1',2'-Epoxy-7,8,11,12,1',2',7',8'-octahydro-beta,psi-caroten-2-ol; 1,2-Epoxyphytoene; 5,8-Epoxyrubixanthin; 5',8'-Epoxy-5,6,5',8'-tetrahydro-beta,beta-carotene-3,5,6,3'-tetrol; 5',6'-Epoxy-5,6,5',6'-tetrahydro-beta,beta-carotene-3,5,6,3'-tetrol; 5,6-Epoxy-3',4',7',8'-tetradehydro-5,6-dihydro-beta,beta-caroten-4-one; 5,6-Epoxy-3,3',5',19'-tetra-hydroxy-6',7'-didehydro-5,6,7,8,5',6'-hexahydro-beta,beta-caroten-8-one 3'-acetate 19'-hexanoate; 5,6-Epoxy-3,3',5'-trihydroxy-6',7'-didehydro-5,6,7,8,5',6'-hexahydro-beta,beta-caroten-8-one; 5,6-Epoxy-3,3',5'-trihydroxy-6',7'-didehydro-5,6,7,8,5',6'-hexahydro-beta, beta-caroten-8-one 3'-acetate; 5',6'-Epoxy-3,5,3'-trihydroxy-6,7-didehydro-5,6,5',6'-tetrahydro-10,11,20-trinor-beta,beta-caroten-19',11'-olide; 5',6'-Epoxy-3,5,3'-trihydroxy-6,7-didehydro-5,6,5',6'-tetrahydro-10,11,20-trinor-beta,beta-caroten-19',11'-olide 3-acetate; 4',5'-Epoxy-3,6,3'-trihydroxy-7,8,4',5',7',8'-hexahydro-gamma,[epsi]-caroten-8-one; 5,6-Epoxyzeaxanthin; 5,8-Epoxyzeaxanthin; Eschscholtzxanthin; Eschscholtzxanthone; 4'-Ethoxy-beta,beta-caroten-4-one; 4'-Ethoxy-4-keto-beta-carotene; Euglenanone; Euglenarhodon; Eutreptiellanone;
Flavacin; Flavochrome; Flavorhodin; Flavoxanthin; Flexixanthin; Foliachrome; Foliaxanthin;
Gazaniaxanthin; beta,D-Gentiobiosyl beta,D-glucosyl 8,8'-diapocarotene-8,8'-dioate; Gentiobiosyl hydrogen-8,8'-dioate; Gentiobiosyl neapolitanosyl 8,8'-diapocarotene-8,8'-dioate; beta,D-Glucosyl hydrogen-4,4'-diapocarotene-4,4'-dioate; 4'-beta,D-Glucosyl 4-hydrogen-7',8'-dihydro-4,4'-diapocarotene-4,4'-dioate; beta,D-Glucosyl hydrogen-8,8'-diapocarotene-8,8'-dioate; beta,D-Glucosyl methyl-8,8'-diapocarotene-8,8'-dioate; Glucopyranosyloxy (see Glucosyloxy); 4-Glucosyloxy-4,4' diaponeurosporene; 1'-Glucosyloxy-3',4'-didehydro-1',2'-dihydro-beta,psi-carotene; 1-Glucosyloxy-3,4-didehydro-1,2-dihydro-psi,psi-carotene; 2'-Glucosyloxy-3',4'-didehydro-1',2'-dihydro-beta, psi-carotene-3,1'-diol; 1'-Glucosyloxy-3',4'-didehydro-1',2'-dihydro-beta,psi-caroten-3-ol; 1'-Glucosyloxy-3',4'-didehydro-1',2'-dihydro-beta,psi-caroten-2'-ol; 1'-Glucosyloxy-3',4'-didehydro-1',2'-dihydro-beta,psi-caroten-4-one; 1-Glucosyloxy-3,4-didehydro-1,2,7',8'- tetrahydro-psi,psi-carotene; 1-Glucosyloxy-1,2-dihydro-psi,psi-caroten-20-al; 1-Glucosyloxy-1',2'-dihydro-beta,psi-carotene; 1'-Glucosyloxy-1',2'-dihydro-[phi],psi-carotene; 1-Glucosyloxy-1,2-dihydro-psi,psi-carotene; 4-Glucosyloxy-7',8'-dihydro-4,4'-diapocarotene; 1'-Glucosyloxy-2'-hydroxy-3',4'-didehydro-1',2'-dihydro-beta,psi-caroten-4-one; 2-(4-Glucosyloxy-3-methyl-2-butenyl)-2'-(4-hydroxy-3-methyl-2-butenyl)-gamma,gamma-carotene; 2-(4-Glucosyloxy-3-methyl-2-butenyl)-2'-(4-hydroxy-3-methyl-2-butenyl)-[epsi],[epsi]-carotene; 2-(4-Glucosyloxy-3-methyl-2-butenyl)-2'-(4-hydroxy-3-methyl-2-butenyl)-7,8-dihydro-[epsi],[epsi]-carotene; 2'-(4-Glucosyloxy-3-methyl-2-butenyl)-2-(3-methyl-2-butenyl)-[epsi],[epsi]-caroten-18-ol; 2-[3-(Glucosyloxy)-3-methylbutyl]-2'-(3-hydroxy-3-methylbutyl)-3,4,3',4'-tetradehydro-1,2,1',2'-tetrahydro-psi,psi-carotene-1,1'-diol; 1'-Glucosyloxy-3,4,3',4'-tetradehydro-1',2'-dihydro-beta,psi-carotene; Glycymerin; Guaraxanthin;
Halocynthiaxanthin; Helenien; Heteroxanthin; Hexadecahydrolycopene; 2,3,2',3',45'-Hexadehydro-4,5'-retro-beta,beta-carotene; 1,2,7,8,11,12-Hexahydro-psi,psi-carotene; 1,2,7,8,1',2'-Hexahydro-psi,psi-carotene; 1,2,7,8,7',8'-Hexahydro-psi,psi- carotene; 7,8,11,12,7',8'-Hexahydro-psi,psi-carotene; 7,8,11,12,7',8'-Hexahydro-beta,psi-caroten-2-ol; 15,7',8',11',12',15'-Hexahydro-beta,psi-caroten-2-ol; 1,2,7',8',11',12'-Hexahydro-psi,psi-caroten-1-ol; 7,8,11,12,7',8'-Hexahydro-psi,psi-caroten-16-ol; 7,8,11,12,7',8'-Hexahydro-4,4'-diapocarotene; 1,2,7,8,11,12-Hexahydrolycopene; 1',2',7', 8'11',12'-Hexahydrolycopene; 7,8,11,12,7',8'-Hexahydrolycopene; 7,8,1',2', 7',8'-Hexahydrolycopene; 3,4,3',4',7',8'-Hexahydrospirilloxanthin; 19'-Hexanoyloxyfucoxanthin; 19-Hexanoyloxyparacentrone; 1-Hexosyl-1,2-dihydro-3,4-didehydroapo-8'-lycopenol; O-Hexosyl-1'-hydroxy-1',2'-dihydro-gamma-carotene; O-Hexosy-1-4-keto-1'-hydroxy-1',2'-dihydro-3',4'-didehydro-gammna-carotene; Hopkinsiaxanthin; Hydroxy-, see also Monohydroxy-, OH or -ol3-Hydroxy-beta-apo-2-carotenal; 3-Hydroxy-8'-apo-beta-caroten- 8'-al; 3-Hydroxy-10'-apo-beta-caroten-10'-al: 3-Hydroxy-12'-apo-beta-caroten-12'-al; 3-Hydroxy-8'-apo-[epsi]-caroten-8'-al; 3-Hydroxy-8'-apo- beta-caroten-8'-oic acid; 9'-Hydroxy-9'-apo-beta-caroten-3-one; 9'-Hydroxy-9'-apo-[epsi]-caroten-3-one; Hydroxyasteroidenone; 3-Hydroxycanthaxanthin; 3-Hydroxy-beta,psi-caroten-18'-al; 3-Hydroxy-alpha-carotene; 3'-Hydroxy-alpha-carotene; 4-Hydroxy-alpha-carotene; 2-Hydroxy-beta-carotene; 3-Hydroxy-beta-carotene; 4-Hydroxy-beta-carotene; 3-Hydroxy-gamma-carotene; 4'-Hydroxy-gamma-carotene; 3-Hydroxy-[delta]-carotene; 2-Hydroxy-beta,beta-carotene-4,4'-dione; 3-Hydroxy-beta,beta-carotene-4,4'-dione; 3'-Hydroxy-beta,beta-carotene-3,4-dione; 4'-Hydroxy-beta,beta-carotene-3,4-dione; 3-Hydroxy-beta,[epsi]-carotene-4,3'-dione; 3'-Hydroxy-beta,[epsi]-carotene-3,4-dione; 3-Hydroxy-beta,chi-carotene-3',6'-dione; 3'-Hydroxy-beta,beta-carotene-3,4,4'-trione; 2'-Hydroxy-beta,beta-caroten-2-one; 2-Hydroxy-beta,beta-caroten-4-one; 3-Hydroxy-beta, beta-caroten-4-one; 3'-Hydroxy-beta,beta-caroten-4-one; 4'-Hydroxy-beta,beta-caroten-4-one; 3-Hydroxy-beta,[epsi]-caroten-4-one; 3-Hydroxy-beta,[epsi]-caroten-3'-one; 3'-Hydroxy-beta,chi-caroten-6'-one; 3Hydroxy-beta,psi-caroten-4'-one; 3-Hydroxy-beta,psi-caroten-4-one; 3-Hydroxy-[epsi],[epsi]-caroten-3'-one; 3'-Hydroxy-psi,psi-caroten-4-one; 3-Hydroxycitranaxanthin; 3-Hydroxy-7,8-dehydro-alpha-carotene; 3'-Hydroxy-3,4-dehydro-beta-carotene; 3-Hydroxy-3',4'-dehydro-gamma-carotene; 4-Hydroxy-4,4'-diaponeurosporene; 3-Hydroxy-2,3-didehydro-beta,beta-carotene-4,4'-dione; 2'-Hydroxy-3,4-didehydro-beta,beta-caroten-2-one; 3-Hydroxy-2,3-didehydro-beta,beta-caroten-4-one; 3-Hydroxy-2,3-didehydro-beta,[epsi]-caroten-4-one; 3-Hydroxy-2,3-didehydro-beta,X-caroten-4-one; 3-Hydroxy-2,3-didehydro-beta,[phi]-caroten-4-one; 3-Hydroxy-3',4'-didehydro-beta,psi-caroten-4-one; 3-Hydroxy-7,8-didehydro-7',8'-dihydro-7'-apo-beta-carotene-4,8'-dione; 3-Hydroxy-7,8-didehydro-7',8'-dihydro-7'-apo-beta-caroten-8'-one; 3-Hydroxy-7',8'-didehydro-7,8-dihydro-chi,X-carotene-6,8-dione; 1'-Hydroxy-3',4'-didehydro-1',2'-dihydro-beta,psi-caroten-4-one; 1'-Hydroxy-3',4'-didehydro-1',2'-dihydro-beta,psi-caroten-2'-one; 2'-Hydroxy-3',4'-didehydro-1',2'-dihydro-beta,psi-caroten-4-one; 5-Hydroxy-4',5'-didehydro-4,5-dihydro-4,5'-retro-beta,beta-carotene-3,3'-dione; 3'-Hydroxy-2',3'-didehydro-2-nor-beta,beta-carotene-3,4,4'-trione; 3'-Hydroxy-4',5'-didehydro-4,5'-retro-beta,beta-caroten-3-one; 3-Hydroxy-5,8,5',8'-diepoxy-beta-carotene; 3-Hydroxy-7',8'-dihydro-7'-apo-beta-caroten-8'-one; 3-Hydroxy-5',6'-dihydro-5'-apo-18'-nor-beta-caroten-6'-one; 1-Hydroxy-1,2-dihydro-psi,psi-caroten-20-al; 1'-Hydroxy-1',2'-dihydro-gamma-carotene; 3-Hydroxy-7,8-dihydro-chi,X-carotene-6,8-dione; 4'-Hydroxy-5',6'-dihydro-beta,beta-caroten-4-one; 1'-Hydroxy-1',2'-dihydro-beta,psi-caroten-4-one; 8'-Hydroxy-7',8'-dihydrocitranaxanthin; 4-Hydroxy-7',8'-dihydro-4,4'-diapocarotene; 4'-Hydroxy-5',6'-dihydroechinenone; 1'-Hydroxy-1',2'-dihydro-2-isopentenyl- 2'-(hydroxyisopentenyl)torulene; 1-Hydroxy-1,2-dihydrolycopene; 1-Hydroxy-1,2-dihydroneurosporene; 1'-Hydroxy-1',2'-dihydroneurosporene; 1-Hydroxy-1,2-dihydrophytoene; 1 (or 1')-Hydroxy-1,2 (or 1',2')-dihydrophytofluene; 8'-Hydroxy-7',8'-dihydroreticulataxanthin; 1'-Hydroxy-1',2'-dihydrospheroidene; 2'-Hydroxy-1',2'-dihydrotorulene; 2-Hydroxy-1',2'-dihydrotorulene-1',2'-oxide; 5-Hydroxy-5,6-dihydrozeaxanthin; 3-Hydroxy-3',4'-diketo-alpha-carotene; 3-Hydroxy-4,4'-diketo-beta-carotene; 3'-Hydroxy-3,4-diketo-beta-carotene; 2'-Hydroxy-3,1'-dimethoxy-3',4'-didehydro-1',2'-dihydro-beta,psi-caroten-4-one; 4-Hydroxy-3',4'-dioxo-beta-carotene; 2-Hydroxyechinenone; 3-Hydroxyechinenone; 3'-Hydroxyechinenone; 4'-Hydroxyechinenone; 3-Hydroxy-5,8-epoxy-beta-carotene; 3'-Hydroxy-3,6-epoxy-5,6-dihydro-beta,[epsi]-caroten-4-one; 3'-Hydroxy-3,6-epoxy-7',8'-didehydro-5,6-dihydro-beta,beta-caroten-4-one; 3'-Hydroxyeuglenanone; 2'-Hydroxyflexixanthin; 1-Hydroxy-1,2,7',8',11',12'-hexahydrolycopene; 1'-Hydroxy-3,4,1',2',11', 12'hexahydrospheroidene; 2-(4-Hydroxy-3-hydroxymethyl-2-butenyl)-2'-(3-methyl-2-butenyl)-beta,beta-carotene; 3-Hydroxyisorenieratene; 3-Hydroxy-4-keto-alpha-carotene; 3-Hydroxy-3'-keto-alpha-carotene; 3-Hydroxy-4-keto-beta-carotene; 3-Hydroxy-4'-keto-beta-carotene; 4-Hydroxy-4'-keto-beta-carotene; 1'-Hydroxy-2'-keto-1',2'-dihydrotorulene; 3-Hydroxy-3'-keto-retrodehydrocarotene; 19-Hydroxylutein; 16-Hydroxylycopene; 3-Hydroxy-3'-methoxy-beta-carotene; 1'-Hydroxy-1-methoxy-3,4-didehydro-1,2,1',2',7',8'-hexahydro-psi,psi-caroten-2-one; 1'-Hydroxy-1-methoxy-1,2,1',2',7',8'-hexahydro-psi,psi-caroten-4-one; 1'-Hydroxy-1-methoxy-3,4,3',4'-tetradehydro-1,2,1',2'-tetrahydro-psi,psi-caroten-2-one; 1'-Hydroxy-1-methoxy-1,2,1',2'-tetrahydro-psi,psi-caroten-4-one; 2-(4-Hydroxy-3-methyl-2-butenyl)-beta,beta-carotene; 2-(4-Hydroxy-3-methyl-2-butenyl)-[epsi],psi-carotene; 2-(3-Hydroxymethyl-but-2-enyl)-7',8'-dihydro-[delta]-carotene; 2-(4-Hydroxy-3-methyl-2-butenyl)-7',8'-dihydro-[epsi],psi-carotene; 2-(4-Hydroxy-3-methyl-2-butenyi)-2'-(3-methyl-2-butenyl)-[epsi],[epsi]-carotene; 2-(4Hydroxy-3-methyl-2-butenyl)-2'-(3-methyl-2-butenyl)-[epsi],[epsi]-caroten-18-ol; 2'-(4-Hydroxy-3-methyl-2-butenyl)-2-(3-methyl-2-butenyl)-3',4'-didehydro-1',2'-dihydro-beta,psi-caroten-1'-ol; 2 (or 2')-(4-Hydroxy-3-methyl-2-butenyl)-2' (or 2)-(3-methyl-2-butenyl)-3',4'-didehydro-1',2'-dihydro-[epsi],psi-caroten-1'-ol; 2'-(4-Hydroxy-3-methyl-2-butenyl)-2-(3-methyl-2-butenyl)-7,8 (or 7',8')-dihydro-[epsi],[epsi]-caroten-18-ol; 2-(4-Hydroxy-3-methyl-2-butenyl)-7,8,7',8'-tetrahydro-[epsi],psi-carotene; 2-(4-Hydroxy-3-methyl-2-butenyl)-7',8',11',12'-tetrahydro-[epsi],psi-carotene; 16-(3-Hydroxy-3-methylbutyl)-16'-(3-methyl-2-butenyl)-7,8,11,12,15,7',8',11',12',15'-decahydro-psi,psi-carotene; 2-(3-Hydroxy-3-methylbutyl)-2'-(3-methyl-2-butenyl)-3,4,3',4'-tetradehydro-1,2,1',2'-tetrahydro-psi,psi-carotene-1,1'-diol; 2-Hydroxy-monocyclic-phytofluene; 4-Hydroxymyxoxanthophyll; Hydroxyneurosporene; 15-Hydroxy-7',8',9',10',11', 12',13',14'-octahydro-6'-apo-beta-caroten-7'-one; 1'-Hydroxy-3,4,7,8,1',2',11',12'-octahydrospheroidene; 3'-Hydroxy-4-oxo-beta-carotene; 3-Hydroxy-4-oxo-2,3-dehydro-beta-carotene; 4'-Hydroxy-3-oxoechinenone; Hydroxyphytoene; Hydroxyphytofluene; 4'-Hydroxy-4-oxo-pirardixanthin; 2-Hydroxyplectaniaxanthin; 3-Hydroxy-4,5'-retro-5'-apo-beta-caroten-5'-one; 3-Hydroxy-4',12'-retro-beta,beta-carotene-3',12'-dione; 3'-Hydroxyrubixanthin; 3'-Hydroxy-5,6-seco-beta,beta-carotene-5,6-dione; 3-Hydroxysemi-beta-carotenone; 3-Hydroxysintaxanthin; Hydroxyspheroidene; Hydroxyspheroidenone; Hydroxyspirilloxanthin; 8'-Hydroxy-5',6',7',8'-tetrahydro-5'-apo-18'-nor-beta-caroten-6'-one; 4'-Hydroxy-5,6,5',6'-tetrahydro-beta,beta-caroten-4-one; 1-Hydroxy-3,4,3',4'-tetradehydro-1,2-dihydro-psi,psi-caroten-2-one; 1-Hydroxy-1,2,7',8'-tetrahydrolycopene; 1'-Hydroxy-3,4,1',2'-tetrahydrospheroidene; 3-Hydroxytorulene; 16'-Hydroxytorulene; 18'-Hydroxytorulene; 3-Hydroxy-3',4'-triketo-beta-carotene; 3-Hydroxy-beta-zeacarotene; 5-Hydroxyzeaxanthin;
Idoxanthin; Isoagelaxanthin A; Isobixin; Isocarotene; Iso- zeta-carotene; Iso-zeta-carotene; Isocrocetin; Isocryptoxanthin; Isofucoxanthin; Isofucoxanthinol; Isolutein; Isomethylbixin; Isomytiloxanthin; 2-Isopentenyl-3,4-dehydrorhodopin; Isorenieratene; beta-Isorenieratene; 3,3'-Isorenieratenediol; 3-Isorenieratenol; Isotedaniaxanthin; Isotedanin; Isozeaxanthin;
Karpoxanthin; Keto-, see also oxo or -one Ketocapsanthin; 4-Ketocapsanthin; 4-Keto-alpha-carotene; 4-Keto-beta-carotene; 4-Keto-gamma-carotene; 4-Ketocynthiaxanthin: 4-Keto-3',4'-dehydro-beta-carotene; 4-Keto-1',2'-dihydro-1'-hydroxytorulene; 2-Keto-7',8'-dihydrorhodovibrin; 4-Keto-3,3'-dihydroxy-alpha-carotene; 4'-Keto-3-hydroxy-gamma-carotene; 4-Keto-3'-hydroxylycopene; 4-Ketolutein 332 4-Ketomyxol 2'-(methylpentoside); 4-Ketomyxoxanthophyll; 2-Keto-OH-spirilloxanthin; 4-Ketophleixanthophyll; 2-Ketorhodovibrin; 4'-Ketorubixanthin; 2-Ketospirilloxanthin; 4-Ketotorulene; 4-Ketozeaxanthin;
Lactucaxanthin; Latochrome; Latoxanthin; leprotene; Lilixanthin; Loniceraxanthin; Loroxanthin; Lusomycin; Lutein; Lutein dimethyl ether; Lutein dipalmitate; Lutein epoxide; Luteochrome; Luteol; Luteoxanthin; Lycopenal; Lycopen-20-al; Lycopene; Lycopene-16,16'-diol; Lycopene 1,2-epoxide; Lycopene 5,6-epoxide; Lycopen-16-ol; Lycopen-20-ol; Lycopersene; Lycophyll; Lycoxanthin;
Mactraxanthin; Manixanthin; 1-Mannosyloxy-3,4-didehydro-1,2-dihydro-8'-apo-psi-caroten-8'-ol; 3'-Methoxy-beta,beta-caroten-3-ol; 3-Methoxy-beta,X-carotene; 1-Methoxy-1,2,7,8,11,12,7',8',11',12'-decahydro-psi,psi-carotene; 1'-Methoxy-1,2,7,8,11,12,1',2',7',8'-decahydro-psi,psi-caroten-1-ol; 1-Methoxy-3,4-didehydro-1,2-dihydro-psi,psi-caroten-20-al; 1'-Methoxy-3',4'-didehydro-1',2'-dihydro-beta,psi-carotene; 1-Methoxy-3,4-didehydro-1,2-dihydro-psi,psi-carotene; 1-Methoxy-3,4-didehydro-1,2,7',8',11',12'-hexahydro-psi,psi-carotene; 1'-Methoxy-3',4'-didehydro-1,2,7,8,1',2'-hexahydro-psi,psi-caroten-1-ol; 1-Methoxy-3,4-didehydro-1,2,7',8'-tetrahydro-psi,psi-carotene; 1'-Methoxy-3',4'-didehydro-1,2,1',2'-tetrahydro-psi,psi-caroten-1-ol; 1-Methoxy-3,4-didehydro-1,2,7',8'-tetrahydro-psi,psi-caroten-2-one; 1-Methoxy-1,2-dihydro-psi,psi-caroten-20-al; 1-Methoxy-1,2-dihydro-psi,psi-carotene; 1'-Methoxy-1',2'-dihydro-beta,psi-caroten-4'-one; 1'-Methoxy-1',2'-dihydro-X,psi-caroten-4'-one; 1-Methoxy-1,2-dihydro-psi,psi-caroten-4-one; 1'-Methoxy-1',2'-dihydro-3',4'-dehydro-gamma-carotene; 1-Methoxy-1,2-dihydro-3,4-dehydrolycopene; 1-Methoxy-1,2-dihydro-3,4-didehydrolycopen-20-al; 1-Methoxy-1,2-dihydrolycopene; 4-Methoxy-5,6-dihydrolycopene; 1-Methoxy-1,2-dihydroneurosporene; 1-Methoxy-1,2-dihydrophytoene; 1-Methoxy-1,2-dihydrophytofluene; 1'-Methoxy-1',2'-dihydrospheroidene; 3-Methoxy-19,3'-dihydroxy-7,8-didehydro-beta,chi-carotene-6',8'-dione; 1-Methoxy-1,2,7',8',11',12'-hexahydro-psi,psi-carotene; 1'-Methoxy-1,2,7,8,1',2'-hexahydro-psi,psi-caroten-1-ol; 1-Methoxy-1,2,7',8'11',12'-hexahydro-psi,psi-caroten-4-one; 1-Methoxy-1'-hydroxy-1,2,1',2'-tetrahydrophytofluene; 1-Methoxy-2-keto-7',8'-dihydro-3,4-dehydrolycopene; Methoxylycopenal; 1-Methoxy-1,2,7,8,7',8',11',12'-octahydro-psi,psi-carotene; 1'-Methoxy-1,2,7,8,11,12,1',2'-octahydro-psi,psi-caroten-1-ol; 1-Methoxy-4-oxo-1,2-dihydro-8'-apo-psi-caroten-8'-al; 1-Methoxy-4-oxo-1,2-dihydro-12'-apo-psi-caroten-12'-al; Methoxyphytoene; Methoxyphytofluene; Methoxyspheroidene; 1'-Methoxy-3,4,3',4'-teradehydro-1,2,1',2'-tetrahydro-psi,psi-caroten-1-ol; 1-Methoxy-1,2,7',8'-tetrahydro-psi,psi-carotene; 1-Methoxy-1,2,7',8'-tetrahydro-psi,psi-caroten-4-one; 1-Methoxy-1,2,7',8'-tetrahydro-3,4-dehydrolycopene; 3Methoxy-19,3',8'-trihydroxy-7,8-didehydro-beta,chi-caroten-6'-one; Methyl 4'-apo-beta-caroten-4'-oate; Methyl 8'-apo-beta-caroten-8'-oate; Methyl 6'-apo-psi-caroten-6'-oate; Methyl apo- 6'-lycopenoate; Methylbixin; 2-(3-Methyl-2-butenyl)-beta,beta-caroten-18 (or 18')-ol; 2-(3-Methyl-2-butenyl)-3,4-didehydro-1,2-dihydro-psi,psi-caroten-1-ol; 2-(3-Methyl-2-butenyl)-7,8,7',8'-tetrahydro-[epsi], psi-caroten-18-ol; Methyl 3',4'-didehydro-beta,psi-caroten-16'-oate; Methyl 1-hexosyl-1,2-dihydro-3,4-didehydro-apo-8'-lycopenoate; Methyl hydrogen 6,6'-diapocarotene-dioate; Methyl 1-mannosyloxy-3,4-didehydro-1,2-dihydro-8'-apo-psi-caroten-8'-oate; Methyl 1'-methoxy-4'-oxo-1',2'-dihydro-X,psi-caroten-16 (or 17 or 18)-oate; 2'-(O-Methyl-5-C-methylpentosyloxy)-3',4'-didehydro-1',2'-dihydro-beta,psi-carotene-3,1'-diol; Metridene; Mimulaxanthin; Monadoxanthin; Monoanhydrobacterioruberin; Monodehydro-beta-carotene; Monodehydrolycopene; Monodemethyl(ated) spirilloxanthin; Monoepoxy-, see Epoxy-Monohydroxy cyclophytoene; Monohydroxy cyclophytofluene; Mutatochrome; Mutatoxanthin; Mytiloxanthin; Mytiloxanthinone; Myxobactin; Myxobactone; Myxol 2'-glucoside; Myxol 2'-O-methyl-methylpentoside; Myxol 2'-rhamnoside; Myxoxanthin; Myxoxanthol; Myxoxanthophyll;
Neocarotene; Neochrome; Neo-beta-carotene B; Neo-beta- cryptoxanthin A; Neoxanthin; Neoxanthin 3-acetate; Neurosporaxanthin; Neurosporaxanthin methyl ester; Neurosporene;
Octahydro-beta-carotene; 1,2,7,8,11,12,7',8'-Octahydro-psi,psi-carotene; 7,8,11,12,7',8',11',12'-Octahydro-psi,psi-carotene; 1,2,7,8,11,12,7',8'-Octahydro-psi,psi-carotene-1,2-diol; 1,27,8,1',2',7',8'-Octahydro-psi,psi-carotene-1,1'-diol; 1,2,7,8,11,12, 7',8'-Octahydro-psi,psi-caroten-1-ol; 7,8,11,12,7',8',11',12'-Octahydro-beta,psi-caroten-2-ol; 1,2,7,8,7',8',11',12'-Octahydro-psi,psi-caroten-1-ol; 7,8,11,12,7',8',11',12'-Octahydro-4,4'-diapocarotene; Octahydrolycopene; 5,6,7,8,5',6',7',8'-Octahydrolycopene; 7,8,11,12,7',8',11',12'-Octahydrolycopene; 3,4,3',4',7',8',11',12'-Octahydrospirilloxanthin; OH, see also Hydroxy- or -ol OH-Chlorobactene; OH-Chlorobactene glucoside; OH-Lycopene; 2-OH-Monocyclophytoene; 2-OH-Monocyclophytofluene; OH-Neurosporene; OH-Okenone; OH-P 481; OH-P 482; OH- P 511; OH-R; OH-Rhodopin; OH-Sintaxanthin 5,6-epoxide; OH-Spheroidene; OH-Spheroidenone; OH-7,8,11,12-Tetrahydrolycopene; OH-Y; Okenone; Ophioxanthin; Oscillaxanthin; Oscillol 2,2'-di(O-methyl-methylpentoside); Oscillol 2,2'-dirhamnoside; Ovoester; Oxo-, see also Keto or -one 3-Oxocanthaxanthin; 4'-Oxo-4,4'-diapocaroten-4-oic acid; 8'-Oxo-8,8'- diapocarotenoic acid; 3-Oxoechinenone; 4-Oxosaproxanthin; 16'-Oxotorulene; 6'-Oxychrysanthemaxanthin; P 412; P 444; P 450; P 452; P 481; P 500; P 518; 1'-[(chi-O-Palmitoyl-beta,D-glucosyl)oxy]-3',4'-didehydro-1',2'-dihydro-beta, psi-caroten-2'-ol; Papilioerythrin; Papilioerythrinone; Paracentrone; Parasiloxanthin; Pectenol; Pectenolone; Pectenoxanthin; Pentaxanthin; Peridinin; Peridininol; Persicachrome; Persicaxanthin; Phillipsiaxanthin; Philosamiaxanthin; Phleixanthophyll; Phleixanthophyll palmitate; Phoeniconone; Phoenicopterone; Phoenicoxanthin; Physalien; Physoxanthin; Phytoene; C (30)-Phytoene; Phytoene 1,2-(ep)oxide; Phytoenol; Phytofluene; Phytofluene epoxide; Phytofluenol; Pigment R; Pigment X; Pigment Y; Plectaniaxanthin; Poly-cis-gamma-carotene; Poly-cis-psi- carotene; Poly-cis-lycopene; Prasinoxanthin; Prelycopersene pyrophosphate; Prephytoene pyrophosphate; Pro-gamma-carotene; Prolycopene; Proneurosporene; Protetrahydrolycopene; Pseudo-alpha-carotene; Pyrenoxanthin; Pyrrhoxanthin; Pyrrhoxanthinol;
7-cis: Renieracistene; Renierapurpurin; Renieratene; Reticulaxanthin; Retinylidenetiglic acid; Retrobisdehydro(-beta-) carotene; Retrodehydro(-beta-)carotene; Retrodehydro-gamma-carotene; Retrodehydrozeaxanthin; Rhamnopyranosyloxy-, see Rhamnosyloxy-2'-O-Rhamnosyl-4-ketomyxol; 2'-O-Rhamnosylmyxol; 3'-Rhamnosyloxy-beta,beta- caroten-3-ol; 1-Rhamnosyloxy-3',4'-didehydro-1',2'-dihydro-beta,psi-carotene; 2'-Rhamnosyloxy-3',4'-didehydro-1',2'-dihydro-beta,psi-carotene-3,1'-diol; 2'-Rhamnosyloxy-3',4'-didehydro-1',2'-dihydro-beta,psi-carotene-3,4,1'-triol; 1'-Rhamnosyloxy-3',4'-didehydro-1',2'-dihydro-beta,psi-caroten-3-ol; Rhodoauranxanthin; Rhodopin; Rhodopin(-20-)al; Rhodopinal glucoside; Rhodopin glucoside; Rhodopinol; Rhodopurpurin; Rhodotorulene; Rhodovibrin; Rhodoviolascin; Rhodoxanthin; Roserythrin; Rubichrome; Rubixanthin; Rubixanthin 5,6-epoxide; Rubixanthone;
Salmon acid; Salmoxanthin; Saproxanthin; Sarcinaxanthin; Sarcinaxanthin diglucoside; Sarcinaxanthin monoglucoside; Sarcinene; 5,6- Seco-beta,beta-carotene-5,6-dione; 5,6-Seco-beta,[epsi]-carotene-5,6-dione; Semi-alpha-carotenone; Semi-beta-carotenone; Sidnyaxanthin; Sintaxanthin; Siphonaxanthin; Siphonein; Sodium-3,19-dihydroxy-7,8-di-dehydro-beta,chi-carotene-3',6'-dione 3-sulfate; Sodium-3,19-dihydroxy-3',6'-dioxo-7,8-didehydro-beta,chi-caroten-17'-al 3-sulfate; Sodium-3,19,3'-trihydroxy-7,8-didehydro-6'-oxo-beta,chi-caroten-17'-oate 3-sulfate; Sodium-3,19,17'-trihydroxy-7,8-didehydro-beta,chi-carotene-3',6'-dione 3-sulfate; Sphaerobolin; Spheroidene; Spheroidenone; Spirilloxanthin; Sulcatoxanthin;
Tangeraxanthin; Taraxanthin; Taraxanthin dipalmitate; Taraxien; Tareoxanthin; Tedaniaxanthin; Tedanin; Ternstroemiaxanthin; Tethyatene; 7,8,7',8'-Tetradehydroastaxanthin; 3,4,3',4'-Tetradehydro- beta,beta-carotene; 3,4,3',4'-Tetradehydro-psi,psi-carotene; 7,8,7',8'-Tetradehydro-beta,beta-carotene-3,3'-diol; 3,4,3',4'- Tetradehydro-beta,beta-carotene-2,2'-dione; 3',4',7',8'-Tetradehydro- beta,beta-caroten-3-ol; 3,4,3',4'Tetradehydrolycopene; 6,7,6',7'- Tetradehydro-5,6,5',6'-tetrahydro-beta,beta-carotene-3,3'-diol; 6,7, 6',7'-Tetradehydro-5,6,5',6'-tetrahydro-beta,beta-carotene-3,5,3',5'-tetrol; 7,8,7',8'-Tetradehydrozeaxanthin; 3,4,3',4'-Tetradehydrobisanhydrobacterioruberin; 5,6,5',6'-Tetrahydrocanthaxanthin; 7,8,7',8'-Tetrahydrocapsorubin; Tetrahydro-beta-carotene; 7,8,7',8'-Tetrahydro-beta,beta-carotene; 7',8',11',12'-Tetrahydro-beta,psi-carotene; 7',8',11',12'-Tetrahydro-gamma-carotene; 7',8',11',12'-Tetrahydro-gamma,psi-carotene; 1,2,7,8-Tetrahydro-psi,psi-carotene; 1,2,1',2'-Tetrahydro-psi,psi-carotene; 7,8,11,12-Tetrahydro-psi,psi-carotene; 7,8,7',8'-Tetrahydro-psi,psi-carotene; 5,6,5',6'-Tetrahydro-beta,beta-carotene-4,4'-diol; 7,8,7',8'-Tetrahydro-beta,beta-carotene-3,3'-diol; 7',8',9',10'-Tetrahydro-beta,psi-carotene-3,17'-diol; 1,2,1',2'-Tetrahydro-psi,psi-carotene-1,1'-diol; 5,6,5',6'-Tetrahydro-beta,beta-carotene-4,4'-dione; 5,6,5',6'-Tetrahydro-beta,beta-carotene-3,5,6,3',5',6'-hexol; 1,2,7,8-Tetrahydro-psi,psi-caroten-1-ol; 1,2,7',8'-Tetrahydro-psi,psi-caroten-1-ol; 7,8,11,12-Tetrahydro-4,4'-diapocarotene; 7,8,7',8'-Tetrahydro-4,4'-diapocarotene; Tetrahydrolycopene; 1,2,1',2'-Tetrahydrolycopene; 5,6,5',6'-Tetrahydrolycopene; 7,8,11,12-Tetrahydrolycopene; 7,8,7',8'-Tetrahydrolycopene; 7',8',11',12'-Tetrahydrolycopene; 1,2,1',2'-Tetrahydrolycopene-1,1'-diol; 1,2,1',2'-Tetrahydroneurosporene; 3,4,11',12'-Tetrahydrospheroidene; 3,4,7,8-Tetrahydrospirilloxanthin; 3,4,3',4'-Tetrahydrospirilloxanthin; 3,4,3',4'-Tetrahydrospirilloxanthin-20-al; 5,6,5',6'-Tetrahydro-3,4,3',4'-tetrol 4,4'-disulfate; 2,3,2',3'-Tetrahydroxy-beta,beta-carotene-4,4'-dione; 2,3,2',3'-Tetrahydroxy-beta,beta-caroten-4-one; 3,19,3',17'-Tetrahydroxy-beta,chi-caroten-6'-one 3-sulfate; 3,5,3',5'-Tetrahydroxy-6',7'-didehydro-5,8,5',6'-tetrahydro-beta,beta-caroten-8-one; 3,3',5,5'-Tetrahydroxy-6'-hydro-7-dehydro-beta-carotene; 3,4,3',4'-Tetrahydroxypirardixanthin; 3,4,3',4'-Tetrahydroxy-5,6,5',6'-tetrahydro-beta,beta-carotene; (3,4,3'4')-Tetraketo-beta-carotene; 4,5,4',5'-Tetraketo-beta-carotene; Thiothece-425; Thiothece-460; Thiothece-474; Thiothece-478; Thiothece-484; Thiothece-OH-484; Tilefishxanthin I; Tilefishxanthin II; Tilefishxanthin III; Tilefishxanthin IV; Torularhodin; Torularhodinaldehyde; Torularhodin methyl ester; Torulenal; Torulene; Torulenecarboxylic acid; 2,3,2'-Trihydroxy-beta,beta-caroten-4-one; 3, 3',4'-Trihydroxy-beta,beta-caroten-4-one; 3,4,3'-Trihydroxy-beta, chi-caroten-6'-one; 3,3',5'-Trihydroxy-6',7'-dehydro-alpha-carotene; 3,3',8'-Trihydroxy-7,8-didehydro-beta,chi-carotene-4,6'-dione; 3,3',8'-Trihydroxy-7,8-didehydro-beta,chi-caroten-6'-one; 3,19,3'-Trihydroxy-7,8-didehydro-beta,chi-caroten-6'-one 3-sulfate; 3,1',2'-Trihydroxy-3',4'-didehydro-1',2'-dihydro-beta,psi-caroten-4-one; 3,5,19-Trihydroxy-6,7-didehydro-5,6,7',8'-tetrahydro-7'-apo-beta-caroten-8'-one 3-acetate 19-hexanoate; 3,5,6'-Trihydroxy-6,7-didehydro-5,6,7',8'-tetrahydro-beta,[epsi]-carotene-3',8'-dione; 3,5,3'-Trihydroxy-5,6-dihydro-beta-carotene; 3,3',5'-Trihydroxy-5',6'-dihydro-beta-carotene 5',6'-epoxide; 3,19,3'-Trihydroxy-7,8-dihydro-beta,[epsi]-caroten-8-one; 3,19,3'-Trihydroxy-7,8-dihydro-beta,beta-caroten-8-one 19-laurate; 3,6,3'-Trihydroxy-7,8-dihydro-gamma,[epsi]-caroten-8-one; 3,3',19-Trihydroxy-7,8-dihydro-8-oxo-alpha-carotene; 3,3',6'-Trihydroxy-5,8-epoxy-alpha-carotene; 3,4,4'-Trihydroxypirardixanthin; 1,1',2'-Trihydroxy-3,4,3',4'-tetradehydro-1,2,1',2'-tetrahydro-psi,psi-caroten-2-one; 3,4,4'-Trihydroxy-5,6,5',6'-tetrahydro-beta,beta-carotene; Trikentriorhodin; 3,4,4'-Triketo-beta-carotene; 3,1',2'-Trimethoxy-3',4'-didehydro-1',2' dihydro-beta,psi-caroten-4-one; Triophaxanthin; Triphasiaxanthin; Trisanhydrobacterioruberin; Trollein; Trollichrome; Trolliflavin; Trolliflor; Trollixanthin; Tunaxanthin; Uriolide; Vaucheriaxanthin; Violaxanthin; Violeoxanthin; Violerythrin; Warmingol; Warmingone; Webbiaxanthin; Xanthophyll; Xanthophyll K (1); Xanthophyll K (1)S; Xanthophyll dipalmitate; Xanthophyll epoxide; alpha-Zeacarotene; beta-Zeacarotene; beta (1)- Zeacarotene; alpha-Zeacarotene-3,17'-diol; beta-Zeacarotene-3,17'-diol; beta-Zeacaroten-3-ol; Zeaxanthene; Zeaxanthin; Zeaxanthin diepoxide; Zeaxanthin dimethyl ether; Zeaxanthin dirhamnoside; Zeaxanthin dipalmitate; Zeaxanthin 5,6-epoxide; Zeaxanthin 5,8-epoxide; Zeaxanthin furanoxide; Zeaxanthin monomethyl ether; Zeaxanthin monorhamnoside; Zeaxanthol; and Zeinoxanthin.

The above list of naturally occurring carotenoids is meant to be a non-limiting example of naturally occurring carotenoids. This list is not comprehensive as more naturally occurring molecules are being discovered which will fall within the category of carotenoids.

### Sulfonylureas

Non-limiting examples of sulfonylureas include, but are not limited to acetohexamide, DiaBeta, glibenclamide, gliclazide, glipizide (Glucotrol), glyclopyramide, chlorpropamide, tolazamide, tolbutamide, glimepiride (Amaryl), tolbutamide and meglitinide analogues (for example, repaglinide, nateglinide, meglitinide and mitiglinide (KAD-1229)) and the like.

### Niacin and related derivatives

The term niacin is the generic descriptor for nicotinic acid (pyridine-3-carboxylic acid) and its derivatives. Non-limiting examples of nicotinic acid derivatives include nicofuranose, Acipimox (5-methyl pyrazine-2-carboxylic acid 4-oxide), niceritrol, probucol, isonicotinic acid, Cholexamin, oxiniacic acid, nicoclonate, nicomol, NIASPAN, nicerikol and tocopherol nicotinate.

Further examples of other active agents which may be suitable for this invention include, without limitation, AGI-1067, abecarnil, acamprostate, acavir, acebutolol, aceclofenac, acemetacin, acetaminophen, acetaminosalol, acetanilide, acetohexamide, acetophenazine maleate, acetophenazine, acetoxolone, acetoxypregnenolone, acetretin, acrisorcin, acrivastine, acyclovir, adinazolam, adiphenine hydrochloride, adrafinil, adrenolone, agatroban, ahnitrine, akatinol, alatrofloxacin, albendazole, albuterol, aldioxa, alendronate, alfentanil, alibendol, alitretinoin, allopurinol, allylamines, allylestrenol, alminoprofen, almotriptan, alosetron, aloxiprin, alprazolam, alprenolol, amantadine, ambucetamide, amidephrine, amidinomycin, amiloride, aminoarylcarboxylic acid derivatives, aminoglutethimide, aminoglycosides, aminopentamide, aminopromazine, aminorex, amiodarone, amiphenazole, amiprilose, amisulpride, amitriptyline, amlexanox, amlodipine, amodiaquine, amosulalol, amotriphene, amoxapine, amoxicillin, amphecloral, amphetamine, amphomycin, amphotericin, ampicillin, ampiroxicam, amprenavir, amrinone, amsacrine, amyl nitrate, amylobarbitone, anagestone acetate, anastrozole, andinocillin, androstenediol, androstenediol-17- acetate, androstenediol- 17-benzoate, androstenediol-3-acetate, androstenediol-3-acetate-17- benzoate, androstenedione, androsterone acetate, androsterone benzoate, androsterone propionate, androsterone, angiotensin, anidulatungin, aniracetam, apazone, apicycline, apoatropine, apomorphine, apraclonidine, aprepitant, aprotinin, arbaprostil, ardeparin, aripiprazole, amikacin, arotinolol, arstiinol, arylacetic acid derivatives, arylalkylamines, arylbutyric acid derivatives, arylcarboxylic acids, arylpiperazines, arylpropionic acid derivatives, aspirin, astemizole, atenolol, atomoxetine, atorvastatin, atovaquone, atropine, auranofn, azapropazone, azathioprine, azelastine, azetazolamide, azithromycin, baclofen, bambuterol, bamethan, barbitone, barnidipine, basalazide, beclamide, beclobrate, befimolol, bemegride, benazepril, bencyclane, bendazac, bendazol, bendroflumethiazide, benethamine penicillin, benexate hydrochloride, benfurodil hemisuccinate, benidipine, benorylate, bentazepam, benzhexol, benziodarone, benznidazole, benzoctamine, benzodiazepine derivatives, benzodiazepine, benzonatate, benzphetamine, benzylmorphine, beperiden, bephenium hydroxynaphthoate, bepridil, betahistine, betamethasone, betaxolol, bevantolol, bevonium methyl sulfate, bexarotene, bezadoxifine, bezafibrate, bialamicol, biapenem, bicalutamide, bietamiverine, bifonazole, binedaline, binifibrate, biricodar, bisacodyl, bisantrene, bisoprolol, bitolterol, bopindolol, boswellic acid, bradykinin, bretylium, bromazepam, bromocriptine, bromperidol, brotizolam, brovincamine, buciclate, bucloxic acid, bucumolol, budralazine, buieniode, bufetolol, buflomedil, bufuralol, bumetanide, bunitrolol, bupranolol, buprenorphine, bupropion, buspirone, busulfan, butalamine, butorphanol, butaverine, butenatme, butidrine hydrochloride, butobarbitone, butoconazole nitrate, butoconazole, butofilol, butropium bromide, cabergoline, calcifediol, calcipotriene, calcitriol, caldibine, cambendazole, camioxirole, camostat, campesterol, camptothecin, candesartan, candoxatril, capecitabine, caprate, capsaicin, captopril, carazolol, carbacephems, carbamates, carbamazepine, carbapenems, carbarsone, Carbatrol, carbenoxolone, carbimazole, carbromal, carbuterol, carisoprodol, carotenes, caroverine, carteolol, carvedilol, cefaclor, cefazolin, cefbuperazone, cefepime, cefoselis, ceftibuten, celecoxib, celiprolol, cephaeline, cephalosporin C, cephalosporins, cephamycins, cerivastatin, certoparin, cetamolol, cetiedil, cetirizine, cetraxate, chloracizine, chlorambucil, chlorbetamide, chlordantoin, chlordiazepoxide, chlormadinone acetate, chlormethiazole, chloroquine, chlorothiazide, chlorpheniramine, chlorphenoxamide, chlorphentermine, chlorproguanil, chlorpromazine, chlorpropamide, chlorprothixene, chlortetracycline, chlorthalidone, cholecalciferol, chromonar, ciclesonide, ciclonicate, cidofovir, ciglitazone, cilansetron, cilostazol, cimetidine, cimetropium bromide, cinepazet maleate, cinnamedrine, cinnarizine, cinolazepam, cinoxacin, ciprofibrate, ciprofloxacin, cisapride, cisplatin, citalopram, citicoline, clarithromycin, clebopride, clemastine, clenbuterol, clidanac, clinofibrate, clioquinol, clobazam, clobenfurol, clobenzorex, clofazimine, clofibrate, clofibric acid, cloforex, clomipramine, clonazepam, clonidine, clonitrate, clopidogrel, clopirac indomethacin, cloranolol, cloricromen, clorprenaline, clortermine, clotiazepam, clotrimazole, cloxacillin, clozapine, cmepazide, codeine methyl bromide, codeine phosphate, codeine sulfate, codeine, colloidal bismuth subcitrate, cromafiban, cromolyn, cropropamide, crotethamide, curcumin, cyclandelate, cyclarbamate, cyclazocine, cyclexedrine, cyclizine, cyclobenzaprine, cyclodrine, cyclonium iodide, cyclopentamine, cyclosporin, cypionate, cyproheptadine, cyproterone acetate, cytarabine, dacarbazine, dalfopristine, dantrolene sodium, dapiprazole, darodipine, decanoate, decitabine, decoquinate, dehydroemetine, delavirdine, delaviridine, demeclo cycline, denopamine, deramciclone, descitalopram, desipramine, desloratadine, 3- ketodesogeskel, desomorphine, desoxymethasone, detomidine, dexamphetamine, dexanabinol, dexchlorpheniramine, dexfenfluramine, dexmethylphenidate, dexrazoxane, dextroamphetamine sulfate, dextroamphetamine, dextropropoxyphene, DHEA, diacetate, diamorphine, diazemine, diazepam, diaziquinone, diazoxide, dibromopropamidine, dichlorophen, diclofenac, dicoumarol, didanosine, dideoxyadenosine, diethylpropion, difemerine, difenamizole, diflunisal, digitoxin, digoxin, dihydroergotamine, dihydrocodeine, dihydrocodeinone enol acetate, dihydroergotamine mesylate, dihydroergotamine, dihydrogesterone, dihydromorphine, dihydropyridine derivatives, dihydrostreptomycin, dihydrotachysterol, dihydroxyaluminum acetylsalicylate, diiodohydroxyquinoline, diisopromine, dilazep, dilevalol, diltiazem, diloxanide furoate, diloxanide, diltiazem, dimefline, dimenhydrinate, dimethisterone, dimetotrine, dimorpholamine, dinitolmide, dioxaphetyl butyrate, dioxethedrine, diphemethoxidine, diphenhydramine, diphenoxylate, diphetarsone, dipivefrin, diponium bromide, dipyridamole, dirithromycin, disopyramide, divalproex sodium, dofetilide, domperidone, donepezil, dopexamine, dopradil, dosmalfate, doxapram, doxazosin, doxefazepam, doxepin, doxycycline, drofenine, dromostanolone propionate, dromostanolone, dronabinol, droperidol, droprenilamine, d-threo - methylphenidate, duloxetine, ebrotidine, eburnamonine, ecabet, ecenofloxacin, econazole nitrate, edavarone, edoxudine, efavirenz, effivarenz, efloxate, eledoisin, eletriptan, elgodipine, ellipticine, emepronium bromide, emetine, enalapril, enanthate, encainide, enlopitat, enoximone, enprostil, entacapone, epanolol, ephedrine, epinastine, epinephrine, epirubicin, eplerenone, eprosartan, ergocalciferol, ergoloid mesylates, ergotamine, ertapenum, erythromycin, erytlirityl tetranitrate, esaprazole, escitalopram, esmolol, esomeprazole, esonarimod, estazolam, estradiol benzoate, estramustine, eskiol succinate, estrone acetate, estrone sulfate, etafedrine, etafenone, ethacrynic acid, ethamivan, ethinamate, ethinyleskadiol 3-acetate, ethinyleskadiol 3-benzoate, ethinylestradiol, ethionamide, ethisterone (17a-ethinyltestosterone), ethopropazine, ethotoin, ethoxyphenamine, ethylestrenol, ethylmorphine, ethylnorepinephrine, ethynodiol diacetate, etodolac, etofibrate, etoposide, etoricoxib, etretinate, everolimus, exalamide, examestane, examorelin, ezemitibe, falecalcitriol, famciclovir, famotidine, fantofarone, farapenum, farglitazar, fasudil, felbamate, felodipine, fenalamide, fenbuLen, fenbutrazate, fendiline, fenfluramine, fenoldopam, fenoprofen, fenoterol, fenoverine, fenoxazoline, fenoxedil, fenpiprane, fenproporex, fenspiride, fentanyl, fexofenadine, flavoxate, flecainide, flopropione, floredil, floxuridine, fluconazole, flucytosine, fludarabine, fludiazepam, fludrocortisone, flulenamic acid, flunanisone, flunarizine, flunisolide, flunitrazepam, fluocortolone, fluoxetine, flupenthixol decanoate, fluphenazine decanoate, fluphenazine enanthate, fluphenazine, fluproquazone, flurazepam, flurbiprofen, flurogestone acetate, fluticasone propionate, fluvastatin, fluvoxamine, fominoben, formoterol, foscarnet, foscarnet, fosinopril, fosphenytoin, frovatriptan, fudosteine, fumagillin, furazolidone, furazolidone, furfurylmethyl amphetamine, furosemide, gabapentin, gabexate, gaboxadol, galanthamine, gallopamil, gammaparin, ganciclovir, ganglefene, gefarnate, gemcitabine, gemfibrozil, gepirone, gestadene, ghrelin, glatiramer, glaucarubin, glibenclamide, gliclazide, glimepiride, glipizide, gluconic acid, glutamic acid, glyburide, glyceryl trinitrate, glimepiride, granisetron, grepafloxacin, griseofulvin, guaiazulene, guanabenz, guanfacine, halofankine, haloperidol decanoate, haloperidol, haloxazolam, hepronicate, heptanoate, hexobendine, hexoprenaline, hydramitrazine, hydrazides, hydrocodone, hydrocortisone, hydromorphone, hydroxyamphetamine, hydroxymethylprogesterone acetate, hydroxymethylprogesterone, hydroxyprogesterone acetate, hydroxyprogesterone caproate, hydroxyprogesterone, hymecromone, hyoscyamine, ibopamine, ibudilast, ibutenac, ibuprofen, ibutilide, idoxuridine, ifenprodil, igmesine, iloprost, imatinib, imidapril, imidazoles, imipenem, imipramine, imolamine, incadronic acid pergolide, indanazoline, indenolol, indinavir, indomethacin, indoramin, inosine pranobex, inositol niacinate, iodoquinol, ipidracine, iproniazid, irbesartan, irinotecan, irsogladine, isobutyrate, isocaprate esters, isoetharine, isometheptene, isoproterenol, isosorbide dinitrate, isosorbide mononitrate, isosorbide dinitrate, isoxsuprine, isradipine, itasetron, itraconazole, itramintosylate, ivermectin, kallidin, kallikrein, kanamycin, ketamine, ketoconazole, ketoprofen, ketorolac, ketotifen, labetalol, lafutidine, lamifiban, lamivudine, lamotrigine, lanatoside c, lansoprazole, lasofoxifene, leflunomide, leminoprazole, lercanidipine, lesopitron, letrozole, leucovorin, levalbuterol, levallorphan, levetiracetam, levetriacetam, levobunolol, levodopa, levofloxacin, levophacetoperane, levorphanol, lidocaine, lidoflazine, lifibrol, limaprost, linezolid, lintitript, liranaftate, lisinopril, lisuride, lobeline, lobucavir, lodoxamide, lomefloxacin, lomerizine, lomustine, loperamide, lopinavir, loprazolam, loracarbef, loratadine, lorazepam, lorefloxacin, lormetazepam, losartan, lovastatin, lovastatin, loxapine succinate, loxapine,1-threo methylphenidate, lumiracoxib, lysine acetylsalicylate, lysozyme, lisuride, mabuterol, mafenide, magnesium acetylsalicylate, malgramostin, mannitol hexanitrate, maprotiline, mazindol, mebendazole, meclizine, meclofenamic acid, mecloxaminepentapiperide, medazepam, medibazine, medigoxin, medrogestone, medroxyprogesterone acetate, mefenamic acid, mefenorex, mefloquin, mefloquine, megestrol acetate, melengestrol acetate, melphalan, memantine, mepenzolate bromide, meperidine, mephenoxalone, mephentermine, mepindolol, mepixanox, meprobamate, meptazinol, mercaptopurine, meropenum, mesalamine, mesalazine, mesoridazine besylate, mesoridazine, metaclazepam, metamfepramone, metampicillin, metaproterenol, metaraminol, methacycline, methadone hydrochloride, methadone, methamphetamine, methaqualone, metharnphetamine, methoin, methotrexate, methoxamine, methsuximide, methylhexaneamine, methylphenidate d-threo-methylphenidate, methylphenidate, methylphenobarbitone, methylprednisolone, methysergide, metiazinic acid, metizoline, metoclopramide, metolazone, metoprolol, metoxalone, metripranolol, metronidazole, mexiletine, metaxalone, mianserin, mibefradil, miconazole, midazolam, midodrine, miglitol, milnacipran, milrinone, minoxidil, mirtazapine, misoprostol, mitomycin, mitotane, mitoxantrone, mizolastine, modafinil, mofebutazone, mofetil, molindone hydrochloride, molindone, molsidomine, monatepil, montelukast, Monteplase, moprolol, moricizine, morphine hydrochloride, morphine sulfate, morphine, morpholine salicylate, mosapramine, moxifloxacin, moxisylyte, moxonidine, mycophenolate, nabumetone, nadolol, nadoxolol, nadroparin, nafamostat, nafronyl, naftopidil, nalbuphine, nalidixic acid, nalmefene, nalorphine, naloxone, naltrexone, nandrolone benzoate, nandrolone cyclohexanecarboxylate, nandrolone cyclohexane-propionate, nandrolone decanoate, nandrolone furylpropionate, nandrolone phenpropionate, naphazoline, naproxen, naratriptan, natamycin, nateglinide, nedocromil, nefazodone, nefopam, nelfinavir, nemonapride, neomycin undecylenate, neomycin, neokofin, nesiritide, n- ethylamphetamine, nevirapine, nexopamil, nicametate, nicardipine, nicergoline, nicofibrate, nicofuranose, nicomorphine, nicorandil, nicotinyl alcohol, nicoumalone, nifedipine, nifenalol, nikethamide, nilutamide, nilvadipine, nimodipine, nimorazole, nipradilol, nisoldipine, nitisonone, nitrazepam, nitrofurantoin, nitrofurazone, nitroglycerin, nizatidine, norastemizole, norepinephrine, norethynodrel, norfenefrine, norfloxacin, norgestimate, norgeskel, norgestrienone, normethadone, normethisterone, normorphine, norpseudoephedrine, nortriptyline, novantrone, nylidrin, nystatin, octamylamine, octodrine, octopamine, ofloxacin, olanzapine, olanzapine, olapatadine, olmesartan, Olapatadine, olsalazine, omapatrilat, omeprazole, ondansetron, opium, oprevelkin, orlistat, ornidazole, ornoprostil, oseltamivir, oxaliplatin, oxamniquine, oxandrolone, oxantel embonate, oxaprozin, oxatomide pemirolast, oxatomide, oxazepam, oxcarbazepine, oxfendazole, oxiconazole, oxiracetam, oxolinic acid, oxprenolol, oxycodone, oxyfedrine, oxymetazoline, oxymorphone, oxyphenbutazone, oxyphencyclimine, ozagrel, paclitaxel, palonosetron, pantoprazole, papaverine, paricalcitol, paramethadione, parecoxib, pariprazole, paromomycin, paroxetine, parsalmide, pazinaclone, pemoline, penbutolol, penciclovir, penicillin G benzathine, penicillin G procaine, penicillin V, penicillins, pentaerythritol tetranitrate, pentaerythritol tetranitrate, pentapiperide, pentazocine, pentifylline, pentigetide, pentobarbitone, pentorex, pentoxifylline, pentrinitrol, pirbuterol, pirenzepine, pergolide, perhexiline, perindopril erbumine, perospone, perphenazine pimozide, perphenazine, phanquinone, phenacemide, phenacetin, phenazopyridine, phencarbamide, phendimetrazine, phenelzine, phenindione, phenmetrazine, phenobarbitone, phenoperidine, phenothiazines, phenoxybenzamine, phensuximide, phentermine, phentolamine, phenylsalicylate, phenylacetate, phenylbutazone, phenylephrine hydrochloride, phenylpropanolamine hydrochloride, phenylpropanolamine hydrochloride, phenylpropyl-methylamine, phenytoin, phloroglucinol, pholedrine, physostigmine salicylate, physostigmine, phytonadiol, phytosterols, piapenum, picilorex, piclamilast, picrotoxin, picumast, pifarnine, pilsicainide, pimagedine, pimeclone, pimecrolimus, pimefylline, pimozide, pinaverium bromide, pindolol, pioglitazone, piperacillin, piperazine estrone sulfate, piperazine derivatives, piperilate, piracetam, piribedil, pirifibrate, piroxicam, pitavastatin, pizotyline, plaunotol, polaprezinc, polybenzarsol, polyestrol phosphate, practolol, pralnacasan, pramipexole, pranlukast, pravastatin, prazepam, praziquantel, prazosin, pregabalin, prenalterol, prenylamine, pridinol, prifinium bromide, primidone, primipramine, probenecid, probucol, procainamide, procarbazine, procaterol, prochlorperazine, proguanil, pronethalol, propafenone, propamidine, propatyl nitrate, propentoffyline, propionate, propiram, propoxyphene, propranolol, propylhexedrine, propylthiouracil, protokylol, protriptyline, proxazole, pseudoephedrine, purines, pyrantel embonate, pyrazoles, pyrazolones, pyridofylline, pyrimethamine, pyrimidines, pyrrolidones, quazepam, quetiapine, quetuapine, quinagolide, quinapril, quinestrol, quinfamide, quinidine, quinine sulfate, quinolones, quinupritin, rabalzotan, rabeprazole sodium, rabeprazole, racefimine, ramahroban, ramipril, ranitidine, ranolazine, ransoprazole, rasagiline, rebamipide, refludan, repaglinide, repinotan, repirinast, reproterol, reserpine, retinoids, ribavirin, rifabutine, rifampicin, rifapentine, rilmenidine, riluzole, rimantadine, rimiterol, rioprostil, risperidone, ritanovir, ritapentine, ritipenem, ritodrine, ritonavir, rivastigrnine, rizatriptan, rociverine, rofecoxib, rohypnol, rolipram, remoxipride, ronifibrate, ropinirole, ropivacaine, rosaprostol, rosiglitazone, rosuvastatin, rotinolol, rotraxate, roxatidine acetate, roxindole, rubitecan, salacetamide, salicin, salicylamide, salicylic acid derivatives, salmeterol, saquinavir, saquinavir, scopolamine, secnidazole, selegiline, semotiadil, sertindole, sertraline, sibutramine, sildenafil, simvastatin, siramesine, sirolimus, sitaxsentan, sofalcone, somotiadil, sorivudine, sotalol, soterenol, sparfloxacin, spasmolytol, spectinomycin, spiramycin, spizofurone, stavudine, streptomycin, succinylsulfathiazole, sucralfate, sufentanil, sulconazole nitrate, sulfacetamide, sulfadiazine, sulfaloxicacid, sulfarside, sulfmalol, sulindac, suloctidil, sulphabenzamide, sulphacetamide, sulphadiazine, sulphadoxine, sulphafurazole, sulphamerazine, sulphamethoxazole, sulphapyridine, sulphasalazine, sulphinpyrazone, sulpiride, sulthiame, sultopride, sulbroponium, sumanirole, sumahriptan, sunepihon, superoxide dismutase, suplatast, suramin sodium, synephrine, tacrine, tacrolimus, tacrolimus, tadalafil, talinolol, talipexole, tamoxifen, tamsulosin, targretin, tazanolast, tazarotene, tazobactam, tecastimezole, teclozan, tedisamil, tegaserod, telenzepine, telmisartan, temazepam, teniposide, teprenone, terazosin, terbinafine, terbinafine, terbutaline sulfate, terbutaline, terconazole, terfenadine, terodiline, terofenamate, tertatolol, testolactone, 4-dihydrotestosterone, tetracyclics, tetracycline, tetrahydrocannabinol, tetrahydrozoline, thalidomide, theofibrate, thiabendazole, thiazinecarboxamides, thiocarbamates, thiocarbamizine, thiocarbarsone, thioridazine, thiothixene, tiagabine, tiamenidine, tianeptine, tiaprofenic acid, tiaramide, ticlopidine, tigloidine, tilisolol, timolol, tinidazole, tinofedrine, tinzaparin, tioconazole, tipranavir, tirapazamine, tirofiban, tiropramide, titanicene, tizanadine, tizanidine, tizinadine, tocainide, tolazamide, tolazoline, tolbutamide, tolcapone, tolciclate, tolfenamic acid, toliprolol, tolteridine, tolterodine, tonaberstat, topiramate, topotecan, torsemide, toremifene cibrate, toremifene, tosufloxacin, tramadol, tramazoline, trandolapril, tranilast, tranylcypromine, trapidil, traxanox, trazodone, tretoquinol, triacetin, triamcinolone, triamterine, triamterene, triazolam, trifluoperazine hydrochloride, trifluoperazine, triflupromazine, trihexyphenidyl, trimazosin, trimebutine, trimetazidine, trimethoprim, trimgestone, trimipramine, trimoprostil, trithiozine, troglitazone, trolnibrate phosphate, tromethamine, tropicamide, trovafloxacin, troxipide, tuaminoheptane, tulobuterol, tymazoline, tyramine, undecanoate, undecenoic acid, urinastatin, valacyclovir, valdecoxib, valerate, valganciclovir, valproic acid, valsartan, vancomycin, vardenafil, venlafaxine, venorelbine, verapamil, vidarabine, vigabakin, vincamine, vinpocetine, viomycin, viquidil, visnadine, vitamin a derivatives, vitamin a, vitamin b₂, vitamin d, vitamin e, vitamin k, voglibose, voriconazole, xaliproden, xamoterol, xanthinol niacinate, xenytropium bromide, xibenolol, ximelagatran, xylometazoline, yohimbine, zacopride, zafirlukast, zalcitabine, zaleplon, zanamivir, zatebradine, ziconotide, zidovudine, zileuton, zimeldine, zinc propionate, ziprasidone, zolimidine, zolmitriptan, zolpidem, zonisamide, zopiclone, and mixtures thereof.

The pharmaceutical compositions according to the invention may be formulated as pharmaceutical compositions suitable for oral, buccal, parenteral, transdermal or rectal administration or in a form suitable for administration by inhalation or insufflation. Parenteral administration includes subcutaneous injections, intravenous, intramuscular, intrasternal injection, or infusion techniques.

The pharmaceutical compositions may also be formulated as a therapeutically effective amount in a dosage unit form for ease of administration and uniformity of dosage. Dosage unit form as used in the specification and claims herein refers to physically discrete units suitable as unitary dosages, each unit containing a predetermined quantity of active ingredient calculated to produce the desired therapeutic effect in association with the required pharmaceutical carrier. Examples of such dosage unit forms are tablets (including scored or coated tablets), capsules, pills, powder packets, wafers, injectable solutions or suspensions, teaspoonfuls, tablespoonfuls and the like, and segregated multiples thereof Such dosage forms can also comprise, as in conventional practice, additional substances other than inert diluents, e.g., lubricating agents such as magnesium stearate. In the case of capsules, tablets, effervescent tablets, and pills, the dosage forms can also comprise buffering agents. Soft gelatin capsules can be prepared to contain a mixture of the active compounds or compositions of the invention and vegetable oil. Hard gelatin capsules can contain granules of the active compound in combination with a solid, pulverulent carrier such as lactose, saccharose, sorbitol, mannitol, potato starch, corn starch, amylopectin, cellulose derivatives of gelatin. Tablets and pills can be prepared with enteric coatings.

Liquid dosage forms for oral administration can include pharmaceutically acceptable emulsions, solutions, suspensions, syrups, and elixirs containing inert diluents commonly used in the art, such as water. Such compositions can also comprise adjuvants, such as wetting agents, emulsifying and suspending agents, and sweetening, flavoring, and perfuming agents.

Suppositories for vaginal or rectal administration of the compounds and compositions of the invention can be prepared by mixing the compounds or compositions with a suitable nonirritating excipient such as cocoa butter and polyethylene glycols which are solid at room temperature but liquid at body temperature, such that they will melt and release the drug.

Injectable preparations, for example, sterile injectable aqueous or oleaginous suspensions can be formulated according to the known art using suitable dispersing agents, wetting agents and/or suspending agents. The sterile injectable preparation can also be a sterile injectable solution or suspension in a nontoxic parenterally acceptable diluent or solvent, for example, as a solution in 1,3-butanediol. Among the acceptable vehicles and solvents that can be used are water, Ringer's solution, and isotonic sodium chloride solution. Sterile fixed oils are also conventionally used as a solvent or suspending medium.

The compositions of this invention can further include conventional excipients, i.e., pharmaceutically acceptable organic or inorganic carrier substances suitable for oral, buccal, parenteral, transdermal or rectal administration or in a form suitable for administration by inhalation or insufflation, which do not deleteriously react with the active compounds. Suitable pharmaceutically acceptable carriers include, for example, water, salt solutions, alcohol, vegetable oils, polyethylene glycols, gelatin, lactose, amylose, magnesium stearate, talc, surfactants, silicic acid, viscous paraffin, perfume oil, fatty acid monoglycerides and diglycerides, petroethral fatty acid esters, hydroxymethyl-cellulose, polyvinylpyrrolidone, and the like. The pharmaceutical preparations can be sterilized and if desired, mixed with auxiliary agents, e.g., lubricants, preservatives, stabilizers, wetting agents, emulsifiers, salts for influencing osmotic pressure, buffers, colorings, flavoring and/or aromatic substances and the like which do not deleteriously react with the active compounds. For parenteral application, particularly suitable vehicles consist of solutions, preferably oily or aqueous solutions, as well as suspensions, emulsions, or implants. Aqueous suspensions may contain substances that increase the viscosity of the suspension and include, for example, sodium carboxymethyl cellulose, sorbitol and/or dextran. Optionally, the suspension may also contain stabilizers.

Both tablets and capsules may also be manufactured in the form of sustained release formulations, such that they provide a controlled continuous release of the compounds according to the invention over a period of hours.

Administration of the compositions of the present invention will be in an amount sufficient to achieve a therapeutic effect as recognized by one of ordinary skill in the art.

The dosage of any compositions of the present invention will vary depending on the symptoms, age and body weight of the patient, the nature and severity of the disorder to be treated or prevented, the route of administration, and the form of the subject composition. Any of the subject formulations maybe administered in a single dose or in divided doses. Dosages for the compositions of the present invention may be readily determined by techniques known to those of skill in the art or as taught herein.

The dosage range for nebivolol ranges from about 0.1 mg to about 100 mg per day. In another embodiment, the dosage range may be from about 0.75 mg to about 50 mg per day. In yet another embodiment, the dosage range may be from about 1.25 mg to about 10 mg per day.

In certain embodiments, the dosage of the co-active compounds will generally be in the range of about 0.01 ng to about 1 g per kg body weight, specifically in the range of about 1 ng to about 0.1 g per kg, and more specifically in the range of about 100 ng to about 10 mg per kg body weight.

An effective dose or amount, and any possible affects on the timing of administration of the formulation, may need to be identified for any particular composition of the present invention. This may be accomplished by routine experiment as described herein, using one or more groups of animals (preferably at least 5 animals per group), or in human trials if appropriate. The effectiveness of any subject composition and method of treatment or prevention may be assessed by administering the composition and assessing the effect of the administration by measuring one or more applicable indices, and comparing the post-treatment values of these indices to the values of the same indices prior to treatment.

The precise time of administration and amount of any particular subject composition that will yield the most effective treatment in a given patient will depend upon the activity, pharmacokinetics, and bioavailability of a subject composition, physiological condition of the patient (including age, sex, disease type and stage, general physical condition, responsiveness to a given dosage and type of medication), route of administration, and the like. The guidelines presented herein may be used to optimize the treatment, e.g., determining the optimum time and/or amount of administration, which will require no more than routine experimentation consisting of monitoring the subject and adjusting the dosage and/or timing.

While the subject is being treated, the health of the patient may be monitored by measuring one or more of the relevant indices at predetermined times during the treatment period. Treatment, including composition, amounts, times of administration and formulation, may be optimized according to the results of such monitoring. The patient may be periodically reevaluated to determine the extent of improvement by measuring the same parameters. Adjustments to the amount(s) of subject composition administered and possibly to the time of administration may be made based on these reevaluations.

Treatment may be initiated with smaller dosages which are less than the optimum dose of the compound. Thereafter, the dosage may be increased by small increments until the optimum therapeutic effect is attained.

The use of the subject compositions may reduce the required dosage for any individual agent contained in the compositions (e.g., the steroidal anti inflammatory drug) because the onset and duration of effect of the different agents may be complimentary.

Toxicity and therapeutic efficacy of subject compositions may be determined by standard pharmaceutical procedures in cell cultures or experimental animals, e.g., for determining the LD₅₀ and the ED₅₀.

The data obtained from the cell culture assays and animal studies may be used in formulating a range of dosage for use in humans. The dosage of any subject composition lies preferably within a range of circulating concentrations that include the ED₅₀ with little or no toxicity. The dosage may vary within this range depending upon the dosage form employed and the route of administration utilized. For compositions of the present invention, the therapeutically effective dose may be estimated initially from cell culture assays.

In general, the doses of an active agent will be chosen by a physician based on the age, physical condition, weight and other factors known in the medical arts.

Those of skill in treating subjects suffering from a vascular disease, for instance, an increased blood pressure, could easily determine the therapeutically effective amount from the test results presented hereinafter. In general it is contemplated that an effective daily dose of the 4(S)-OH-(R,S,S,S)-nebivolol or its pharmaceutically acceptable acid-addition salt would be from about 0.01 mg/kg to about 50 mg/kg body weight, in particular from about 0.1 mg/kg to about 10 mg/kg body weight, and preferably from about 0.1 mg/kg to about 1 mg/kg body weight.

The invention also provides pharmaceutical kits comprising one or more containers filled with one or more of the ingredients of the pharmaceutical compositions of the invention, including the 4-hydroxylated nebivolol metabolite. Such kits can also include, for example, other compounds and/or compositions (e.g., diuretics, digoxin, compounds used to treat cardiovascular diseases, therapeutic agents, permeation enhancers, lubricants, and the like), a device(s) for administering the compounds and/or compositions, and written instructions in a form prescribed by a governmental agency regulating the manufacture, use or sale of pharmaceuticals or biological products, which instructions can also reflects approval by the agency of manufacture, use or sale for human administration.

Another embodiment of the invention relates to the local administration of the 4-hydroxylated metabolite of nebivolol of the invention, and, optionally, at least one additional therapeutic agent to treat injured tissue, such as damaged blood vessels.

The compound used in the compositions of the present invention and, optionally, other therapeutic agents, can be incorporated into a natural or synthetic matrix which can then be applied with specificity to a biological site of interest. Accordingly the nebivolol metabolite is "bound to the matrix" which means that the nebivolol metabolite along with other therapeutic agents is physically and/or chemically associated with part of, incorporated with, attached to, or contained within the natural or synthetic matrix.

Any of a wide variety of natural or synthetic polymers can be used as the matrix in the context of the invention. It is only necessary for the matrix to be biologically acceptable. Exemplary matrixes suitable for use in the invention are polymers including, for example, polyolefins (such as polystyrene, polypropylene, polyethylene, high density polyethylene, polytetrafluorethylene, polyvinylidene diflouride and polyvinylchloride), polyethylenimine or derivatives thereof, polyethers (such as polyethylene glycol), polyesters (such as poly-L-lactic acid, poly-D, L-lactic, poly-D-lactic, polyglycolic, poly-(lactide/glycolide)), polyanhydrides, polyhydroxybutyrates, polyamides (such as nylon), polyurethanes, polyurethane copolymers (such as pellethane polymers), polyacrylates (such as polymethacrylate, poly (2-(methacryloyloxyethyl)-2'-(trimethylammonium)ethyl phosphate inner salt-con-dodecyl methacrylate), mixtures of polymers (such as polylactic acid/polylysine copolymers, polyurethane/polyester copolymers, polyurethane/polyether copolymers, nylon/polyether copolymers, such as vestamid), biopolymers (such as peptides, proteins, oligonucleotides, antibodies, peptide hormones, glycoproteins, glycogen and nucleic acids), starburst dendrimers, natural fibrous matrix (such as filter paper), synthetic fibrous matrix materials (such as three-dimensional lattice of synthetic polymers and copolymers) and the like. Exemplary polymers are described in U.S. Pat. Nos. 5,705,583, 5,770,645 and 5,994,444.

The physical and structural characteristics of the matrixes suitable for use in the invention are not critical, but depend on the application. It will be appreciated by one skilled in the art that where the matrix-metabolite of nebivolol composition of the invention is intended for local, relatively short term administration or similar administration they need not be biodegradable. For some uses, such as postangioplasty, coronary bypass surgery or intimal hyperplasia associated with vascular graft implants or the like, it may be desirable for the matrix to slowly dissolve in a physiological environment or to be biodegradable or bioresorbable.

In preventing and/or treating cardiovascular diseases or disorders, the nebivolol metabolite used in the compositions of the present invention and optionally at least one therapeutic agent can be administered directly to the damaged vascular surface intravenously by using an intraarterial or intravenous catheter, suitable for delivery of the compounds to the desired location. The location of damaged arterial surfaces is determined by conventional diagnostic methods, such as X-ray angiography, performed using routine and well-known methods available to one skilled in the art.

When administered in vivo, the compositions of the invention can be administered in combination with pharmaceutically acceptable carriers and in a therapeutically effective dosage, as described herein.

When the compositions of the present invention are administered as a mixture of the 4-hydroxylated nebivolol metabolite, they can also be used in combination with one or more additional therapeutic agents which are known to be effective against the specific disease state targeted for treatment, such as for example diuretics and/or different antihypertensive agents such as beta-blockers, calcium channel blockers or ACE inhibitors. It is to be understood that such combination therapy constitutes a further aspect of the present invention.

"Concurrent administration" encompasses simultaneous or sequential treatment with the components of the combination, as well as regimens in which the drugs are alternated, or wherein one component is administered long-term and the other(s) are administered intermittently. Components can be administered in the same or in separate compositions, and by the same or different routes of administration.

The nebivolol metabolite of the present invention may be used in combination with other drugs that are used in the treatment/prevention/suppression or amelioration of the diseases or conditions for which the hydroxylated nebivolol metabolite is useful. Other drugs include, for example, an antithrombogenic agent, a thrombolytic agent, a fibrinolytic agent, a vasospasm inhibitor, a potassium channel activator, a calcium channel blocker, an antihypertensive agent, an antimicrobial agent, an antibiotic, an antiplatelet agent, an antimitotic agent, an antiproliferative agent, a microtubule inhibitor, an antisecretory agent, a remodelling inhibitor, an antisense nucleotide, an anti-cancer chemotherapeutic agent, a steroid, a non-steroidal antiinflammatory agent, a selective COX-2 inhibitor, an immunosuppressive agent, a growth factor antagonist or antibody, a dopamine agonist, a radiotherapeutic agent, a biologic agent, an angiotensin converting enzyme inhibitor, an angiotensin II receptor antagonist, a renin inhbitior, a free radical scavenger, an iron chelator, an antioxidant, a sex hormone, an antipolymerase, an antiviral agent, a photodynamic therapy agent, an antibody targeted therapy agent, a gene therapy agent, biphosphonates or other osteoporosis agents, cholesterol lowering agents, including but not limited to statins, niacin, or gemfibrozil, or mixtures thereof.

Such other drugs may be administered, by a route and in an amount commonly used therefore, contemporaneously or sequentially with the 4-hydroxylated nebivolol metabolite. When the nebivolol metabolite used in the present invention is used contemporaneously with one or more other drugs, a pharmaceutical composition containing such other drugs in addition to the compound of the present invention is preferred. Accordingly, the pharmaceutical compositions of the present invention include those that also contain one or more other active ingredients, in addition to the nebivolol metabolite used in the present invention. Examples of other active ingredients that may be combined with the nebivolol metabolite used in the present invention, either administered separately or in the same pharmaceutical compositions, include, but are not limited to: (1) morphine and other opiate receptor agonists including propoxyphene (Darvon); (2) non-steroidal anti-inflammatory drugs (NSAIDs) including COX-2 inhibitors such as propionic acid derivatives (alminoprofen, benoxaprofen, bucloxic acid, carprofen, fenbufen, fenoprofen, fluprofen, flurbiprofen, ibuprofen, indoprofen, ketoprofen, miroprofen, naproxen, oxaprozin, pirprofen, pranoprofen, suprofen, tiaprofenic acid, and tioxaprofen), acetic acid derivatives (indomethacin, acemetacin, alclofenac, clidanac, diclofenac, fenclofenac, fenclozic acid, fentiazac, furofenac, ibufenac, isoxepac, oxpinac, sulindac, tiopinac, tolmetin, zidometacin, and zomepirac), fenamic acid derivatives (flufenamic acid, meclofenamic acid, mefenamic acid, niflumic acid and tolfenamic acid), biphenylcarboxylic acid derivatives (diflunisal and flufenisal), oxicams (isoxicam, piroxicam, sudoxicam and tenoxican), salicylates (acetyl salicylic acid, sulfasalazine) and the pyrazolones (apazone, bezpiperylon, feprazone, mofebutazone, oxyphenbutazone, phenylbutazone), and the coxibs (celecoxib, valecoxib, rofecoxib and etoricoxib); (3) corticosteroids such as betamethasone, budesonide, cortisone, dexamethasone, hydrocortisone, methylprednisolone, prednisolone, prednisone and triamcinolone; (4) histamine H1 receptor antagonists such as bromopheniramine, chlorpheniramine, dexchlorpheniramine, triprolidine, clemastine, diphenhydramine, diphenylpyraline, tripelennamine, hydroxyzine, methdilazine, promethazine, trimeprazine, azatadine, cyproheptadine, antazoline, pheniramine pyrilamine, astemizole, terfenadine, loratadine, cetirizine, desloratadine, fexofenadine and levocetirizine; (5) histamine H2 receptor antagonists such as cimetidine, famotidine and ranitidine; (6) proton pump inhibitors such as omeprazole, lansoprazole, pantoprazole and esomeprazole; (7) leukotriene antagonists and 5-lipoxygenase inhibitors such as zafirlukast, montelukast, pranlukast and zileuton; (8) drugs used for angina, myocardial ischemia including nitrates such as nitroglycerin and isosorbide nitrates, beta blockers such as atenolol, metoprolol, propranolol, acebutolol, betaxolol, bisoprolol, carteolol, labetalol, nadolol, oxprenolol, penbutolol, pindolol, sotalol and timolol, and calcium channel blockers such as diltiazam, verapamil, nifedipine, bepridil, felodipine, flunarizine, isradipine, nicardipine and nimodipine; (9) incontinence medications such as antimuscarinics, (e.g., tolterodine and oxybutinin); (10) gastrointestinal antispasmodics (such as atropine, scopolamine, dicyclomine, antimuscarinics, as well as diphenoxylate); skeletal muscle relaxants (cyclobenzaprine, carisoprodol, chlorphenesin, chlorzoxazone, metaxalone, methocarbamol, baclofen, dantrolene, diazepam, or orphenadrine); (11) gout medications such as allopurinol, probenicid and colchicine; (12) drugs for rheumatoid arthritis such as methotrexate, auranofin, aurothioglucose and gold sodium thiomalate; (13) drugs for osteoporosis such as alendronate and raloxifene; decongestants such as pseudoephedrine and phenylpropanolamine; (14) local anesthetics; (15) anti-herpes drugs such as acyclovir, valacyclovir and famcyclovir; (16) anti-emetics such as ondansetron and granisetron; (17) advanced glycosylation end-product crosslink breakers (AGE crosslink breakers), for example, alagebrium; and (18) advanced glycosylation end-product formation inhibitors (AGE formation inhibitors), for example, pimagebine.

The term "nitric oxide" encompasses uncharged nitric oxide (NO) and charged nitrogen monoxide species, preferably charged nitrogen monoxide species, such as nitrosonium ion (NO⁺) and nitroxyl ion (NO⁻). The nitrogen monoxide releasing, delivering or transferring compounds have the structure F--NO, wherein F is a nitrogen monoxide releasing, delivering or transferring moiety, and include any and all such compounds which provide nitrogen monoxide to its intended site of action in a form active for its intended purpose.

### Administration of a nebivolol metabolite with a blood pressure reducing agent

The nebivolol metabolite used in the compositions of the present invention and the acid addition salts thereof may be administered before, during or after the administration of a blood pressure reducing agent provided that the time of the administration of the compound used in the present invention in relation to the administration of the blood pressure reducing agent allows the compound used in the present invention to be effective in potentiating the effects of the blood pressure reducing agent.

As blood pressure reducing agents of which the activity is potentiated there may be mentioned agents having adrenergic and/or vasodilating activity. In particular such agents maybe the compounds mentioned in U.S. Pat. Nos. 3,663,607 and 3,836,671, in particular atenolol; U.S. Pat. Nos. 3,337,628 and 3,520,919, in particular propranolol; U.S. Pat. No. 3,873, 600, in particular metoprolol; U.S. Pat. No. 3,511,836, in particular prazosin; U.S. Pat. No. 2,484,029, in particular hydralazine; U.S. Pat. No. 2,928,829 in particular guanethidine; U.S. Pat. No. 2,503,059, in particular phentolamine; U.S. Pat. No. 3,261,859, in particular verapamil; U.S. Pat. No. 3,485,847 in particular nifedipine; U.S. Pat. No. 3,910, 924, in particular carteolol; German Pat. Nos. 2,458,624 and 2,458,625, in particular celiprolol. These groups of active ingredients are listed with the purpose of providing representative examples but not with the purpose of restricting the scope of the present invention.

Preferably the nebivolol metabolite of the present invention and the blood pressure reducing agent are administered in the form of suitable compositions. Said compositions are meant to also comprise products containing a nebivolol metabolite as defined herein and a blood-pressure reducing agent as a combined preparation for simultaneous, separate, timed-release of either or both components, or sequential use in blood-pressure reducing therapy. Such products may, for example, comprise a kit comprising a container with a suitable composition containing a nebivolol metabolite of the present invention and another container containing a composition with a blood pressure reducing agent. Such a product may have the advantage that the physician wishing to administer blood pressure reducing therapy can select, based on the diagnosis of the patient to be treated, the appropriate amounts of both components and the sequence of administration.

When administered during the administration of the blood pressure reducing agent, a composition containing both the blood pressure reducing agent and the active ingredient of the nebivolol metabolite used in the present invention may particularly be convenient.

In a further aspect of the present invention there may be provided a composition comprising an amount capable of potentiating the effects of blood pressure reducing agents of the nebivolol metabolite used in the compositions of the present invention as defined herein and a blood pressure reducing agent. In the said composition, the molar ratio between the nebivolol metabolite and the blood pressure reducing agent may be other than about 1:1, or may be about 1:1. The amount of the active ingredient of the nebivolol metabolite in such a composition will be such that a potentiation of the effects of the blood-pressure reducing agent is obtained; the amount of the blood pressure reducing agent will be such that when potentiated, a blood pressure reducing effect is obtained upon administration. In particular, it is contemplated that the molar ratio of the 4(S)-OH-(R,S,S,S)-nebivolol to the blood pressure reducing compound may be situated between about 50:1 and about 1:50, in particular between about 20:1 and about 1:20, or between about 10:1 and about 1:10, or between about 5:1 and about 1:5, more particularly between about 2:1 and about 1:2. Particular such compositions are those wherein the blood pressure reducing agent is one of the agents pertaining to the patents cited hereinabove, and more particularly the agents specifically mentioned herein.

The present invention also provides a composition comprising a pharmaceutically acceptable carrier and, as active ingredient, an amount capable of potentiating the effects of blood pressure reducing agents of the nebivolol metabolite used in the present invention or a pharmaceutically acceptable acid-addition salt thereof, as defined herein.

To prepare such pharmaceutical compositions, an effective amount of the particular compound or compounds, in base or acid-addition salt form, as the active ingredient or active ingredients is combined in intimate admixture with a pharmaceutically acceptable carrier, which carrier may take a wide variety of forms depending on the form of preparation desired for administration. These pharmaceutical compositions are desirably in unitary dosage form suitable, preferably, for administration orally, rectally or by parenteral injection. For example, in preparing the compositions in oral dosage form, any of the usual pharmaceutical media may be employed, such as, for example, water, glycols, oils, alcohols and the like in the case of oral liquid preparations such as suspensions, syrups, elixirs and solutions; or solid carriers such as starches, sugars, kaolin, lubricants, binders, disintegrating agents and the like in the case of powders, pills, capsules and tablets. Because of their ease in administration, tablets and capsules represent the most advantageous oral dosage unit form, in which case solid pharmaceutical carriers are obviously employed. For parenteral compositions, the carrier will usually comprise sterile water, at least in large part, though other ingredients, for example, to aid solubility, maybe included. Injectable solutions, for example, maybe prepared in which the carrier comprises saline solution, glucose solution or a mixture of saline and glucose solution. Injectable suspensions may also be prepared in which case appropriate liquid carriers, suspending agents and the like may be employed. In the compositions suitable for percutaneous administration, the carrier optionally comprises a penetration enhancing agent and/or a suitable wetting agent, optionally combined with suitable additives of any nature in minor proportions, which additives do not cause a significant deleterious effect to the skin. Said additives may facilitate the administration to the skin and/or may be helpful for preparing the desired compositions. These compositions may be administered in various ways, e.g., as a transdermal patch, as a spot-on, as an ointment. Acid addition salts of the nebivolol metabolites of the present invention, due to their increased water solubility over the corresponding base form, are obviously more suitable in the preparation of aqueous compositions.

Also described is a method of potentiating the effects of blood pressure reducing agents in warm-blooded animals in need of blood pressure reducing medication, said method comprising administering to said warm-blooded animals of an effective amount of a blood pressure reducing agent and a nebivolol metabolite of the present invention, as defined hereinabove.

Or alternatively, there is described a method of lowering the blood pressure in warm-blooded animals suffering therefrom, said method comprising administering to said warm-blooded animals of an effective amount of a blood pressure reducing agent and a nebivolol metabolite as defined hereinabove.

Those of skill in treating subjects suffering from an increased blood pressure could easily determine the safe and effective amount from the test results presented hereinafter. In general it is contemplated that an effective daily dose of the nebivolol metabolites of the present invention or their pharmaceutically acceptable acid-addition salts would be from 0.01 mg/kg to 50 mg/kg body weight, in particular from 0.1 mg/kg to 10 mg/kg body weight, and preferably from 0.1 mg/kg to 1 mg/kg body weight.

### Nebivolol metabolites and nitric oxide release from normotensive Black American and White Donors

A higher prevalence of cardiovascular risk factors, especially hypertension, has been observed among Black Americans in the United States. The increased CV risk among Black Americans may be attributed to differences in vascular physiology, including reduced NO bioavailability. In support of this concept, a clinical evaluation of brachial artery activity demonstrates reduced responsiveness of conductance vessels to both endogenous and exogenous NO in healthy Black Americans, as compared with age-matched Whites. The basis for this difference is due to the low bioavailability of NO from endothelium of Black Americans, despite much higher levels of endothelial-dependent NO synthase (eNOS). The cellular basis for this paradox is the finding that excessive O₂⁻ generation by NAD(P)H -oxidase and uncoupled eNOS resulted in the loss of functional NO due to its reactivity with O₂⁻, resulting in peroxynitrite (ONOO⁻) formation, a potent oxidant.

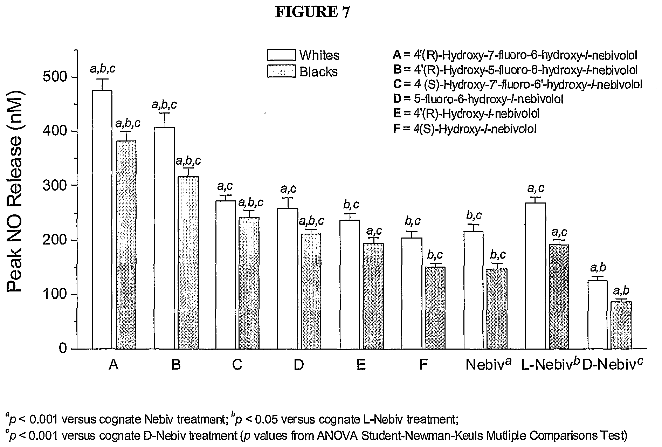

Six stereoselective hydroxy metabolites significantly enhanced NO bioavailability in endothelial cells from both white and black American donors (Figure 7). Several metabolites had NO release activity that was dramatically greater than nebivolol, and even *l*-nebivolol due to key placements of hydroxyl substituents on the nebivolol molecule. Thus, steoselective hydroxy metabolites of nebivolol are potent stimulants of NO release from human endothelial cells. Also, certain hydroxy metabolites have NO release activity that is dramatically greater than nebivolol or *l*-nebivolol due to select hydroxyl substituents.

*Experimental details:* Acute treatment with six stereoselective hydroxy metabolites in endothelial cells from both white and black American donors shows that all six hydroxy metabolites have activity that is much superior to *d-*nebivolol, and most are superior to nebivolol racemate. As compared to nebivolol, the 4'(*R*)-hydroxy-7-fluoro-6-hydroxy-*l*-nebivolol metabolite causes a nearly two-fold increase in NO release to 476 ± 20 and 383 ± 18 nmol/L, respectively, from white and black donors. The 4'(*R*)-hydroxy-5-fluoro-*l*-nebivolol metabolite also exceeds the activity of nebivolol and even *l-*nebivolol by >50%. These findings indicate that hydroxy metabolites of nebivolol have distinct and highly reproducible effects on acute NO release in human endothelial cells.

**Table 1: Acute NO Release with Compounds**

| **Name** | **White Donors NO (nM)** | **Black Donors NO (nM)** |
|---|---|---|
| 4'(R)-Hydroxy-7-fluoro-6-hydroxy-*l*-nebivolol | **476** ± **20** | **383** ± **18** |
| 4'(R)-Hydroxy-5-fluoro-6-hydroxy-*l*-nebivolol | **407** ± 2**7** | **316** ± **17** |
| 4(S)-Hydroxy-7'-fluoro-6'-hydroxy-*l*-nebivolol | **272** ± **12** | **242** ± **12** |
| 5-fluoro-6-hydroxy-*l*-nebivolol | **258** ± **20** | **212** ± **9** |
| 4'(R)-Hydroxy-*l*-nebivolol | **237** ± **11** | **194** ± **10** |
| 4(S)-Hydroxy-*l*-nebivolol | **204** ± **12** | **151** ± **8** |
| *l*-nebivolol | **268** ± **12** | **192** ± **9** |
| nebivolol | **218** ± **11** | **148** ± **10** |
| *d*-nebivolol | **127** ± **17** | **87** ± **6** |

In **Table 1** and **Figure 7**, the comparative effects of nebivolol, *d*-nebivolol, *l-*nebivolol and six stereoselective hydroxy metabolites on NO release are shown for cells harvested from white (open bars) and black American (solid bars) donors. The release of the NO from these endothelial cells was evaluated with electrochemical nanosensors placed within close proximity to the cell surface. All six hydroxy metabolites have activity that is significantly greater than *d*-nebivolol, and four are superior to nebivolol racemate. As compared to nebivolol, the 4(*R*)-hydroxy-7-fluoro-6-hydroxy-*l*-nebivolol (1689-64A) metabolite causes a nearly two-fold increase in NO release to 476 ± 20 and 383 ± 18 nmol/L, respectively, from white and black donors. The 4'(*R*)-hydroxy-5-fluoro-*l*-nebivolol metabolite (1689-64B) also exceeds the activity of *l*-nebivolol by >50%. The metabolite with the least activity is similar to nebivolol. These findings indicate that hydroxy metabolites of nebivolol have distinct and highly reproducible effects on acute NO release in human endothelial cells.

### Other Pharmacological Examples

### Methods and Materials: Measurements of NO Release from Human Endothelium

All measurements presented are recorded *in vitro* using a sensitive porphyrinic probe. NO release is measured directly from human umbilical vein endothelial cells (HUVEC). HUVEC cells were obtained from American Type Culture Collection (Manassas, VA) and grown in Ham's F12K medium with 2mM L-glutamine adjusted to contain 1.5g/l sodium bicarbonate and supplemented with 0.1mg/ml heparin and 0.03-0.05mg/ml endothelial cell growth supplement (ECGS) = 10% fetal bovine serum. The HUVEC cells were kept in an atmosphere of elevated CO₂ concentration (5%). Nebivolol, its enantiomers, and nebivolol metabolites including the nebivolol glucuronides, 4-hydroxy-5'phenol metabolite, 4-hydroxy-8'-phenol metabolite, 4-hydroxy, 5-hydroxy, 8-hydroxy metabolites, N-DAD, N-DACA and N-DAAA, are obtained from Mylan Laboratories (Morgantown, WV.)

All measurements of endothelial NO release are conducted in Hank's balance solution at 37°C. Cell wells are transferred to a Faraday cage and a porphyrinic sensor (diameter 0.5mm) is positioned at a distance of 5±2 µm from the surface of the endothelial cells using an inverted microscope (Leica Microsystems, Wetzler, Germany) and a computer-assisted micromanipulator. The sensor operates with a three-electrode system: nanosensor (working electrode), saturated calomel electrode (reference electrode) and platinum wire (counter electrode, 0.5mm diameter). The three electrodes are connected to a potentiostat/galvanostat PAR273. The baseline is stabilized after about 20 seconds. The test compounds are injected with a nanoinjector on to the surface of the cells following solubilization in buffer. The current proportional to the NO concentration is measured with the sensor, which operates in amperometric mode at a constant potential of 0.63V. Data are acquired with the use of an IBM computer with custom software and amperograms (current vs. time curves) and are recorded with a Guniry FAS1 Femtostat (Warminster, PA).

Nanosensors are prepared from carbon fibers. The size of the tip of carbon fiber is reduced from 6 µm to less than 1 µm by temperature controlled burning. The sensors are sensitized to NO by deposition of electrically conductive polymeric porphyrin and covered with a thin layer of Nafion. The prophyrinic microsensor has a response time of 0.1ms at a micromolar NO concentration and 10ms at the detection limit of 1 nM.

The nanosensor for NO is calibrated using saturated solution (concentration1.82mM verified with the coulometric method). Linear calibration curves are constructed for each sensor from 5 x to 3 x NO before and after measurements of cell activity. The data are presented as the mean ±SEM for each of the triplicate measurements. The data (calculation and plotting) are transferred to Microcal Origin Software (OriginLab Corp., Northampton, MA).

The HUVEC preparation is stable over the course of these experiments with the cells remaining viable in culture for >24 hours. Under non-stimulating conditions, basal levels of NO release are very low (<30 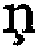 M). Measurement of NO release as a function of treatment is conducted in individual endothelial cells. Multiple measurements of NO release can be conducted on single cells following a brief refractory period. For robust statistical analysis, separate cells are used for each concentration and type of drug used in these analyses.

### Comparative Effects of Acute Treatment with Nebivolol Metabolites on NO Release from Human Endothelial Cells

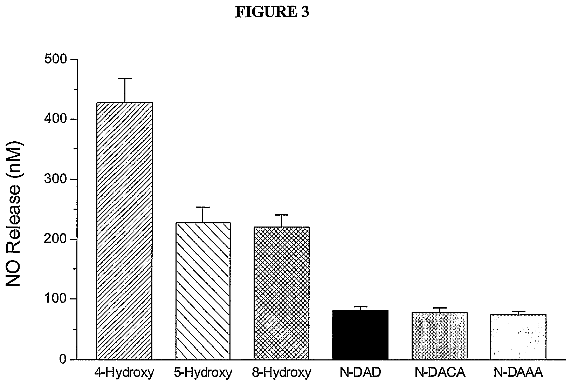

The results of these analyses demonstrate that six metabolites of the ß₁-adrenoceptor-selective antagonist nebivolol have different effects on NO release from human endothelial cells over a range of concentrations. FIGURE 3 compares peak NO release from HUVEC preparations for the six metabolites following addition of the agents at a concentration of 10µM. It is apparent from this figure that the metabolites have quantitatively different effects on NO release under identical experimental conditions. At a relatively high concentration of 10µM, only one metabolite (4-hydroxy nebivolol) produced NO levels that exceed 400 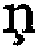 M whereas three of the metabolites (N-DAD, N-DACA and N-DAAA) stimulate less than 90 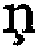 M peak NO levels, even at this relatively high concentration. The remaining two metabolites (5-hydroxy and 8-hydroxy nebivolol) have intermediate effects on NO release of approximately 220 nM NO at a concentration of 10µM. The dihydroxy nebivolol metabolites, including the 4-hydroxy-5'-phenol and 4-hydroxy-8'-phenol, as well as glucuronidated metabolites, are all active in regards to NO releasing capacity.

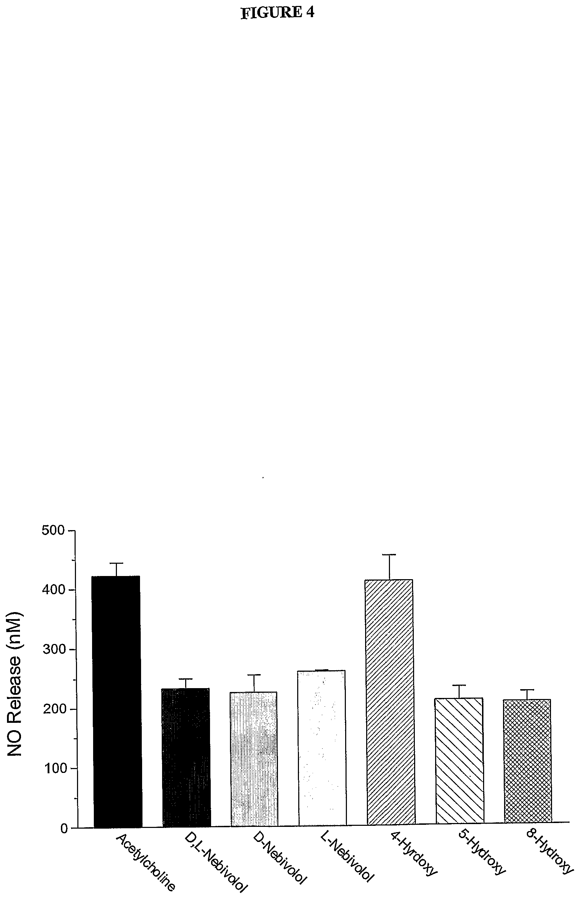

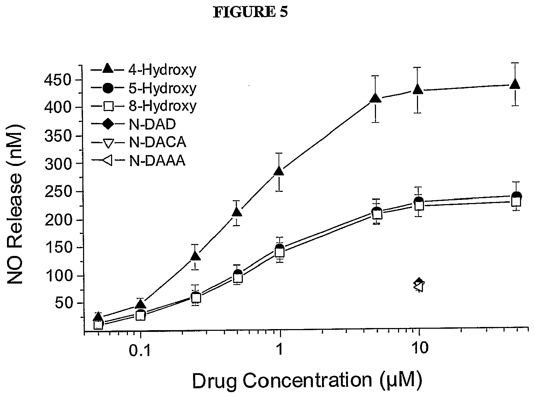

The NO releasing effects of certain nebivolol metabolites are then compared directly to acetylcholine, nebivolol and the nebivolol enantiomers in FIGURE 4. These data show that the metabolite, 4-hydroxy nebivolol, has the greatest NO releasing effects compared to nebivolol or its enantiomers. At an identical concentration of 5µM, the release of NO by 4-hydroxy nebivolol exceeds 400 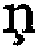 M whereas nebivolol produces approximately a 225 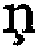 M level of NO peak release. Interestingly, 4-hydroxy nebivolol demonstrates an NO releasing effect that approximates values measured with acetylcholine, the natural mediator for NO release from the endothelium. The release of endothelial NO following treatment with either 5-hydroxy or 8-hydroxy nebivolol is similar to that for the parent nebivolol compound and its enantiomers, as shown in FIGURES 1-6.

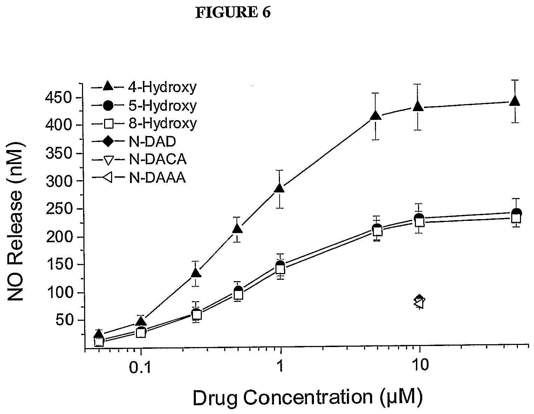

In FIGURE 6, the most efficacious metabolite, 4-hydroxy nebivolol, is compared to the parent nebivolol compound and its enantiomers. Consistent with the previous figures, there are significant differences in the relative potency and efficacy of the agents when administered under identical experimental conditions. Comparing nebivolol and the other metabolites, 4-hydroxy nebivolol exhibits the greatest NO releasing effects over a broad range of drug concentrations with an EC₅₀ of 500 NM. Comparing treatment with the parent compound or its enantiomers, significant differences in efficacy are observed for 4-hydroxy nebivolol at concentrations as low as 500 nM.

## Claims

1. A pharmaceutical composition for use in preventing or treating endothelian dysfunction and for preventing or treating cardiovascular diseases by increasing NO release from endothelial cells, comprising: 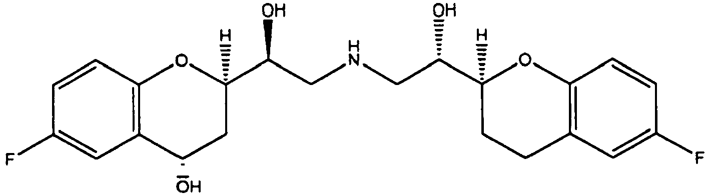 or a pharmaceutically acceptable salt thereof, and a pharmaceutically-acceptable carrier.

2. The pharmaceutical composition for use of claim 1, further comprising at least one other active agent.

3. The pharmaceutical composition for use of claim 1, further comprising only one other active agent.

4. A pharmaceutical composition for use in the treatment of cardiovascular diseases, comprising: 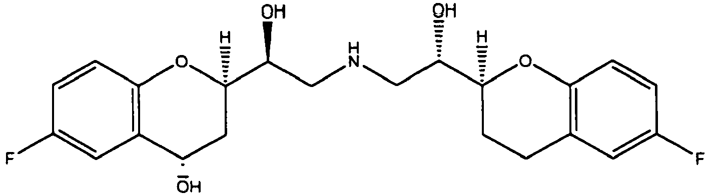 or a pharmaceutically acceptable salt thereof, and at least one other active agent.

5. The pharmaceutical composition for use of any of claims 2 or 4 wherein the at least one other active agent is a cardiovascular agent.

6. The pharmaceutical composition for use of claim 5 wherein the cardiovascular agent is selected from the group consisting of ACE inhibitors, ARB's, adrenergic blockers, adrenergic agonists, agents for pheochromocytoma, antiarrhythmics, antiplatelet agents, anticoagulants, antihypertensives, antilipemic agents, antidiabetics, antiinflammatory agents, calcium channel blockers, CETP inhibitors, COX-2 inhibitors, direct thrombin inhibitors, diuretics, endothelin receptor antagonists, HMG Co-A reductase inhibitors, inotropic agents, rennin inhibitors, vasodilators, vasopressors, AGE crosslink breakers, AGE formation inhibitors, and mixtures thereof.

7. The pharmaceutical composition for use of claim 5, wherein the cardiovascular agent is selected from the group consisting of an ACE inhibitor, an ARB, and mixtures thereof.

8. The pharmaceutical composition for use of claim 7, wherein the cardiovascular agent is an ACE inhibitor.

9. The pharmaceutical composition for use of claim 8 wherein the ACE inhibitor is selected from the group consisting of alacepril, benazepril, captopril, ceronapril, cilazapril, delapril, enalapril, enalaprilat, fosinopril, imidapril, lisinopril, perindopril, quinapril, ramipril, ramiprilat, spirapril, temocapril, trandolapril, and mixtures thereof.

10. The pharmaceutical composition for use of claim 8 wherein the ACE inhibitor is selected from the group consisting of enalapril, ramipril and ramiprilat and mixtures thereof.

11. The pharmaceutical composition for use of claim 2 wherein the at least one other active agent is a cardiovascular agent.

12. The pharmaceutical composition for use of claim 11 wherein the cardiovascular agent is selected from the group consisting of ACE inhibitors, ARB's, adrenergic blockers, adrenergic agonists, agents for pheochromocytoma, antiarrhythmics, antiplatelet agents, anticoagulants, antihypertensives, antilipemic agents, antidiabetics, antiinflammatory agents, calcium channel blockers, CETP inhibitors, COX-2 inhibitors, direct thrombin inhibitors, diuretics, endothelin receptor antagonists, HMG Co-A reductase inhibitors, inotropic agents, rennin inhibitors, vasodilators, vasopressors, AGE crosslink breakers and AGE formation inhibitors.

13. The pharmaceutical composition for use of claim 11, wherein the cardiovascular agent is selected from the group consisting of an ACE inhibitor, an ARB, and mixtures thereof.

14. The pharmaceutical composition for use of claim 12, wherein the cardiovascular agent is an ACE inhibitor selected from the group consisting of alacepril, benazepril, captopril, ceronapril, cilazapril, delapril, enalapril, enalaprilat, fosinopril, imidapril, lisinopril, perindopril, quinapril, ramipril, ramiprilat, spirapril, temocapril and trandolapril.

15. The pharmaceutical composition for use of claim 2, wherein said composition comprises a pharmaceutical kit.

## Patentansprüche

1. Pharmazeutische Zusammensetzung zur Benutzung zum Vorbeugen gegen oder Behandeln von endothelialer Dysfunktion und zum Vorbeugen gegen oder Behandeln von Herz-Kreislauf-Erkrankungen durch Erhöhen der NO-Freisetzung aus Endothelzellen, umfassend: 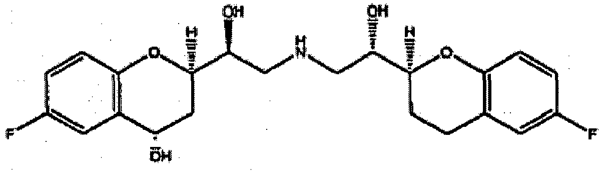 oder ein pharmazeutisch akzeptables Salz davon und einen pharmazeutisch akzeptablen Träger.

2. Pharmazeutische Zusammensetzung zur Benutzung nach Anspruch 1, ferner umfassend mindestens ein anderes aktives Mittel.

3. Pharmazeutische Zusammensetzung zur Benutzung nach Anspruch 1, ferner umfassend nur ein anderes aktives Mittel.

4. Pharmazeutische Zusammensetzung zur Benutzung in der Behandlung von Herz-Kreislauf-Erkrankungen, umfassend: 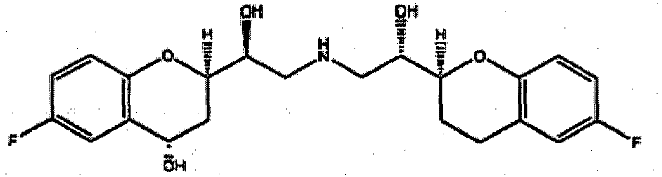 oder ein pharmazeutisch akzeptables Salz davon und mindestens ein anderes aktives Mittel.

5. Pharmazeutische Zusammensetzung zur Benutzung nach einem von Anspruch 2 und 4, wobei das mindestens eine andere aktive Mittel ein Herz-Kreislauf-Mittel ist.

6. Pharmazeutische Zusammensetzung zur Benutzung nach Anspruch 5, wobei das Herz-Kreislauf-Mittel aus der Gruppe ausgewählt ist, die aus ACE-Hemmern, ARB, adrenergen Blockern, adrenergen Agonisten, Mitteln gegen Phäochromozytom, Antiarrhythmika, Antithrombozytenmitteln, Antikoagulantien, Antihypertensiva, lipidsenkenden Mitteln, Antidiabetika, entzündungshemmenden Mitteln, Kalziumkanalblockern, CETP-Hemmern, COX-2-Hemmern, direkten Thrombinhemmern, Diuretika, Endothelrezeptor-Antagonisten, HMG-Co-A-Reduktase-Hemmern, inotropen Mitteln, Reninhemmern, Vasodilatatoren, Vasopressoren, AGE-Crosslink-Breakern, AGE-Bildungshemmern und Gemischen davon besteht.

7. Pharmazeutische Zusammensetzung zur Benutzung nach Anspruch 5, wobei das Herz-Kreislauf-Mittel aus der Gruppe ausgewählt ist, die aus einem ACE-Hemmer, einem ARB und Gemischen davon besteht.

8. Pharmazeutische Zusammensetzung zur Benutzung nach Anspruch 7, wobei das Herz-Kreislauf-Mittel ein ACE-Hemmer ist.

9. Pharmazeutische Zusammensetzung zur Benutzung nach Anspruch 8, wobei der ACE-Hemmer aus der Gruppe ausgewählt ist, die aus Alacepril, Benazepril, Captopril, Ceronapril, Cilazapril, Delapril, Enalapril, Enalaprilat, Fosinopril, Imidapril, Lisinopril, Perindopril, Quinapril, Ramipril, Ramiprilat, Spirapril, Temocapril, Trandolapril und Gemischen davon besteht.

10. Pharmazeutische Zusammensetzung zur Benutzung nach Anspruch 8, wobei der ACE-Hemmer aus der Gruppe ausgewählt ist, die aus Enalapril, Ramipril und Ramiprilat und Gemischen davon besteht.

11. Pharmazeutische Zusammensetzung zur Benutzung nach Anspruch 2, wobei das mindestens eine andere aktive Mittel ein Herz-Kreislauf-Mittel ist.

12. Pharmazeutische Zusammensetzung zur Benutzung nach Anspruch 11, wobei das Herz-Kreislauf-Mittel aus der Gruppe ausgewählt ist, die aus ACE-Hemmern, ARB, adrenergen Blockern, adrenergen Agonisten, Mitteln gegen Phäochromozytom, Antiarrhythmika, Antithrombozytenmitteln, Antikoagulantien, Antihypertensiva, lipidsenkenden Mitteln, Antidiabetika, entzündungshemmenden Mitteln, Kalziumkanalblockern, CETP-Hemmern, COX-2-Hemmern, direkten Thrombinhemmern, Diuretika, Endothelrezeptor-Antagonisten, HMG-Co-A-Reduktase-Hemmern, inotropen Mitteln, Reninhemmern, Vasodilatatoren, Vasopressoren, AGE-Crosslink-Breakern und AGE-Bildungshemmern besteht.

13. Pharmazeutische Zusammensetzung zur Benutzung nach Anspruch 11, wobei das Herz-Kreislauf-Mittel aus der Gruppe ausgewählt ist, die aus einem ACE-Hemmer, einem ARB und Gemischen davon besteht.

14. Pharmazeutische Zusammensetzung zur Benutzung nach Anspruch 12, wobei das Herz-Kreislauf-Mittel ein ACE-Hemmer ist, der aus der Gruppe ausgewählt ist, die aus Alacepril, Benazepril, Captopril, Ceronapril, Cilazapril, Delapril, Enalapril, Enalaprilat, Fosinopril, Imidapril, Lisinopril, Perindopril, Quinapril, Ramipril, Ramiprilat, Spirapril, Temocapril und Trandolapril besteht.

15. Pharmazeutische Zusammensetzung zur Benutzung nach Anspruch 2, wobei die Zusammensetzung ein pharmazeutisches Kit umfasst.

## Revendications

1. Composition pharmaceutique pour une utilisation dans la prévention ou le traitement d'un disfonctionnement endothélial et pour la prévention et le traitement de maladies cardiovasculaires en augmentant la libération de NO à partir des cellules endothéliales, comprenant : 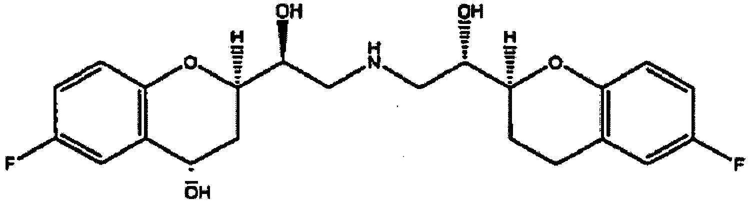 ou un de ses sels pharmaceutiquement acceptables, et un support pharmaceutiquement acceptable.

2. Composition pharmaceutique pour une utilisation selon la revendication 1, comprenant en outre au moins un autre agent actif.

3. Composition pharmaceutique pour une utilisation selon la revendication 1, comprenant en outre uniquement un autre agent actif.

4. Composition pharmaceutique pour une utilisation dans le traitement de maladies cardiovasculaires, comprenant : 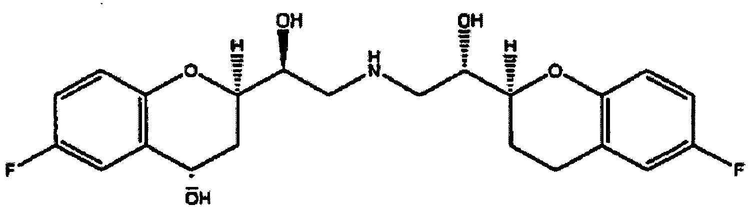 ou un de ses sels pharmaceutiquement acceptables, et au moins un autre agent actif.

5. Composition pharmaceutique pour une utilisation selon l'une quelconque des revendications 2 ou 4, dans laquelle l'au moins un autre agent est un agent cardiovasculaire.

6. Composition pharmaceutique pour une utilisation selon la revendication 5, dans laquelle l'agent cardiovasculaire est choisi dans le groupe constitué d'inhibiteurs des ECA, des ARA II, des bloquants adrénergiques, d'agonistes adrénergiques, d'agents pour un phéochromocytome, d'anti-arythmiques, d'agents antiplaquettaires, d'anticoagulants, d'antihypertenseurs, d'agents antilipémiants, d'antidiabétiques, d'agents anti-inflammatoires, de bloquants de canaux calciques, d'inhibiteurs de la CETP, d'inhibiteurs de la COX-2, d'inhibiteurs directs de la thrombine, de diurétiques, d'antagonistes de récepteurs de l'endothéline, d'inhibiteurs de l'HMG Co-A réductase, d'agents inotropiques, d'inhibiteurs de la rénine, de vasodilatateurs, de vasopresseurs, d'agent de dégradation des pontages d'AGE, d'inhibiteurs de formation d'AGE, et de mélanges de ceux-ci.

7. Composition pharmaceutique pour une utilisation selon la revendication 5, dans laquelle l'agent cardiovasculaire est choisi dans le groupe constitué d'un inhibiteur d'ECA, d'un ARA II, et de mélanges de ceux-ci.

8. Composition pharmaceutique pour une utilisation selon la revendication 7, dans lequel l'agent cardiovasculaire est un inhibiteur d'ECA.

9. Composition pharmaceutique pour une utilisation selon la revendication 8, dans laquelle l'inhibiteur d'ECA est choisi dans le groupe constitué de l'alacépril, du bénazépril, du captopril, du céronapril, du cilazapril, du détapril, de l'énalapril, de l'énalaprilate, du fosinopril, de l'imidapril, du lisinopril, du périndopril, du quinapril, du ramipril, du ramiprilate, du spirapril, du témocapril, du trandolapril, et de mélanges de ceux-ci.

10. Composition pharmaceutique pour une utilisation selon la revendication 8, dans laquelle l'inhibiteur d'ECA est choisi dans le groupe constitué de l'énalapril, du ramipril et du ramiprilate et de mélanges de ceux-ci.

11. Composition pharmaceutique pour une utilisation selon la revendication 2, dans laquelle l'au moins un autre agent actif est un agent cardiovasculaire.

12. Composition pharmaceutique pour une utilisation selon la revendication 11, dans laquelle l'agent cardiovasculaire est choisi dans le groupe constitué d'inhibiteurs des ECA, des ARA II, des bloquants adrénergiques, d'agonistes adrénergiques, d'agents pour un phéochromocytome, d'anti-arythmiques, d'agents antiplaquettaires, d'anticoagulants, d'antihypertenseurs, d'agents antilipémiants, d'antidiabétiques, d'agents anti-inflammatoires, de bloquants de canaux calciques, d'inhibiteurs de la CETP, d'inhibiteurs de la COX-2, d'inhibiteurs directs de la thrombine, de diurétiques, d'antagonistes de récepteurs de l'endothéline, d'inhibiteurs de l'HMG Co-A réductase, d'agents inotropiques, d'inhibiteurs de la rénine, de vasodilatateurs, de vasopresseurs, d'agents de dégradation des pontages d'AGE et d'inhibiteurs de formation d'AGE.

13. Composition pharmaceutique pour une utilisation selon la revendication 11, dans laquelle l'agent cardiovasculaire est choisi dans le groupe constitué d'un inhibiteur d'ECA, d'un ARA II et de mélanges de ceux-ci.

14. Composition pharmaceutique pour une utilisation selon la revendication 12, dans laquelle l'agent cardiovasculaire est un inhibiteur d'ECA choisi dans le groupe constitué de l'alacépril, du bénazépril, du captopril, du céronapril, du cilazapril, du délapril, de l'énalapril, de l'énalaprilate, du fosinopril, de l'imidapril, du lisinopril, du périndopril, du quinapril, du ramipril, du ramiprilate, du spirapril, du témocapril et du trandolapril.

15. Composition pharmaceutique pour une utilisation selon la revendication 2, dans laquelle ladite composition comprend un kit pharmaceutique.
